(19) Europäisches Patentamt
European Patent Office
Office européen des brevets

(11) Numéro de publication: **0 289 380 B1**

(12) **FASCICULE DE BREVET EUROPEEN**

(45) Date de publication de fascicule du brevet: **28.12.94**

(51) Int. Cl.$^5$: **C07D 233/64**, C07D 487/04, C07D 231/12, C07D 401/12, C07D 403/12, C07D 401/06, A61K 31/415

(21) Numéro de dépôt: **88400828.5**

(22) Date de dépôt: **06.04.88**

(54) **Dérivés d'aryl-hétéroaryl carbinols avec activité analgésique.**

(30) Priorité: **10.04.87 FR 8705118**

(43) Date de publication de la demande:
**02.11.88 Bulletin 88/44**

(45) Mention de la délivrance du brevet:
**28.12.94 Bulletin 94/52**

(84) Etats contractants désignés:
**AT BE CH DE FR GB GR IT LI LU NL SE**

(56) Documents cités:
**FR-A- 2 137 839**
**FR-A- 2 166 117**

**SYNTHESIS, no. 9, septembre 1978, pages 675-676, Georg Thieme Publishers; L.A.M. BASTIAANSEN et al.: "2-Aroylimidazoles; a simple one-step synthesis"**

**JOURNAL OF MEDICINAL CHEMISTRY, vol. 27, no. 9, septembre 1984, pages 1245-1253, American Chemical Society; M.J. Ashton et al.: "Heterocyclic analogues of chlorcyclizine with potent hypolipidemic activity"**

(73) Titulaire: **LABORATORIOS DEL DR. ESTEVE, S.A.**
**Av. Mare de Deu de Montserrat, 221**
**E-08026 Barcelona (ES)**

(72) Inventeur: **Colombo, Augusto**
**Av. Chile, 36, 40 1a**
**E-08032 Barcelone (ES)**
Inventeur: **Parés, Juan**
**Padilla, 349, 30 3a**
**E-08025 Barcelone (ES)**
Inventeur: **Frigola, Jordi**
**Av. Diagonal, 299 at. 1a**
**E-08013 Barcelone (ES)**

(74) Mandataire: **Ahner, Francis et al**
**CABINET REGIMBEAU**
**26, avenue Kléber**
**F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**TETRAHEDRON**, vol. 39, no. 12, 1983, pages 2023-2029, Pergamon Press, Oxford,GB; A.R. KATRITZKY et al.: "Alpha-lithiation of N-alkyl groups in pyrazoles"

## Description

La présente invention se rapporte à des nouveaux dérivés d'aryl-hétéroaryl carbinols, leur procédé de préparation, ainsi que leur application en tant que médicaments.

Les composés objet de la présente invention peuvent également être utilisés dans l'industrie pharmaceutique comme intermédiaires et pour la préparation de médicaments.

Ce type de carbinols a été très peu étudié et il n'existe qu'un petit nombre d'exemples dans la littérature scientifique :

Tertov, B.A. Khim.-Farm. Zh. 10, 34-6 (1976)
Ashton, M. et al. J. Med. Chem. 27,1245-53 (1984)
Ohta, S. et al. Tetrah. Lett. 25, 3251-4 (1978)
Bastiaansen L.A.M. et al. Synthesis, 9, 675-6 (1978)
Effenberger, F., J. Org. Chem. 49, 4687-95 (1984)
Katritzky, A. Tetrahedron. 39, 2023-9 (1983)
Okamoto, M. Heterocycles 23, (7) 1759 (1985)

De même, parmi la littérature brevet, seule la demande FR-A-2 166 117 (FARBWERKE HOECHST AG.) décrit des imidazoly-(2)-carbinols ayant une activité hypolipidémique.

Les nouveaux dérivés d'aryl-hétéroaryl carbinols objet de le présente invention répondent à la formule générale (I)

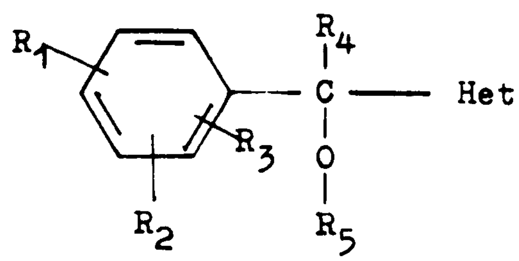

(I)

dans laquelle

- $R_1$, $R_2$, $R_3$, identiques ou différents, représentent un atome d'hydrogène, un halogène, un radical alkyle inférieur, un groupe trifluorométhyle ($CF_3$), ou un radical alcoxy inférieur,
- $R_4$ représente un atome d'hydrogène, un radical alkyle inférieur de $C_1$ à $C_4$, un cycloalkyle, un radical alkényle inférieur ou cycloalkylamino substitué sur l'atome d'azote.
- $R_5$ représente un atome d'hydrogène ou un aminoalkyle de formule

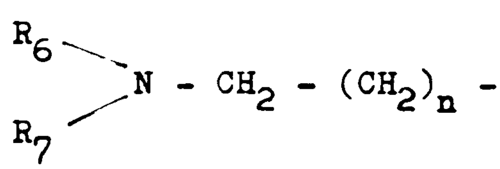

dans laquelle

- $R_6$ et $R_7$, identiques ou différents, représentent un alkyle inférieur ou un cycloalkyle ou un cycloalcoxy et n représente 1 ou 2;
- $R_5$ représente aussi un groupement de formule générale:

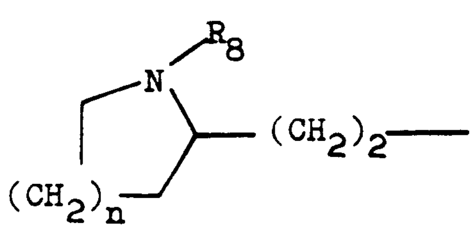

dans laquelle n peut avoir la valeur 1 ou 2, et $R_8$ représente un alkyle inférieur, et

Het représente un azole choisi parmi :

a) Pyrazole de formule générale

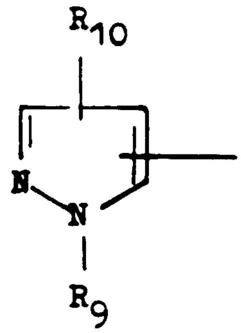

dans laquelle $R_9$ représente un radical alkyle inférieur en $C_1$ à $C_4$ ou un radical dodécyle ou un radical benzyle ou un radical

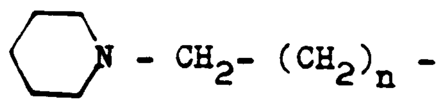

dans lequel n = 1 ou 2

$R_{10}$ représente un atome d'hydrogène, un radical méthyle ou un atome d'halogène,

b) Imidazole de formule générale

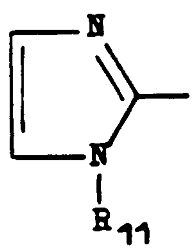

dans laquelle $R_{11}$ représente un atome d'hydrogène, un radical alkyle inférieur en $C_1$ à $C_4$, un radical benzyle, un radical dodécyle ou un radical du type

$A - (CR_2)_n—$

dans lequel n = 2, 3 ou 4 et A représente un radical N-pipéridino, un radical cyano, un radical hydroxy, un radical carboxy ou un radical carboxyméthyl ou un radical amino,

à l'exception toutefois des composés de formule générale I dans laquelle Het représente un Imidazole, $R_5$ représente un atome d'hydrogène et $R_{11}$ représente un atome d'hydrogène, une chaîne hydrocarbonnée contenant de 1 à 10 atomes de carbone, un radical aralkyle ou un radical aryle, et à l'exception des composés suivants :

1H-pyrazole-5-méthanol-1-méthyl-α-phényl, et

1H-pyrazole-5-méthanol-1-méthyl-α-(4-méthyl-phényl),

c) Het peut représenter un imidazole de formule

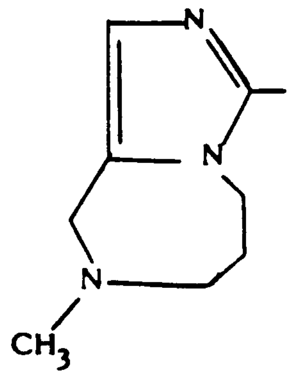

La présente invention se rapporte également aux sels physiologiquement acceptables des composés de formule générale I, en particulier les sels d'addition avec des acides minéraux ou organiques physiologiquement acceptables, par exemple l'oxalate, le tartrate, le citrate ou encore l'hydroquinone sulfonate.

Les dérivés de formule générale I et leurs sels sont appropriés pour prévenir ou traiter les douleurs d'intensité moyenne à forte : sciatique, lumbago, dorsalgie, entorses, fractures et luxations; douleurs post-opératoires de tous genres, douleurs d'origine dentaire, etc...

Les dérivés de formule générale I peuvent également être utilisés dans l'industrie pharmaceutique comme intermédiaires et pour la préparation de médicaments.

Les nouveaux dérivés de formule générale I peuvent être préparés conformément à l'invention selon l'une quelconque des méthodes suivantes.

Méthode A

Par réaction d'un composé de formule générale

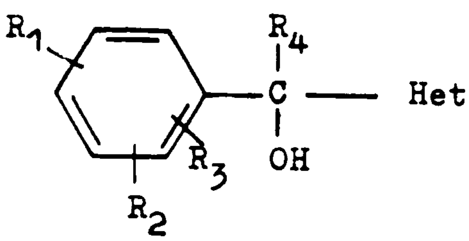

II

avec un composé de formule générale

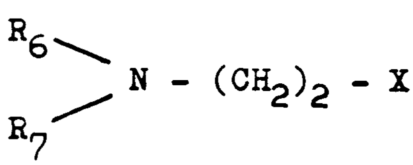

III

dans lesquels $R_1$ à $R_4$, $R_6$ et $R_7$ et Het ont les significations mentionnées précédemment et X représente un atome d'halogène, de préférence le chlore ou un groupe partant choisi parmi le tosyloxy ou mésyloxy.

La réaction du composé de formule générale II avec un composé de formule générale III s'effectue en présence d'un solvant adéquat par exemple les hydrocarbures tels que le benzène ou le toluène ou bien dans les solvants halogénés tels que le chlorure de méthylène ou le tétrachlorure de carbone, ou bien dans les éthers comme le tétrahydrofuranne ou bien d'autres solvants aprotiques tels que le diméthylsulfoxyde, ou la diméthylformamide.

Cette réaction est avantageusement conduite en présence d'une base adéquate, telles que les bases minérales comme l'hydroxyde de sodium ou de potassium ou les carbonates ou bicarbonates de sodium ou

de potassium.

Cette réaction est plus avantageusement conduite en présence d'un catalyseur de transfert de phase tels que le bromure de tétrabutylammonium, le chlorure de triéthylbenzylammonium, ou les éthers couronne, et dans un intervalle de température compris entre celle de l'ambiance et celle de reflux du solvant.

Méthode B

Par réaction d'un composé de formule générale

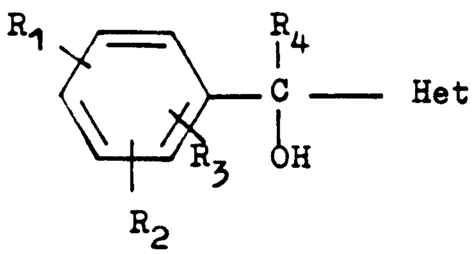

II

avec un composé de formule générale

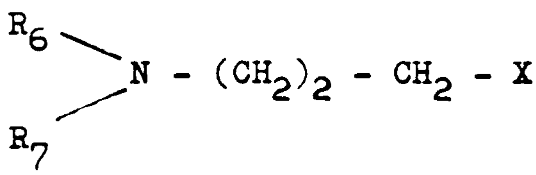

IV

dans lesquels $R_1$ à $R_4$, $R_6$ et $R_7$, X, et Het ont les significations mentionnées précédemment et X représente un atome d'halogène, de préférence le chlor ou un groupe partant, choisi parmi le tosyloxy ou le mésyloxy.

La réaction du composé de formula générale II avec un composé de formule générale IV s'effectue en présence d'un solvant adéquat, par exemple les hydrocarbures tels que le benzène, ou le toluène ou bien dans des éthers tels que le tétrahydrofuranne ou le dioxanne. Cette réaction est avantageusement conduite en présence d'une base adéquate, telles que les bases minérales comme l'hydroxyde de sodium ou de potassium, ou les carbonates et bicarbonates de sodium ou de potassium.

Cette réaction est plus avantageusement conduite en présence d'un catalyseur de transfert de phase tel que le bromure de tétrabutylammonium, chlorure de triéthylbenzylammonium ou les éthers-couronnes.

Cette réaction peut être conduite dans un intervalle de températures entre 80 et 120 ° C.

Les composés de formule générale II peuvent être préparés conformément à l'invention selon l'une quelconque des méthodes suivantes.

Méthode C

Par réduction d'un composé de formule générale V

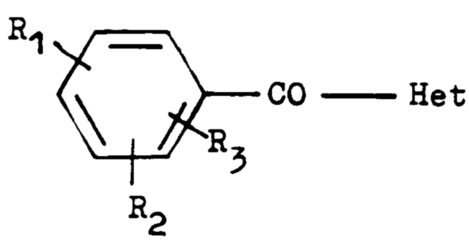

V

dans laquelle $R_1$, $R_2$, $R_3$ et Het ont la signification mentionnée précédemment, on obtient un composé de formule générale II dans laquelle $R_4$ est un atome d'hydrogène.

Cette réduction s'effectue au sein d'un solvant approprié comme les éthers tels que le tétrahydrofuranne, le diméthyléther ou le dioxanne avec des hydrures tels que l'hydrure d'aluminium et de lithium, ou bien au sein d'alcools tels que le méthanol ou l'éthanol avec l'hydrure de bore et de sodium, ou bien avec de l'hydrogène au sein d'un solvant approprié tels que les alcools ou les hydrocarbures cycliques ou les éthers, avec un catalyseur approprié tels que le nickel Raney, le palladium ou l'oxyde de platine.

La pression dans le cas de l'hydrogénation est comprise avantageusement entre $10^5$ Pa et $10^6$ Pa , de préférence entre $10^5$ Pa et $2.10^5$ Pa et les températures les plus adéquates oscillent entre 20 et 100 °C et le temps de réaction est compris entre 1 heure et 48 heures.

Méthode D

Par réaction entre un composé de formule générale V avec des réactifs de Grignard du type

$R_{11}$ - Mg X

dans lequel $R_{11}$ représente un alkyle inférieur en $C_1$ à $C_4$, un cycloalkyle en $C_6$ substitué ou non, un alkényle inférieur ou un cycloalkylamino substitué.

Cette réaction s'effectue au sein d'un solvant approprié par exemple les éthers tels que le diéthyl éther, le tétrahydrofuranne, ou le dioxanne, ou des mélanges de ces éthers avec d'autres solvants tels que le benzène, le toluène ou le xylène.

Méthode E

Par réaction entre aldéhydes de formule générale

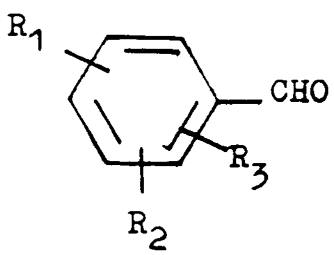

VI

dans laquelle $R_1$, $R_2$ et $R_3$ ont les significations mentionnées précédemment, avec le sel de lithium de l'azole, au sein d'un solvant approprié tels que l'éther ou l'hexane. Cette réaction est de préférence conduite à une température comprise entre -15 °C et 100 °C, de préférence entre -5 °C et +35 °C.

Méthode F

On peut obtenir des composés de formule

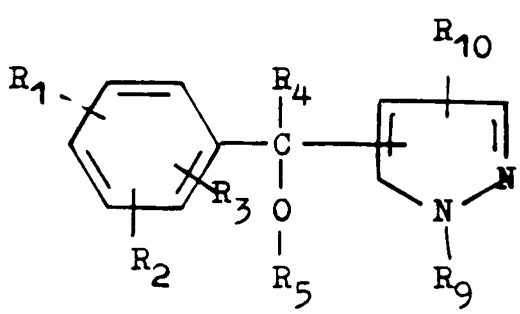

VII

dans laquelle $R_1$, $R_2$, $R_3$, $R_4$, $R_5$ et $R_9$ ont la signification mentionnée précédemment et $R_{10}$ est du chlore ou du brome, par halogénation directe du composé précurseur.

Méthode G

On obtient des composés de formule

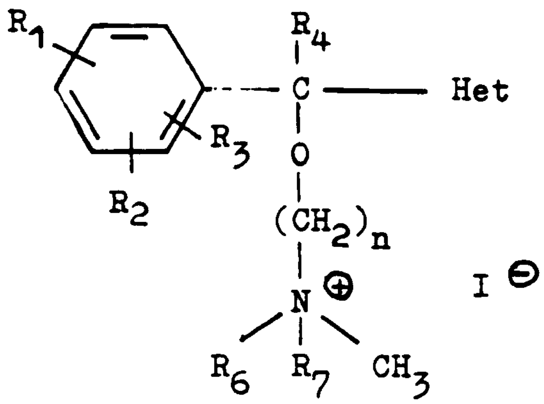

VIII

par alkylation avec l'iodure de méthyle de composés de formule I

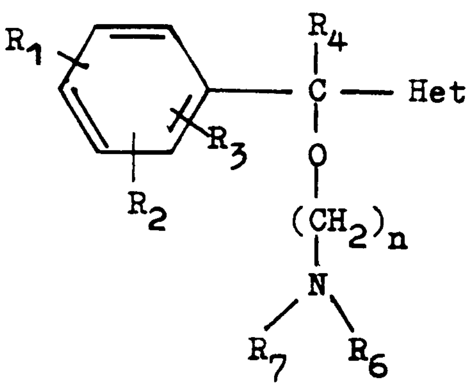

I

dans laquelle $R_1$ à $R_7$ ont les significations mentionnées précédemment, dans des solvants appropriés, tels que les cétones comme l'acétone, ou la méthyléthylcétone, à des températures comprises entre la température ambiante et celle du reflux du solvant.

Méthode H

On obtient des composés de formule générale IX

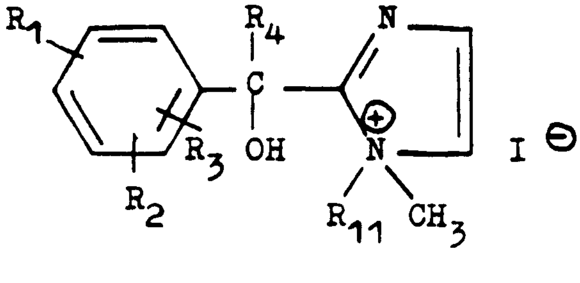

IX

par réaction de composés du type

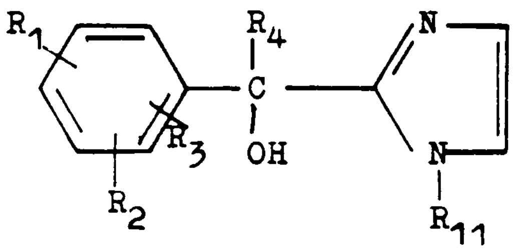

X

avec l'iodure de méthyle,
dans lesquels $R_1$ à $R_{11}$ ont les significations mentionnées précédemment.

Cette réaction est avantageusement conduite dans des solvants adéquats, tels que les cétones comme la méthyléthylcétone, ou l'acétone, ou bien les éthers tels que le dioxanne, le tétrahydrofuranne ou l'éther.

Dans les exemples suivants on indique la préparation de nouveaux dérivés selon l'invention. On décrira également quelques formes d'emploi typiques pour les différents domaines d'application.

Les exemples ci-après, donnés à simple titre d'illustration, ne doivent cependant en aucune façon limiter l'étendue de l'invention.

Méthode A (exemples 1 à 58)

Exemple n° 3

Préparation de 1H-imidazole-2-méthanol-α-(4-chlorophényl)-1-méthyl-O-(2-diméthylamino)éthyle

Dans un erlenmeyer de 500 ml on ajoute :
20 ml de NaOH 5 %
100 ml de benzène
2,2 g de 1H-imidazole-2-méthanol-α-(4-chlorophényl)-2-méthyle
15 g de N,N-diméthylamino-2-chloroéthane et
50 mg de chlorure de benzyltriéthylammonium.

On agite fortement pendant 24 heures, on laisse reposer, on décante et on lave la phase organique avec de l'eau. On sèche sur du sulfate de sodium et on élimine le solvant sous vide.

On obtient ainsi 2,4 g (83 %) de 1H-imidazole-2-méthanol,α-(4-chlorophényl)-1-méthyl-O-(2-diméthylamino)éthyle.

Les composés identifiés par les exemples 1 à 58 sont préparés par la même méthode de préparation décrite dans l'exemple 1 et les données pour l'identification des produits sont présentées dans les tableaux I à V.

**Tableau I**

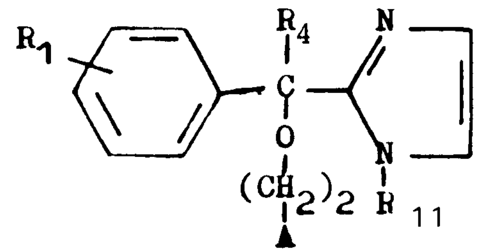

| Exemple n° | $R_1$ | $R_4$ | $R_{11}$ | A | Méthode |
|---|---|---|---|---|---|
| 1 | H | H | Me | DMA | A |
| 2 | 4 Cl | Me | Me | DMA | A |
| 3 | 4 Cl | H | Me | DMA | A |
| 4 | 3 Cl | H | Me | DMA | A |
| 5 | 2 Cl | Me | Me | DMA | A |
| 6 | 4 F | Me | Me | DMA | A |
| 7 | 3 $F_3C$- | Me | Me | DMA | A |
| 8 | 3 Cl | Me | Me | DMA | A |

DMA = diméthylamino

**Tableau I** (suite)

| Exemple n° | $R_1$ | $R_4$ | $R_{11}$ | A | Méthode |
|---|---|---|---|---|---|
| 9 | 3 Cl | n-Bu | Me | DMA | A |
| 10 | 4 Cl | Me | n-Bu | DMA | A |
| 11 | 4 MeO- | Me | Me | DMA | A |
| 12 | 3 Cl | Me | Me | Pyrr | A |
| 13 | 3 4 5 tri MeO- | n-Bu | $C_{12}H_{25}$- | DMA | A |
| 14 | 4 $F_3C$- | H | n-Bu | DMA | A |
| 15 | 3 $F_3C$- | Me | Me | Pip | A |
| 16 | 3 4 di Cl | 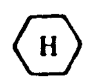 | Me | DMA | A |

DMA = diméthylamino Pyrr = pyrrolidine Pip = pipéridine

Tableau I (suite)

| Exemple n° | $R_1$ | $R_4$ | $R_{11}$ | A | Méthode |
|---|---|---|---|---|---|
| 17 | 3,4 di Cl | n-Bu | Me | DMA | A |
| 18 | 3,4 di Cl | Me | Me | DMA | A |
| 19 | 3,4 di Cl | H | Me | DMA | A |
| 20 | 4 Cl | Me | 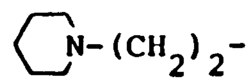 | DMA | A |
| 21 | 4 Cl | Me | 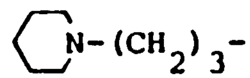 | DMA | A |
| 22 | 4 Cl | H | $N\equiv C-(CH_2)_3-$ | DMA | A |
| 23 | 4 Cl | 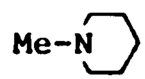 | Me | DMA | A |
| 24 | 4 Cl | H | 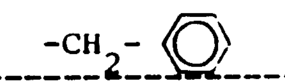 | MBA | A |

DMA = diméthylamino MBA = méthyl-benzylamine

## Tableau II

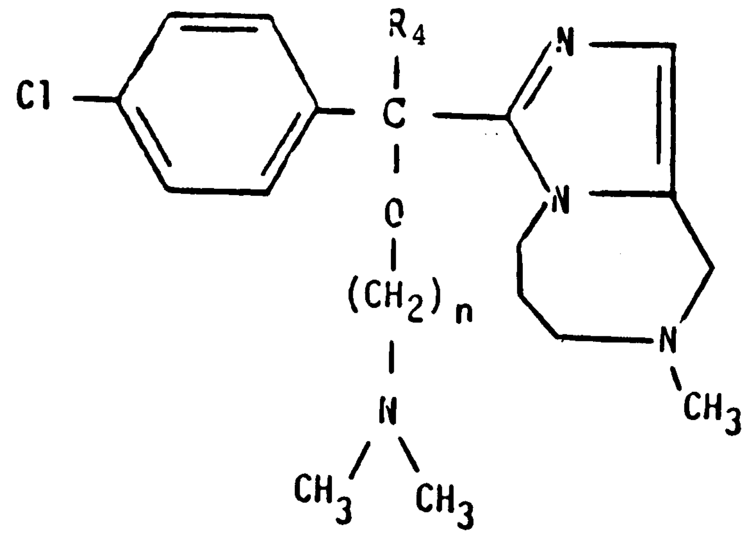

| Exemple n° | $R_4$ | n | Méthode |
|---|---|---|---|
| 25 | Me | 2 | A |
| 26 | H | 2 | A |

Tableau III

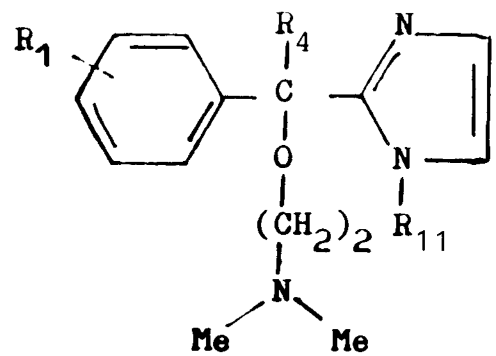

| Exemple n° | $^{1}H$ RMN $-Cl_3CD$ |
|---|---|
| 1 | 7,2 (s,5H); 6,8 (d,2H); 5,7 (s,1H); 3,5 (m,2H); 3,35 (s,3H); 2,6 (t,2H); 2,3 (s,6H) |
| 2 | 7,2 (s,4H); 6,85 (d,2H); 3,7 (m,1H); 3,2 (s,3H); 3,1 (m,1H); 2,5 (t,2H); 2,2 (s,6H); 1,85 (s,3H) |
| 3 | 7,25 (s,4H); 6,85 (d,2H); 6,65 (s,1H); 3,6 (m,2H); 3,45 (s,3H); 2,6 (t,2H); 2,25 (s,6H) |
| 4 | 7,3 (m,4H); 6,9 (d,2H); 5,7 (s,1H); 3,6 (m,2H); 3,5 (s,3H); 2,65 (t,2H); 2,35 (s,6H) |
| 5 | 7,9(m,1H); 7,1 (m,3H); 6,8 (d,2H); 3,55 (m,1H); 3,05 (s,3H); 2,8 (m,1H); 2,4 (t,2H); 2,15 (s,6H); 2,0 (s,3H) |

Tableau III (Suite)

| Exemple n° | $^1H$ RMN - $Cl_3CD$ |
|---|---|
| 6 | 7,0 (m,6H); 3,5 (m,2H); 3,3 (s,3H); 2,5 (t,2H); 2,3 (s,6H); 1,8 (s,3H) |
| 7 | 7,3 (m,3H); 6,8 (d,2H); 3,6 (m,2H); 3,2 (s,3H); 2,5 (m,2H); 2,2 (s,6H); 1,8 (s,3H) |
| 8 | 7,0 (m,3H); 6,8 (d,2H); 3,55 (m,2H); 3,2 (s,3H); 2,5 (m,2H); 2,2 (s,6H); 1,8 (s,3H) |
| 9 | 7,5-6,6 (m,6H); 3,55 (m,1H); 3,2 (s,3H); 3,0 (m,1H); 2,6 (m,2H); 2,2 (s,6H); 1,5-0,5 (m,7H) |
| 10 | 7,2 (s,4H); 6,9 (d,2H); 3,7 (m,3H); 3,0 (m,1H); 2,5 (t,2H); 2,25 (s,6H); 1,9 (s,3H); 1,4-0,6 (m,7H) |
| 11 | 7,0 (q,4H); 6,75 (d,2H); 3,7 (s,3H); 3,6 (m,1H); 3,25 (s,3H); 3,0 (m,1H); 2,55 (t,2H); 2,2 (s,6H); 1,9 (s,3H) |
| 12 | 7,1 (m,6H); 3,7 (m,1H); 3,2 (s,3H); 3,05 (m,1H); 2,7 (m,2H); 2,4 (m,4H); 1,9 (s,3H); 2,75 (m,4H) |

Tableau III (suite)

| Exemple n° | $^1H$ RMN - $Cl_3CD$ |
|---|---|
| 13 | 6,9 (d,2H); 6,5 (s,2H); 3,8 (s,3H); 3,7 (s,6H); 3,55 (m,1H); 3,0 (m,1H); 2,55 (t,2H); 2,2 (S,6H); 1,3-0,7 (m,34H) |
| 14 | 7,4 (s,4H); 6,8 (d,2H); 5,8 (s,1H); 3,6 (m,4H); 2,45 (t,2H); 2,1 (s,6H); 1,6-0,5 (m,7H) |
| 15 | 7,7 (s,1H); 7,35 (m,3H); 6,9 (d,2H); 3,7 (m,3H); 3,2 (s,3H); 3,1 (m,1H); 2,6 (m,2H); 2,4 (m,4H); 1,9 (s,3H); 1,45 (4H) |
| 16 | 7,5-6,7 (m,5H); 3,5 (m,1H); 3,1 (s,3H); 2,8 (m,2H); 2,4 (m,3H); 2,15 (s,6H); 2,0-0,3 (m,9H) |
| 17 | 7,5-6,7 (m,5H); 3,55 (m,2H); 3,20 (s,3H); 1,0 (m,1H); 3,5 (m,3H); 2,2 (s,6H); 1,4-0,6 (m,7H) |
| 18 | 7,4-6,7 (m,5H); 3,6 (m,2H); 3,20 (s,3H); 3,05 (m,2H); 2,5 (m,2H); 2,2 (s,6H); 1,8 (s,3H) |
| 19 | 7,6-6,7 (m,5H); 5,6 (s,1H); 3,6 (m,2H); 3,45 (s,3H); 2,6 (t,2H); 2,2 (s,6H) |

Tableau III (suite)

| Exemple n° | $^1H$ RMN - $Cl_3CD$ |
|---|---|
| 20 | 7,2 (s,4H); 6,95 (d,2H); 3,7 (m,3H); 3,0 (m,1H); 2,5 (t,2H); 2,2 (s,6H); 2,1 (m,6H); 1,8 (s,3H); 1,4 (m,6H) |
| 21 | 7,25 (s,4H); 6,9 (d,2H); 3,7 (m,3H); 3,1 (m,1H); 2,6 (t,2H); 2,20 (s,6H); 2,15 (m,6H); 1,9 (m,5H) |
| 22 | 7,3 (s,4H); 6,95 (d,2H); 5,7 (s,1H); 3,95 (m,4H); 3,6 (m,2H); 2,6 (t,2H); 2,2 (m,8H); 1,9 (m,2H); 1,45 (m,6H) |
| 23 | 7,3-6,7 (m,6H); 3,5 (m,1H); 3,1 (s,3H); 2,8 (m,4H); 2,4 (t,2H); 2,15-1,0 (m,19H) |
| 24 | 7,2 (s,12H); 7,0-6,8 (m,3H); 5,7 (s,1H); 5,0 (s,2H); 3,6 (m,2H); 3,5 (s,2H); 2,6 (t,2H); 2,2 (s,3H) |
| 25 | 7,25 (m,4H); 6,9 (s,1H); 4,0-3,0 (m,9H); 2,75 (m,2H); 2,55 (t,2H); 2,3 (s,6H); 1,9 (s,3H); 1,6-1,1 (m,3H) |
| 26 | 7,3 (s,4H); 6,8 (s,1H); 5,6 (s,1H); 4,5 (m,2H); 4,1-3,5 (m,8H); 2,8 (m,4H); 2,6 (t,2H); 2,2 (d,9H); 1,5 (m,3H) |

Tableau IV

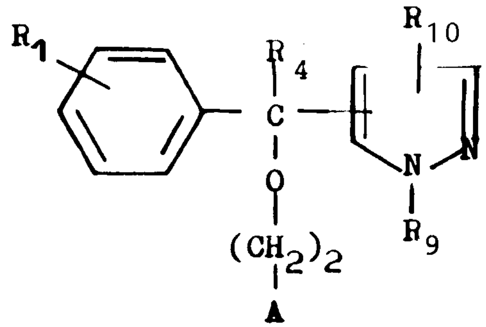

| Exemple n° | $R_1$ | $R_4$ | $R_9$ | A | $R_{10}$ | Pyrazole Substitution | Méthode |
|---|---|---|---|---|---|---|---|
| 27 | H | H | n-Bu | DMA | H | 5 | A |
| 28 | 4 Cl | 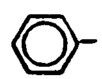 | Me | DMA | H | 5 | A |
| 29 | 3 4 5 tri MeO | H | n-Bu | DMA | H | 5 | A |
| 30 | 4 Cl | Me | n-Bu | DMA | H | 5 | A |
| 31 | H | H | Me | DMA | H | 5 | A |
| 32 | H | Me | Me | DMA | H | 5 | A |

Tableau IV (suite)

| Exemple n° | $R_1$ | $R_4$ | $R_9$ | A | $R_{10}$ | Pyrazole substitution | Méthode |
|---|---|---|---|---|---|---|---|
| 33 | 3 4 5 tri Meo | H | Me | DMA | H | 5 | A |
| 34 | H | H | Me | Pirr. | H | 5 | A |
| 35 | H | H | Me | Mor. | H | 5 | A |
| 36 | 3 4 5 tri Meo | Me | Me | DMA | H | 5 | A |
| 37 | H | H | Me | DMA | 4 Br | 5 | A |
| 38 | H | Me | Me | DMA | 3 $CH_3$ | 5 | A |
| 39 | H | H | Me | DMA | 3 $CH_3$ | 5 | A |
| 40 | 2 Me | H | Me | DMA | H | 5 | A |
| 41 | 4 Cl | H | Me | DMA | 4 Cl | 5 | A |

Mor. = morpholine

Tableau IV (suite)

| Exemple n° | $R_1$ | $R_4$ | $R_9$ | A | $R_{10}$ | Pyrazole Substitution | Méthode |
|---|---|---|---|---|---|---|---|
| 42 | 4 Cl | H | Me | DMA | H | 4 | A |
| 43 | 4 Cl | Me | Me | DMA | H | 4 | A |
| 44 | 4 Cl | H | Me | DMA | H | 5 | A |
| 45 | 3 Cl | H | Me | DMA | H | 5 | A |
| 46 | 4 Me | H | Me | DMA | H | 5 | A |
| 47 | 2 Cl | H | Me | DMA | H | 5 | A |
| 48 | H | H | Me | Pip. | H | 5 | A |
| 49 | H | H | Me | 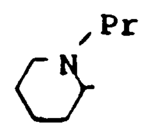 | H | 5 | A |
| 50 | 4 Cl | H | Me | 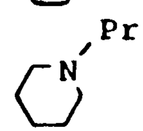 | H | 4 | A |

Tableau IV (suite)

| Exemple n° | $R_1$ | $R_4$ | $R_9$ | A | $R_{10}$ | Pyrazole substitution | Méthode |
|---|---|---|---|---|---|---|---|
| 51 | H | H | Me | 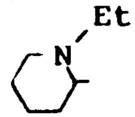 | H | 5 | A |
| 52 | H | H | Me | 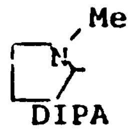 | H | 5 | A |
| 53 | H | H | Me | DIPA | H | 5 | A |
| 54 | 4 Cl | H | Me | 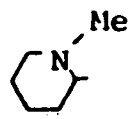 | H | 4 | A |
| 55 | 4 Cl | H | Me' | 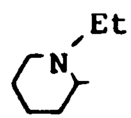 | H | 4 | A |
| 56 | H | H | Me | 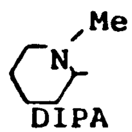 | H | 5 | A |
| 57 | 4 Cl | H | Me | DIPA | H | 4 | A |
| 58 | 4 Cl | H | Me | 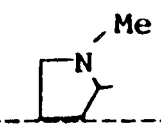 | H | 4 | A |

DIPA = diisopropylamine

**Tableau V**

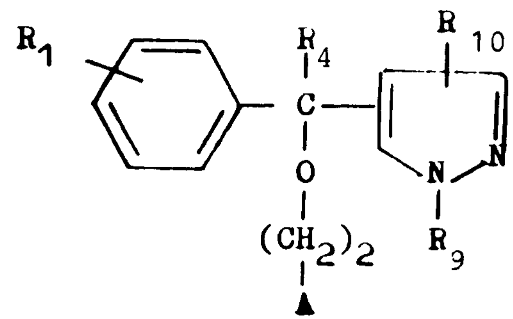

| Exemple n° | $^1H$ RMN - $Cl_3CD$ |
|---|---|
| 27 | 7,35 (m,6H); 5,95 (m,1H); 5,50 (s,1H); 4,05 (t,2H); 3,56 (t,2H); 2,52 (t,2H); 2,20 (s,6H); 1,75-0,7 (m,7H) |
| 28 | 7,5-7,1 (m,9H); 6,3 (d,1H); 3,45 (s,2H); 3,2 (t,2H); 2,55 (t,2H); 2,20 (s,6H) |
| 29 | 7,35 (m,1H); 6,6 (m,2H); 5,9 (t,1H); 5,45 (s,1H); 4,05 (t,2H); 3,8 (m,9H); 3,55 (t,2H); 2,6 (t,2H); 2,25 (d,6H); 1,9-0,7 (m,7H) |
| 30 | 7,45 (m,1H); 7,2 (s,4H), 6,3 (m,1H); 3,7 (t,2H); 3,15 (t,2H); 2,5 (t,2H); 2,25 (s,6H); 1,75 (s,3H); 1,65-0,6 (m,7H) |

Tableau V (suite)

| Exemple n° | $^1H$ RMN - $Cl_3CD$ |
|---|---|
| 31 | 7,2 (m,6H) ; 5,85 (d,1H) ; 5,35 (s,1H) ; 3,65 (s,3H) ; 3,4 (t,2H) ; 2,4 (t,2H) ; 2,1 (s,6H) |
| 32 | 7,45 (d,1H) ; 7,2 (s,5H) ; 6,4 (d,1H) ; 3,6 (m,1H) ; 3,4 (s,3H) ; 3,15 (m,1H) ; 2,55 (t,2H) ; 2,25 (s,6H) ; 1,8 (s,3H) |
| 33 | 7,35 (d,1H) ; 6,6 (s,2H) ; 6,0 (d,1H) ; 5,45 (s,1H) ; 3,85 (m,12H) ; 3,6 (t,2H) ; 2,6 (t,2H) ; 2,25 (s,6H) |
| 34 | 7,15-7,4 (m,6H) ; 5,9 (s,1H) ; 5,4 (s,1H) ; 3,65 (s,3H) ; 3,5 (t,2H) ; 2,65 (t,2H) ; 2,40 (m,4H) ; 1,65 (m,4H) |
| 35 | 7,3 (m,6H) ; 5,85 (d,1H) ; 5,4 (s,1H) ; 3,75 (s,3H) ; 3,55 (m,6H) ; 2,5 (t,2H) ; 2,35 (m,4H) |
| 36 | 7,45 (d,1H) ; 6,5 (s,2H) ; 6,35 (d,1H) ; 3,8 (s,3H) ; 3,75 (s,6H) ; 3,5 (s,3H) ; 2,5 (t,2H) ; 2,3 (s,6H) ; 1,8 (s,2H) |
| 37 | 7,45 (s,1H) ; 7,25 (s,5H) ; 5,85 (s,1H) ; 3,6 (m,5H) ; 2,6 (t,2H) ; 2,25 (s,6H) |

Tableau V (suite)

| Exemple n° | $^1$H RMN - $Cl_3CD$ |
|---|---|
| 38 | 7,2 (s,5H); 6,15 (s,1H); 3,65 (m,1H); 3,35 (s,3H); 3,2 (m,1H); 2,5 (t,2H); 2,2 (s,9H); 1,75 (s,3H) |
| 39 | 7,25 (s,5H); 5,7 (s,1H); 5,45 (s,1H); 3,7 (s,3H); 3,5 (t,2H); 2,6 (b,2H); 2,25 (s,6H); 2,1 (s,3H) |
| 40 | 7,4-7,0 (m,5H); 5,7 (s,1H); 5,6 (s,1H); 3,85 (s,3H); 3,5 (t,2H); 2,55 (t,2H); 2,15 (s,9H) |
| 41 | 7,5-7,0 (m,6H); 6,1 (s,1H); 3,6 (m,5H); 2,7 (t,2H); 2,2 (s,6H) |
| 42 | 7,2 (s,5H); 7,0 (s,1H); 3,75 (s,3H); 3,35 (t,2H); 2,4 (t,2H); 2,1 (s,6H) |
| 43 | 7,2 (m,6H); 3,7 (s,3H); 3,25 (t,2H); 2,4 (t,2H); 2,15 (s,6H); 1,7 (s,3H) |
| 44 | 7,3 (m,5H); 5,9 (s,1H); 5,5 (s,1H); 3,8 (s,3H); 3,5 (t,2H); 2,5 (t,2H); 2,2 (s,6H) |
| 45 | 7,4-7,1 (m,5H); 6,0 (s,1H); 5,5 (s,1H); 3,8 (s,3H); 3,6 (t,2H); 2,6 (t,2H); 2,2 (s,6H) |

Tableau V (suite)

| Exemple n° | $^1H$ RMN - $Cl_3CD$ |
|---|---|
| 46 | 7,3 (s,1H); 7,2 (d,4H); 5,9 (s,1H); 5,4 (s,1H); 3,8 (s,3H); 3,5 (t,2H); 2,5 (t,2H); 2,3 (s,3H); 2,2 (s,6H) |
| 47 | 7,7-7,1 (m,5H); 5,9 (s,1H); 5,8 (s,1H); 3,9 (s,3H); 3,6 (t,2H); 2,5 (t,2H); 2,2 (s,6H) |
| 48 | 7,2 (s,6H); 5,8 (s,1H); 5,4 (s,1H); 3,7 (s,3H); 3,5 (t,2H); 2,5 (t,2H); 2,3 (m,6H); 1,4 (m,6H) |
| 49 | 7,4 (s,6H); 6,0 (s,1H); 5,4 (s,1H); 3,7 (s,3H); 3,6 (t,2H); 3,0-1 (m,5H); 0,9 (t,3H) |
| 50 | 7,3 (s,5H); 7,1 (s,1H); 5,2 (s,1H); 3,7 (s,3H); 3,45 (m,2H); 2,9-1,0 (m,17H); 0,8 (t,3H) |
| 51 | 7,3 (s,6H); 6,0 (s,1H); 5,4 (s,1H); 3,7 (s,3H); 3,5 (t,2H); 2,8-1,1 (m,13H); 1,0 (t,3H) |
| 52 | 7,3 (s,6H); 6,0 (s,1H); 5,4 (s,1H); 3,7 (s,3H); 3,5 (t,2H); 3,0 (m,1H); 2,2-1,0 (m,11H) |

Tableau V (suite)

| Exemple n° | $^1H$ RMN - $Cl_3CD$ |
|---|---|
| 53 | 7,3 (s,6H); 6,0 (s,1H); 5,5 (s,1H); 3,7 (s,3H); 3,4 (t,2H); 2,9 (m,2H); 2,6 (t,2H); 0,95 (d,12H) |
| 54 | 7,3 (s,5H); 7,1 (s,1H); 5,2 (s,1H); 3,8 (s,3H); 3,5 (t,2H); 2,7 (m,1H); 2,2 (m,3H); 2,1-1,0 (m,9H) |
| 55 | 7,3 (s,5H); 7,1 (s,1H); 5,3 (s,3H); 3,5 (t,2H); 2,8-1,1 (m,13H); 1,0 (t,3H) |
| 56 | 7,3 (s,6H); 6,0 (s,1H); 5,5 (s,1H); 3,8 (s,3H); 3,6 (t,2H); 2,8 (m,1H); 2,3 (s,3H); 2,2-1,1 (m,12H) |
| 57 | 7,3 (s,5H); 7,1 (s,1H); 5,3 (s,1H); 3,8 (s,3H); 3,4 (t,2H); 3,0 (m,2H); 2,7 (t,2H); 1,0 (d,12H) |
| 58 | 7,3 (s,5H); 7,1 (s,1H); 5,3 (s,1H); 3,8 (s,3H); 3,5 (m,2H); 3,0 (m,1H); 2,3 (s,3H); 2,2-1,2 (m,8H) |

Méthode B : (exemples 59 à 104)

Exemple n° 59 :

Préparation de 1H-imidazole-2-méthanol, α-(4-chlorophényl)-α-méthyl-1-méthyl-O-(3-diméthylamino)propyle.

Dans un erlenmeyer de 500 ml, avec réfrigérant et forte agitation, on mélange
2,8 g de 1H-imidazole-2-méthanol, α-(4-dichlorophényl)-α-méthyl-1-méthyle,
3,8 g de 3-diméthylaminochloropropane
80 ml de benzène
20 ml de NaOH 50 %
50 mg de bromure de tétrabutylammonium

On chauffe à reflux pendant 24 heures et on ajoute 3,8 g de 3-diméthylaminochloropropane.

On chauffe à nouveau pendant 24 heures en plus, et on laisse refroidir. On décante, on lave plusieurs fois à l'eau, on sèche sur du sulfate de sodium et on élimine le solvant sous vide.

On obtient ainsi 3,0 g (86 %) de 1H-imidazole-2-méthanol, α-(4-chlorophényl)-α-méthyl-1-méthyl-O-(3-diméthylamino)propyle.

Les composés identifiés par les exemples 59 à 104 sont obtenus par la même méthode de préparation décrite dans l'exemple 59.

Les données pour l'identification des produits 59 à 104 se présentent dans les tableaux VI à VIII.

**Tableau VI**

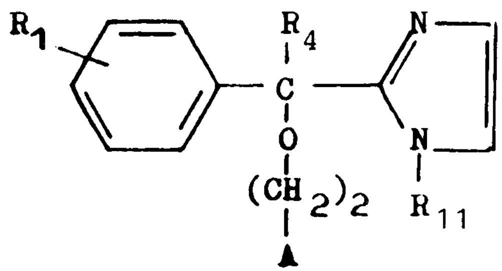

| Exemple n° | $R_1$ | $R_4$ | $R_{11}$ | ▲ | Méthode |
|---|---|---|---|---|---|
| 59 | 4 Cl | Me | Me | DMA | B |
| 60 | 3 Cl | H | Me | DMA | B |
| 61 | 4 Cl | Et | Me | DMA | B |
| 62 | 3 Cl | n-Bu | Me | DMA | B |
| 63 | 4 Cl | 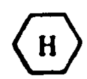 | Me | DMA | B |
| 64 | 4 F | Me | Me | DMA | B |
| 65 | 3 $F_3C$- | Me | Me | DMA | B |
| 66 | 2 Cl | Me | Me | DMA | B |

Tableau VI (suite)

| Exemple n° | $R_1$ | $R_4$ | $R_{11}$ | A | Méthode |
|---|---|---|---|---|---|
| 67 | 3 Cl | Me | Me | DMA | B |
| 68 | 3,4,5 tri MeO | Me | Me | DMA | B |
| 69 | 4 MeO | Me | Me | DMA | B |
| 70 | 4 Cl | H | Me | DMA | B |
| 71 | 3,4,5 tri MeO | H | Me | DMA | B |
| 72 | 4 $F_3C$- | Me | Me | DMA | B |
| 73 | 3 $F_3C$- | H | Me | DMA | B |
| 74 | 4 $F_3C$- | H | Me | DMA | B |
| 75 | 4 MeO- | H | Me | DMA | B |

Tableau VI (suite)

| Exemple n° | $R_1$ | $R_4$ | $R_{11}$ | A | Méthode |
|---|---|---|---|---|---|
| 76 | 3 $F_3C$- | n-Bu | Me | DMA | B |
| 77 | 4 Cl | Me | n-Bu | DMA | B |
| 78 | 3 4 5 tri Meo | n-Bu | n-Bu | DMA | B |
| 79 | 2 Cl | n-Bu | n-Bu | DMA | B |
| 80 | 2 4 di Cl | n-Bu | n-Bu | DMA | B |
| 81 | 4 $F_3C$- | H | n-Bu | DMA | B |
| 82 | 4 Cl | H | Me | Pip. | B |
| 83 | 4 $F_3C$- | Me | Me | Pip. | B |
| 84 | 2 Cl | n-Bu | Me | DMA | B |

Tableau VI (suite)

| Exemple N° | $R_1$ | $R_4$ | $R_{11}$ | A | Méthode |
|---|---|---|---|---|---|
| 85 | 3,4 di Cl | n-Bu | Me | DMA | B |
| 86 | 3,4 di Cl | Me | Me | DMA | B |
| 87 | 3,4 di Cl | H | Me | DMA | B |
| 88 | 3,4 di Cl | 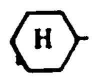 | Me | DMA | B |
| 89 | 4 Cl | Me | 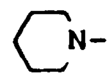 $-(CH_2)_2-$ | DMA | B |
| 90 | 4 Cl | Me | 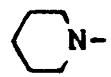 $-(CH_3)_3-$ | DMA | B |
| 91 | 4 Cl | $CH_3-$ 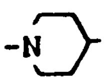 | Me | DMA | B |

Tableau VII

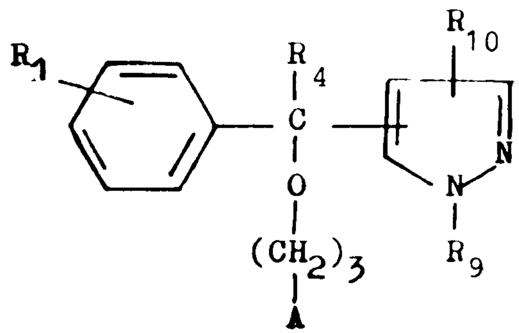

| Exemple n° | $R_1$ | $R_4$ | $R_9$ | A | $R_{10}$ | Pyrazole position | Méthode |
|---|---|---|---|---|---|---|---|
| 92 | H | H | n-Bu | DMA | H | 5 | B |
| 93 | 4 Cl | Me | n-Bu | DMA | H | 5 | B |
| 94 | H | H | Me | DMA | H | 5 | B |
| 95 | H | Me | Me | DMA | H | 5 | B |
| 96 | H | Me | Me | DMA | 3 Me | 5 | B |
| 97 | H | H | Me | DMA | 3 Me | 5 | B |
| 98 | 2 Me | H | Me | DMA | H | 5 | B |

Tableau VII (suite)

| Exemple n° | $R_1$ | $R_4$ | $R_9$ | A | $R_{10}$ | Pyrazole position | Méthode |
|---|---|---|---|---|---|---|---|
| 99 | 4 Cl | H | Me | DMA | H | 5 | B |
| 100 | H | H | Me | DMA | H | 4 | B |
| 101 | 4 Cl | H | Me | Mor. | H | 4 | B |
| 102 | 4 Cl | H | Me | Pir. | H | 4 | B |
| 103 | H | H | Me | Pip. | H | 5 | B |
| 104 | H | H | Me | Pir. | H | 5 | B |

Tableau VIII

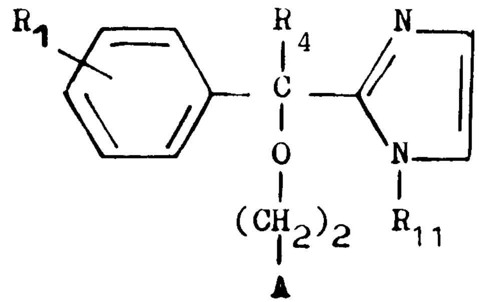

| Exemple n° | $^1H$ RMN - $Cl_3CD$ |
|---|---|
| 59 | 7,2 (m,4H) ; 6,85 (d,2H) ; 3,6 (m,1H) ; 3,2 (s,3H) ; 3,0 (m,1H) ; 2,4 (m,2H) ; 2,15 (s,6H) ; 1,8 (s,3H) |
| 60 | 7,2 (s,1H) ; 7,15 (s,3H) ; 6,8 (d,2H) ; 5,6 (s,1H) ; 3,55 (m,2H) ; 3,45 (s,3H) ; 2,4 (m,2H) ; 2,25 (s,6H) ; 1,8 (m,2H) |
| 61 | 7,2 (s,4H) ; 6,8 (d,2H) ; 3,4 (m,3H) ; 3,1 (s,3H) ; 2,9 (m,1H) ; 2,3 (m,2H) ; 2,2 (s,6H) ; 1,8 (m,2H) ; 0,5 (t,3H) |
| 62 | 7,2 (s,1H) ; 7,0 (s,3H) ; 3,4 (m,3H) ; 3,1 (s,3H) ; 2,9 (m,1H) ; 2,3 (m,2H) ; 2,25 (s,6H) ; 1,8 (m,2H) ; 0,5 (t,3H) |
| 63 | 7,15 (s,4H) ; 6,8 (d,2H) ; 3,5 (m,1H) ; 2,7 (m,1H) ; 2,4 (m,4H) ; 2,5 (s,6H) ; 1,9-0,5 (m,14H) |

Tableau VIII (suite)

| Exemple n° | $^1H$ RMN - $Cl_3CD$ |
|---|---|
| 64 | 7,3-6,7 (m,6H) ; 3,6 (m,1H) ; 3,3 (s,3H) ; 3,1 (m,1H) ; 2,4 (m,2H) ; 2,25 (s,6H) ; 1,9 (s,3H) ; 1,85 (m,2H) |
| 65 | 7,5 (m,4H) ; 6,9 (d,2H) ; 3,5 (m,1H) ; 3,4 (s,3H) ; 3,2 (m,1H) ; 2,4 (m,2H) ; 2,2 (s,6H) ; 1,9 (s,3H) ; 1,8 (m,2H) |
| 66 | 8,0 (m,1H) ; 7,2 (m,3H) ; 6,8 (d,2H) ; 3,6 (m,1H) ; 3,1 (s,3H) ; 2,8 (m,1H) ; 2,3 (m,2H) ; 2,2 (s,6H) ; 2,0 (s,3H) ; 1,7 (m,2H) |
| 67 | 7,3-6,6 (m,6H) ; 3,5 (m,1H) ; 3,3 (s,3H) ; 3,05 (m,1H) ; 2,3 (m,2H) ; 2,15 (s,6H) ; 1,9 (s,3H) ; 1,8 (m,2H) |
| 68 | 6,85 (d,2H) ; 6,45 (s,2H) ; 3,8 (s,3H) ; 3,75 (s,6H) ; 3,7 (m,1H) ; 3,3 (s,3H) ; 3,0 (m,1H) ; 2,4 (m,2H) ; 2,25 (s,6H) ; 1,9 (s,3H) ; 1,8 (m,2H) |
| 69 | 7,2-6,5 (m,6H) ; 3,65 (s,3H) ; 3,5 (m,1H) ; 3,15 (s,3H) ; 2,9 (m,1H) ; 2,3 (m,2H) ; 2,15 (s,6H) ; 1,85 (s,3H) ; 1,8 (m,2H) |

Tableau VIII (suite)

| Exemple n° | $^1H$ RMN - $Cl_3CD$ |
|---|---|
| 70 | 7,25 (s,4H); 6,85 (d,2H); 5,65 (s,1H); 3,5 (m,2H); 3,40 (s,3H); 2,35 (m,2H); 2,2 (s,6H); 1,8 (m,2H) |
| 71 | 6,8 (d,2H); 6,55 (s,2H); 6,6 (s,1H); 3,75 (s,9H); 3,55 (m,2H); 3,45 (s,3H); 2,3 (m,2H); 2,2 (s,6H); 1,8 (m,2H) |
| 72 | 7,4 (q,4H); 6,85 (d,2H); 3,6 (m,1H); 3,25 (s,3H); 3,0 (m,1H); 2,4 (m,2H); 2,25 (s,6H); 1,9 (s,3H); 1,8 (m,2H) |
| 73 | 7,6 (s,1H); 7,4 (s,2H); 6,8 (d,2H); 6,7 (s,1H); 3,6 (m,2H); 3,4 (s,3H); 2,4 (m,2H); 2,15 (s,6H); 1,9 (m,2H) |
| 74 | 7,5 (q,4H); 6,85 (d,2H); 5,65 (s,1H); 3,55 (m,2H); 3,45 (s,3H); 2,4 (m,2H); 2,25 (s,6H); 1,8 (m,2H) |
| 75 | 7,3-6,7 (m,6H); 5,7 (s,1H); 3,8 (s,3H); 3,65 (m,2H); 3,55 (s,3H); 2,4 (m,2H); 2,25 (s,6H); 1,9 (m,2H) |
| 76 | 7,6 (s,1H); 7,4 (m,3H); 6,8 (d,2H); 3,5 (m,1H); 3,2 (s,3H); 2,9 (m,1H); 2,4 (m,2H); 2,25 (s,6H); 1,9 (m,2H); 1,5-0,5 (m,7H) |

Tableau VIII (suite)

| Exemple n° | $^1H$ RMN - $Cl_3CD$ |
|---|---|
| 77 | 7,2 (s,4H) ; 6,9 (d,2H) ; 3,6 (m,3H) ; 3,0 (m,1H) ; 2,3 (m,2H) ; 2,2 (s,6H) ; 1,9 (s,3H) ; 1,7 (m,2H) ; 1,5-0,5 (m,7H) |
| 78 | 6,8 (d,2H) ; 6,4 (s,2H) ; 3,75 (s,3H) ; 3,65 (s,6H) ; 3,6 (m,9H) ; 3,6-2,5 (m,6H) ; 2,2 (s,6H) ; 1,6-0,4 (m,16H) |
| 79 | 7,9 (m,1H) ; 7,15 (m,3H) ; 6,8 (d,2H) ; 3,45 (m,2H) ; 3,0-2,2 (m,4H) ; 2,2 (s,6H) ; 2-0,5 (m,18H) |
| 80 | 7,9 (m,1H) ; 7,4 (m,2H) ; 6,8 (dd,2H) ; 3,4 (m,3H) ; 2,7 (m,2H) ; 2,3 (m,3H) ; 2,1 (d,6H) ; 1,9-0,5 (m,16H) |
| 81 | 7,4 (q,4H) ; 6,8 (d,2H) ; 5,6 (s,1H) ; 3,5 (m,4H) ; 2,2 (m,2H) ; 2,05 (s,6H) ; 1,8-0,5 (m,9H) |
| 82 | 7,3 (s,4H) ; 6,9 (d,2H) ; 5,6 (s,1H) ; 3,4 (m,5H) ; 2,45 (m,6H) ; 2-12 (m,8H) |
| 83 | 7,4 (q,4H) ; 7,85 (d,2H) ; 3,6 (m,1H) ; 3,2 (s,3H) ; 3,0 (m,1H) ; 2,3 (m,6H) ; 1,9 (s,3H) ; 1,4 (m,8H) |

Tableau VIII (suite)

| Exemple n° | $^1H$ RMN - $Cl_3CD$ |
|---|---|
| 84 | 8,0 (d,1H); 7,2 (m,3H); 6,8 (d,2H); 3,4 (m,1H); 3,0 (s,3H); 2,8 (m,1H); 2,3 (m,4H); 2,15 (s,6H); 1,8-0,5 (m,9H) |
| 85 | 7,4-6,6 (m,5H); 3,4 (m,1H); 3,2 (s,3H); 2,9 (1H); 2,3 (m,4H); 2,15 (s,6H); 1,9-0,5 (m,2H) |
| 86 | 7,3 (m,2H); 6,8 (m,3H); 3,6 (m,1H); 3,2 (s,3H); 2,9 (m,1H); 2,3 (m,4H); 2,2 (s,3H); 1,8 (s,3H) |
| 87 | 7,5-6,9 (m,3H); 6,8 (d,2H); 5,6 (s,1H); 3,5 (m,2H); 3,4 (s,3H); 2,3 (m,2H); 2,1 (s,6H); 1,8 (m,2H) |
| 88 | 7,6-6,7 (m,5H); 3,4 (m,1H); 2,7 (m,1H); 2,4 (m,4H); 2,15 (s,6H); 1,9-0,3 (m,14H) |
| 89 | 7,1 (s,4H); 6,95 (s,1H); 6,85 (s,1H); 3,6 (m,4H); 2,3 (m,4H); 2,1 (s,6H); 2,05 (m,4H); 1,8 (m,6H); 1,3 (m,8H) |
| 90 | 7,2 (s,4H); 6,9 (s,1H); 6,85 (s,1H); 3,6 (m,4H); 2,3 (m,4H); 2,1 (s,6H); 2,05 (m,4H); 1,8 (m,6H); 1,4 (m,10H) |

Tableau VIII (suite)

| Exemple n° | $^1H$ RMN - $Cl_3CD$ |
|---|---|
| 91 | 7,2-6,6 (m,6H); 3,4 (m,1H); 3,1-2,5 (m,8H); 2,5-1,5 (m,14H); 1,0 (m,4H) |
| 92 | 7,25 (m,6H); 5,9 (m,1H); 5,4 (s,1H); 3,95 (t,2H); 3,40 (t,2H); 2,25 (t,2H); 2,1 (s,6H); 1,85-0,5 (m,9H) |
| 93 | 7,45 (d,1H); 7,2 (s,4H); 6,3 (d,1H); 3,8 (m,3H); 3,1 (m,1H); 2,35 (t,2H); 2,15 (s,6H); 1,8 (s,3H); 1,9-0,6 (m,9H) |
| 94 | 7,3 (s,6H); 5,95 (d,1H); 5,45 (s,1H); 3,75 (s,3H); 3,5 (t,2H); 2,35 (t,2H); 2,15 (s,6H); 1,8 (m,2H) |
| 95 | 7,4 (d,1H); 7,2 (s,5H); 6,35 (d,1H); 3,55 (m,2H); 3,4 (s,3H); 2,35 (t,2H); 2,2 (s,6H); 1,95-1,6 (m,5H) |
| 96 | 7,15 (s,5H); 6,0 (s,1H); 3,4 (m,1H); 3,25 (s,3H); 3,0 (m,1H); 2,2 (m,11H); 1,6 (m,5H) |
| 97 | 7,3 (s,5H); 5,75 (s,1H); 5,35 (s,1H); 3,7 (s,3H); 3,45 (t,2H); 2,35 (t,2H); 2,15 (s,9H); 1,75 (m,2H) |

Tableau VIII (suite)

| Exemple n° | $^1$H RMN - $Cl_3CD$ |
|---|---|
| 98 | 7,4-7,1 (m,5H); 5,7 (s,1H); 5,5 (s,1H); 3,8 (s,3H); 3,5 (t,2H); 2,3 (m,2H); 2,2 (s,9H); 1,8 (m,2H) |
| 99 | 7,4-7,2 (m,5H); 5,9 (s,1H); 5,4 (s,1H); 3,7 (s,3H); 3,5 (t,2H); 2,3 (m,2H); 2,15 (s,6H); 1,8 (m,2H) |
| 100 | 7,3 (b,6H); 7,1 (s,1H); 5,3 (s,1H); 3,7 (b,3H); 3,7-3,3 (m,2H); 2,3 (m,2H); 2,2 (b,6H); 1,8 (m,2H) |
| 101 | 7,3 (b,5H); 7,1 (s,1H); 5,2 (s,1H); 3,8 (s,3H); 3,6 (m,6H); 2,4 (m,6H); 1,9 (m,2H) |
| 102 | 7,3 (b,5H); 7,1 (s,1H); 5,3 (s,1H); 3,8 (s,3H); 3,5 (m,2H); 2,5 (m,6H); 1,8 (m,6H) |
| 103 | 7,3 (s,6H); 6,0 (s,1H); 5,4 (s,1H); 3,7 (s,3H); 3,5 (t,2H); 2,3 (m,6H); 1,9 (m,2H), 1,5 (m,6H) |
| 104 | 7,3 (b,6H); 6,0 (b,1H); 5,4 (s,1H); 3,7 (s,3H); 3,6 (m,2H); 2,5 (m,6H); 1,8 (m,6H) |

Méthode C (exemples 105 et suivants)

Exemple 110 :

Préparation de 1H-imidazole-2-méthanol, 1-méthyl-α-3-trifluorométhylphényle.

A une solution de 3,6 g de méthanone, 1H-imidazole-1-méthyl-2-yl-3-trifluorométhylphényle dans 25 ml de méthanol, on ajoute 0,6 g d'hydrure de bore et de sodium. On agite pendant 30 minutes et on ajoute

150 ml d'eau. On filtre le précipité obtenu et on lave à l'eau. On recristallise dans un mélange éthanol/eau.

On obtient ainsi 3,2 g (58 %) de cristaux blancs avec p.f. 125-6 ° C qui correspond à 1H-imidazole-2-méthanol, 1-méthyl-α-3-trifluorométhylphényle.

Les composés identifiés par les exemples 105 à 118, 150, 152, 154, 159 et 161 à 167 sont obtenus par la même méthode de préparation décrite dans l'exemple 110.

Les données pour l'identification des produits 105 à 117 sont présentées dans le tableau IX.

Les données pour l'identification des produits 105, 152, 154, 159 et 162 à 163 sont présentées dans le tableau XI, celles des produits 164 à 167 dans le tableau XII, et celles des produits 118 et 161 sont présentées dans le tableau XIII.

Tableau IX

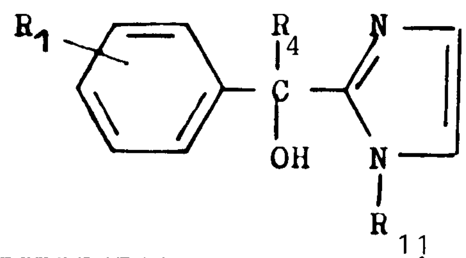

| Exemple n° | $R_1$ | $R_4$ | $R_{11}$ | P.f. | Solvant recrist. | Méthode |
|---|---|---|---|---|---|---|
| 105 | H | H | H | 202-3ºC | $EtOH/H_2O$ | C |
| 106 | 4 Cl | H | H | 196-7ºC | $EtOH/H_2O$ | C |
| 107 | 4 Cl | H | Me | 137-9ºC | $MeOH/H_2O$ | C |
| 108 | 3 Cl | H | Me | 126-8ºC | $MeOH/H_2O$ | C |
| 109 | 4 F | H | Me | 112-5ºC | $MeOH/H_2O$ | C |
| 110 | 3 $F_3C$- | H | Me | 125-6ºC | $EtOH/H_2O$ | C |
| 111 | 4 $F_3C$- | H | Me | 124-5ºC | $MeOH/H_2O$ | C |
| 112 | 3<br>4 tri MeO<br>5 | H | Me | 160-1ºC | $MeOH/H_2O$ | C |

**Tableau IX** (Suite)

| Exemple n° | $R_1$ | $R_4$ | $R_{11}$ | P.f. | Solvant recrist. | Méthode |
|---|---|---|---|---|---|---|
| 113 | 3 4 di Cl | H | Me | 157-9ºC | $EtOH/H_2O$ | C |
| 114 | 4 $F_3C$- | H | n-Bu | 111-2ºC | $EtOH/H_2O$ | C |
| 115 | 2 4 di Cl | H | n-Bu | 94-7ºC | $EtOH/H_2O$ | C |
| 116 | 4 Cl | H | n-Bu | 108-110ºC | Ether/Hexane | C |
| 117 | 3 4 5 tri MeO | H | n-Bu | 122-5ºC | $EtOH/H_2O$ | C |
| 118 | 3 4 5 tri Meo | H | n-dodècyle | huile | - | C |

Méthode D (exemples 119 et suivants)

Exemple 121 :

Préparation de 1H-imidazole-2-méthanol, α-(4-chlorophényl)-α-méthyl-1-méthyle.

A un réactif de Grignard formé à partir de 10 g de magnésium métallique et de 57 g d'iodure d'éthyle dans l'éther éthylique on ajoute lentement une solution de 44 g de méthanone, 4-chlorophényl-(1-méthyl-imidazole)-2-yle dans 50 ml d'éther. On chauffe à reflux pendant 3 heures on laisse refroidir et on hydrolyse avec une solution aqueuse de chlorure d'ammonium.

On filtre et on lave à l'eau et on recristallise dans un mélange éthanol/eau.

On obtient ainsi 40,1 g (85 %) de cristaux blancs avec p.f. 191-2° ce qui correspond à 1H-imidazole-2-méthanol, α-(4-chlorophényl)-α-méthyl-1-méthyle.

Les composés identifiés par les exemples 119 à 149 et 169 à 175 sont obtenus par la même méthode de préparation décrite dans l'exemple 121.

Les données pour l'identification des produits 119 à 149 et 169 à 175 sont présentées dans les tableaux X à XII, et celles des produits 148 et 170 dans le tableau XIII.

<u>**Tableau X**</u>

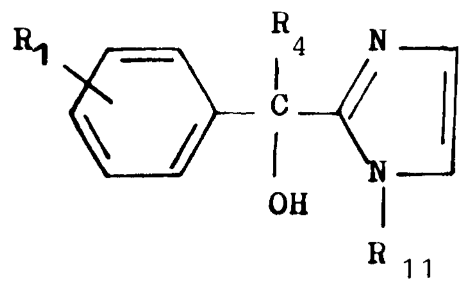

| Exemple n° | $R_1$ | $R_4$ | $R_{11}$ | P.f. | Solvant recrist. | Méthode |
|---|---|---|---|---|---|---|
| 119 | 3 Cl | n-Bu | Me | 125-6°C | $EtOH/H_2O$ | D |
| 120 | 3 Cl | Me | Me | 180-1°C | $EtOH/H_2O$ | D |
| 121 | 4 Cl | Me | Me | 191-2°C | $EtOH/H_2O$ | D |
| 122 | 4 Cl | 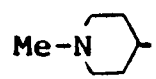 | Me | 183-4°C | AcOEt/Ether | D |
| 123 | 4 Cl | Et | Me | 166-8°C | $EtOH/H_2O$ | D |
| 124 | 4 Cl | n-Bu | Me | 120-2°C | $EtOH/H_2O$ | D |
| 125 | 4 Cl | 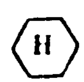 | Me | 161-2°C | EtOH | D |
| 126 | 2 Cl | Me | Me | 181-2°C | $EtOH/H_2O$ | D |

Tableau X (suite)

| Exemple n° | $R_1$ | $R_4$ | $R_{11}$ | P.f. | Solvant recrist. | Méthode |
|---|---|---|---|---|---|---|
| 127 | 2 Cl | n-Bu | Me | 138-41ºC | Ether | D |
| 128 | 3 $F_3C$- | Me | Me | 193-4ºC | EtOH/$H_2O$ | D |
| 129 | 3 $F_3C$- | n-Bu | Me | 140-1ºC | EtOH/$H_2O$ | D |
| 130 | 3 $F_3C$- | 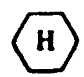 | Me | 156-7ºC | Heptane/Ether | D |
| 131 | 4 $F_3C$- | Me | Me | 167-8ºC | EtOH/$H_2O$ | D |
| 132 | 4 F | Me | Me | 176-7ºC | EtOH/$H_2O$ | D |
| 133 | 4 Me-O | Me | Me | 181-2ºC | EtOH/$H_2O$ | D |
| 134 | 3 4 di Cl | Me | Me | 207-8ºC | EtOH/$H_2O$ | D |
| 135 | 3 4 di Cl | n-Bu | Me | 142-4ºC | ACN | D |

ACN = acétonitrile

Tableau X (suite)

| Exemple n° | $R_1$ | $R_4$ | $R_{11}$ | P.f. | Solvant recrist. | Méthode |
|---|---|---|---|---|---|---|
| 136 | 3 4 di Cl | 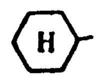 | Me | 158-60ºC | $ACN/H_2O$ | D |
| 137 | 3 4 5 tri MeO | $CH_3$ | Me | 181-2ºC | $EtOH/H_2O$ | D |
| 138 | 4 Cl | Me | n-Bu | 174-5ºC | EtOH | D |
| 139 | 4 Cl | n-Bu | n-Bu | 134-5ºC | ACN | D |
| 140 | 4 Cl | 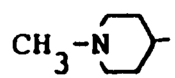 | n-Bu | 184-5ºC | AcOEt/Ether | D |
| 141 | 3 4 5 tri MeO | n-Bu | n-Bu | 125-6ºC | $EtOH/H_2O$ | D |
| 142 | 2 Cl | n-Bu | n-Bu | 132-3ºC | Ether | D |
| 143 | 3 $F_3$ C | Et | n-Bu | 164-5ºC | $EtOH/H_2O$ | D |

**Tableau X** (suite)

| Exemple n° | $R_1$ | $R_4$ | $R_{11}$ | P.f. | Solvant recrist. | Méthode |
|---|---|---|---|---|---|---|
| 144 | 2,4 di Cl | n-Bu | n-Bu | 142-3ºCH | $ACN/H_2O$ | D |
| 145 | 4 Cl | Me | 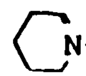$N-CH_2)_2-$ | 136-7ºC | $EtOH/H_2O$ | D |
| 146 | 4 Cl | Me | Me Me $N-(CH_2)_3-$ | 147-8ºC | $EtOH/H_2O$ | D |
| 147 | 3,4,5 tri MeO | n-Bu | n-Dodècyl | 75-7ºC | AcOEt | D |
| 148 | 3 $F_3$ C- | n-Bu | Ø-$CH_2$- | huile | - | D |
| 149 | 4 Cl | Me | Ø-$CH_2$- | 154-5ºC | Bz/Ether | D |

Ø = phényle
Bz = benzène

**Tableau XI**

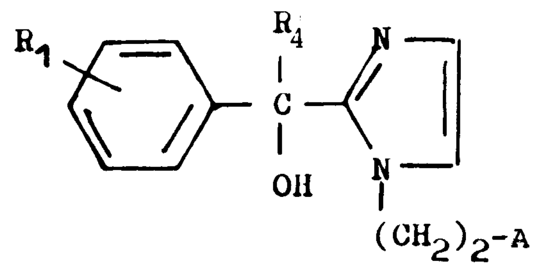

| Exemple n° | $R_1$ | $R_4$ | A | P.f. | Solvant recrist. | Méthode |
|---|---|---|---|---|---|---|
| 150 | 4 Cl | H | CN | 134-5ºC | $EtOH/H_2O$ | C |
| 151 | 4 Cl | H | $NH_2$ | 187-8ºC | $EtOH/H_2O$ | I |
| 152 | 3 Cl | H | $CO_2H$ | 212-3ºC | $MeOH/H_2O$ | C |
| 153 | 4 Cl | H | $CH_2OH$ | 121-2ºC | $MeOH/H_2O$ | I |
| 154 | 4 Cl | H | $CO_2Me$ | 96-7ºC | $MeOH/H_2O$ | C |
| 155 | H | H | $CH_2OH$ | 110-1ºC | Ether | I |
| 156 | 4 Me | H | $CH_2OH$ | 104-5ºC | Ether | I |
| 157 | 4 MeO- | H | $CH_2OH$ | huile | - | I |

Tableau XI (suite)

| Exemple n° | $R_1$ | $R_4$ | A | P.f. | Solvant recrist. | Méthode |
|---|---|---|---|---|---|---|
| 158 | 3 4 di Cl | H | $CH_2OH$ | 138-9ºC | Ether | I |
| 159 | H | H | $CO_2Me$ | 93-6ºC | $MeOH/H_2O$ | C |
| 160 | 4 Cl | H | $(CH_2)_2$-OH | 131-2ºC | Ether | I |
| 161 | 4 Cl | H | $CH_2$-CN | huile | - | C |
| 162 | 4 Cl | H | $CH_2$-$CO_2H$ | $>$300ºC | - | C |
| 163 | 4 Cl | H | $CH_2$-$CO_2Me$ | 85-7ºC | Ether | C |

Méthode E (exemples 164 et suivants)

Exemple 164 :

Préparation de 1H-pyrazole-5-méthanol, 1-butyl-α-phényle.

Dans un tricol pourvu d'un agitateur, d'un réfrigérant, d'un embout d'addition et d'un thermomètre on met une solution de 12,4 g de 1-butylpyrazole dans 100 ml d'éther absolu, on refroidit avec de la glace et on additionne lentement 6,1 g de solution de butyllithium 1,6 M dans l'hexane. On agite pendant 30 minutes et on additionne lentement une solution de 14,5 g de p-chlorobenzaldéhyde dans 30 ml d'éther absolu. Après la réaction, on laisse en agitation jusqu'à ce que le mélange atteigne la température ambiante et on hydrolyse avec 100 ml d'eau. On extrait avec du benzène, on lave, on sèche sur du sulfate de sodium et on élimine le solvant à vide.

On obtient ainsi 18,9 g (82 %) d'une huile qu'on peut cristalliser d'un mélange méthanol/eau, p.f. 46-7 °C et qui est l'1H-pyrazole-5-méthanol, 1-butyl-α-phényle.

Les composés identifiés par les exemples 164 à 167 et 177 à 178 sont obtenus par la même méthode de préparation décrite.

Les données pour l'identification de ces produits sont présentées dans les tableaux XII et XIII.

Méthode F

Exemple 168 :

Préparation de 1H-pyrazole-5-méthanol, 4-bromo-1,α-diméthyle

On additionne goutte à goutte une solution de 0,9 g de brome dans 3 ml de chloroforme, à une solution de 1 g de 1H-imidazole-2-méthanol, α-phényle dans 10 ml de chloroforme. On laisse à température ambiante pendant 4 heures et on élimine le solvant sous vide. On reprend le résidu dans l'éthanol et on ajoute de l'eau. On filtre et on recristallise dans un mélange éthanol/eau. On obtient ainsi 1,3 g (92 %) de cristaux jaune de 1H-pyrazole-5-méthanol, 4-bromo-1,α-diméthyle, de p.f. 116 °C.

Les données pour l'identification des produits 168 et 176 se trouvent dans le tableau XII.

Tableau XII

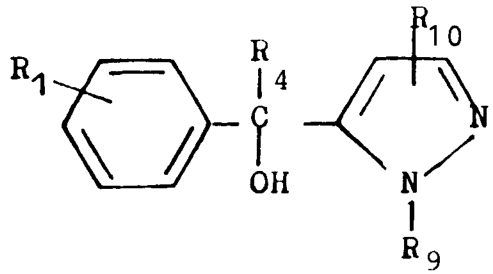

| Exemple n° | $R_1$ | $R_4$ | $R_9$ | $R_{10}$ | P.f. | Solvant recrist. | Méthode |
|---|---|---|---|---|---|---|---|
| 164 | H | H | n-Bu | H | 47-8ºC | MeOH/$H_2O$ | C/E |
| 165 | 4 Cl | H | Me | H | 94-7ºC | MeOH/$H_2O$ | C/E |
| 166 | 3 4 5 tri Meo | H | Me | H | huile | - | C/E |
| 167 | 3 4 5 tri Meo | H | n-Bu | H | huile | - | C/E |
| 168 | H | H | Me | 4-Br | 110-11ºC | EtOH/$H_2O$ | F |
| 169 | 4 Cl | Ø | Me | H | 167-8ºC | Bz/ACN | D |

Bz = benzène Ø = phényle

Tableau XII (suite)

| Exemple n° | $R_1$ | $R_4$ | $R_9$ | $R_{10}$ | P.f. | Solvant recrist. | Méthode |
|---|---|---|---|---|---|---|---|
| 170 | 4 Cl | Me | n-Bu | H | huile | - | D |
| 171 | H | Me | Me | H | 99-100ºC | Ether/Hex | D |
| 172 | 3 4 5 tri MeO | Me | Me | H | 144-5ºC | Bz/Ether | D |
| 173 | H | Me | Me | 3 Me | 137-8ºC | $EtOH/H_2O$ | D |
| 174 | H | $CH_2$=CH- | Me | H | 95-6ºC | Ether/Pen | D |
| 175 | 4 Cl | $CH_2$=CH- | n-Bu | H | 84-5ºC | Ether/Pen | D |

Hex = hexane
Pen = pentane
Bz = benzène
Ø = phényle

Tableau XII (suite)

| Exemple n° | $R_1$ | $R_4$ | $R_9$ | $R_{10}$ | P.f. | Solv nt Recrist. | Méthode |
|---|---|---|---|---|---|---|---|
| 176 | H | H | Me | 4 Cl | huile | - | F |
| 177 | 2-Me | H | Me | H | 113-4ºC | $EtoH/H_2O$ | C/E |
| 178 | 3 Cl | H | Me | H | 128-9ºC | Ether | C/E |
| 179 | 4 Me | H | Me | H | 123-6ºC | Ether | C |
| 180 | 2 Cl | H | Me | H | 96-8ºC | Ether | C |
| 181 | 4 MeO | H | Me | H | 129-30ºC | Ether | C |

Méthode G (exemples 182 et suivants)

Exemple 182 :

Préparation d'iodure de 1H-imidazole-2-méthanol, α-(4-chlorophényl)-α-méthyl-1-méthyl-O-(2-triméthylammonium)éthyle.

On chauffe une solution de 2,9 g de 1H-imidazole-2-méthano, α-(4-chlorophényl)-α-méthyl-1-méthyle et 1,3 g d'iodure de méthyle dans 20 ml d'acétone, à 70 °C, dans un récipient clos, pendant 70 heures. On refroidit et on élimine le solvant sous vide. On obtient ainsi 4,2 g (100 % Rdt.) d'iodure de 1H-imidazole-2-méthanol, α-(4-chlorophényl)-α-méthyl-1-méthyl-0-(2-triméthylammonium)éthyle de p.f. 55 °C.

Les composés identifiés par les exemples 182 à 185 sont obtenus par la même méthode de préparation décrite.

Les données pour l'identification des produits 182 à 185 sont présentées dans les tableaux XIV et XV.

Méthode H

Exemple 186 :

Préparation d'iodure de 1,1-diméthyl-imidazolium 2-méthanol-(4-chlorophényl)-α-méthyle.

Une solution de 1,4 g de 1H-imidazole-2-méthanol, α-(4-méthylphényl)-α-méthyl-1-méthyle et 0,9 g d'iodure de méthyle dans 30 ml d'acétone est chauffée pendant 48 heures à 60 °C dans un récipient clos. On refroidit, on évapore sous vide et on obtient 2,2 g d'iodure de 1,1-diméthylimidazolium-2-méthanol, α-(4-chlorophényl)-α-méthyle, p.f. 45 °C.

Les données pour l'identification de ce produit sont présentées dans les tableaux XIV et XV.

Méthode I (exemples 151, 153, 155 et suivants)

Exemple 153 :

Préparation de 1H-imidazole, 2-méthanol, (1-butan-4-ol), α-(4-chlorophényl).

A une solution de méthanone, 1H-imidazole-(1-méthylbutanoate),-(4-chlorophényl) 2,9 g (0,01 M) dans l'éther (100 ml) on ajoute avec précaution l'hydrure d'aluminium et de lithium (0,8 g, 0,02 M) et on maintient à reflux pendant 3 heures.

On évapore l'éther et on reprend avec du chloroforme, on lave à l'eau, on sèche et on évapore. On obtient ainsi 1,8 g de produit qu'on recristallise dans un mélange méthanol/eau.

Les composés identifiés par les exemples 155 à 158 et 160 sont obtenus par la même méthode et les données pour son identification sont exposées dans le tableau XI et celles du produit 157 dans le tableau XIII.

Tableau XIII

| Exemple n° | $^{1}H$ RMN - $Cl_3CD$ |
|---|---|
| 118 | 6,8 (d,2H); 6,5 (s,2H); 4,6 (b,1H); 3,7 (m,11H); 2,4 (m,2H); 1,5-0,6 (m,22H) |
| 148 | 7,9-6,1 (m,10H); 4,9 (m,1H); 2,5 (t,2H); 1,5-0,5 (m,9H) |
| 161 | 8,0 (b,1H); 7,2 (s,4H); 6,8 (s,1H); 5,9 (s,1H); 5,2 (s,1H); 4,8 (t,2H); 2,3-1,6 (m,4H) |
| 166 | 7,3 (d,1H); 6,5 (s,2H); 5,9 (s,1H); 5,7 (s-1H); 3,6 (m,12H) |
| 167 | 7,2 (d,1H); 6,5 (s,2H); 5,9 (s,1H); 5,7 (s,1H); 3,7 (m,11H); 1,7-0,4 (m,7H) |
| 176 | 7,3 (s,6H); 5,8 (s,1H); 3,6 (m,5H); 2,6 (t,2H); 2,3 (s,6H) |

Tableau XIV

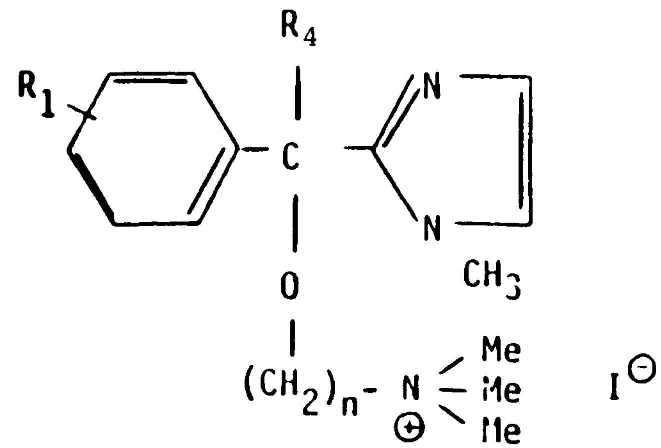

| Exemple n° | $R_1$ | $R_4$ | H | P.F. | Rdt. | Méthode |
|---|---|---|---|---|---|---|
| 182 | 4 Cl | Me | 2 | 55 | Quant. | G |
| 183 | 4 Cl | Me | 3 | 60 | Quant. | G |
| 184 | 4 MeO | Me | 2 | 50 | Quant. | G |
| 185 | 4 MeO | Me | 3 | 50 | Quant. | G |

Tableau XIV (suite)

| Exemple n° | $R_1$ | $R_4$ | H | P.f. | Rdt. | Méthode |
|---|---|---|---|---|---|---|
| 186 | 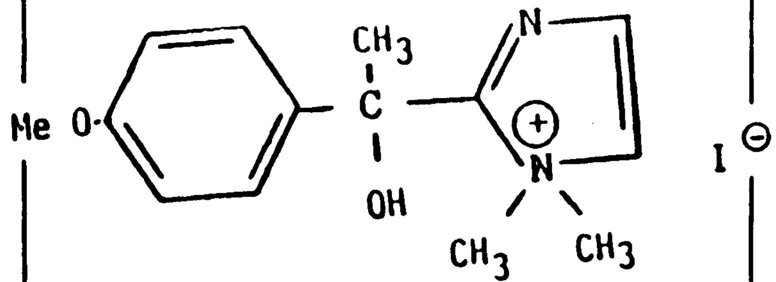 | | | 45 | Quant. | H |

Tableau XV

| N° Pat | $^1$H RMN - $Cl_3CD$ |
|---|---|
| 182 | 7,2 (q,4H); 6,9 (d,2H); 4,1 (m,2H); 3,6 (s,9H); 3,4 (m,2H); 3,25 (s,3H); 2,0 (s,3H) |
| 183 | 7,25 (q,4H); 6,9 (d,2H); 3,8 (m,2H); 3,5 (s,9H); 3,2 (s,3H); 3,1 (m,2H); 2,2 (m,2H); 1,8 (s,3H) |
| 184 | 6,9 (m,6H); 4,1 (m,2H); 3,8 (s,3H); 3,6 (s,9H); 3,3 (m,2H); 3,2 (s,3H); 1,9 (s,3H) |
| 185 | 7,3-6,7 (m,6H); 3,8 (m,5H); 3,4 (s,9H); 3,2 (m,5H); 2,15 (m,5H) |
| 186 | 8,0-6,8 (m,6H); 4,0 (s,3H); 3,8 (m,3H) |

Activité analgésique : Inhibition des contorsions induites par le bromure d'acétylcholine chez la souris

Méthode de Collier (Collier, H.O.W.; Dinneen, L.C.; Johnson, C.A. et Schneider, C.; Br. J. Pharmac. Chemother, 32: 295 (1968))

On utilise des souris Swiss, mâles, de 17-22 rammes de poids. On utilise des groupes de 4 animaux comme minimum.

Les contorsions sont induites par injection i.p. de bromure d'acétylcholine (7,2 mg/kg, dissous dans du sérum physiologique en administrant un volume de 10 ml/kg de poids), au début de l'expérience, et on

compte le nombre de contorsions pendant 5 minutes, qui seront celles de référence pour chaque animal (on rejette les souris avec moins de 2 contorsions). Immédiatement après, on administre les produits étudiés par voie orale, à la dose de 160 mg/kg, mis en suspension ou dissous dans de la gomme arabique à 5 % (p/v) dans l'eau distillée. 40 et 120 minutes après le traitement, on administre à nouveau le bromure d'acétylcholine, on note les contorsions et on évalue les lectures à 40 et 120 minutes, en prenant comme référence les contorsions de la lecture initiale. La moyenne des valeurs à 40 et 120 minutes nous donne le pourcentage d'inhibition des contorsions qui définit l'activité analgésique des produits étudiés.

Les résultats obtenus avec certains des produits décrits sont résumés dans le Tableau XVI.

Tableau XVI

Activité analgésique : Inhibition des contorsions induites par le bromure d'acétylcholine chez la souris

Dose de produit : 160 mg/kg, p.o.

| Produit | % inhibition des contorsions |
|---|---|
| 2 | 100,0 |
| 4 | 95,0 |
| 5 | 100,0 |
| 6 | 75,0 |
| 7 | 95,0 |
| 8 | 95,0 |
| 9 | 97,5 |
| 14 | 85,0 |
| 59 | 100,0 |
| 60 | 82,5 |
| 61 | 97,5 |
| 64 | 100,0 |
| 68 | 95,0 |
| 69 | 95,0 |
| 70 | 100,0 |
| 72 | 100,0 |
| 73 | 100,0 |
| 74 | 100,0 |
| 75 | 100,0 |
| 76 | 100,0 |
| 77 | 100,0 |
| 78 | 100,0 |
| 82 | 83,8 |
| 83 | 88,3 |
| 107 | 57,5 |
| 110 | 57,5 |
| 111 | 87,5 |
| 117 | 60,0 |
| 119 | 90,0 |
| 121 | 70,0 |
| 126 | 65,0 |
| 127 | 70,0 |
| 128 | 82,5 |
| 129 | 55,0 |
| 133 | 52,5 |
| 137 | 85,0 |
| 141 | 62,5 |
| 142 | 80,0 |
| 144 | 88,8 |
| Acide acétylsalicylique | 92,5 |
| Paracétamol | 72,5 |
| Néfopam | 100,0 |

Activité analgésique : Inhibition des contorsions induites par la phénylbenzoquinone chez la souris

Méthode de Siegmund (Siegmund, E; Cadmus, R. et Lu, G.: Proc. Soc. Exp. Biol. Med., 95: 729 (1957)).

On utilise des souris Swiss, mâles, de 17-22 grammes de poids. On utilise des groupes de 4 animaux comme minimum.

Les contorsions sont induites par injection i.p. de phényl-p-benzoquinone (25 ml/kg d'une solution à 0,02 % en solution d'éthanol à 5 % (v/v) dans l'eau distillée, avec du bleu d'Evans en proportion de 0,1 % (p/v) et on les comptes pendant 15 minutes à partir du moment de l'injection. Chaque produit est mis en suspension ou dissous dans la gomme arabique à 5 %(p/v) dans l'eau distillée et on l'administre par voie orale, à la dose de 160 mg/kg, 60 minutes avant l'injection de phénylbenzoquinone. On détermine l'inhibition des contorsions due à chaque produit, en prenant comme référence les contorsions d'un groupe d'animaux témoins, qui ne reçoivent que le véhicule par voie orale, 60 minutes avant la phénylbenzoquinone.

Les résultats obtenus azvec certains des produits décrits sont résumés dans le tableau XVII.

Tableau XVII

Activité analgésique : Inhibition des contorsions induites par phénylbenzoquinone chez la souris

Dose de produit : 160 mg/kg p.o.

| Produit | % Inhibition des contorsions |
|---|---|
| 18 | 82,5 |
| 19 | 87,5 |
| 20 | 25,0 |
| 27 | 40,0 |
| 30 | 27,5 |
| 31 | 80,0 |
| 32 | 37,5 |
| 33 | 35,0 |
| 34 | 40,0 |
| 37 | 77,5 |
| 38 | 42,5 |
| 39 | 37,5 |
| 86 | 98,8 |
| 87 | 77,5 |
| 91 | 57,5 |
| 94 | 40,0 |
| 95 | 45,0 |
| 113 | 57,5 |
| 115 | 39,5 |
| 116 | 27,5 |
| 128 | 67,5 |
| 146 | 28,5 |
| Acide acétylsalicylique | 75,0 |
| Paracétamol | 47,5 |
| Néfopam | 97,5 |

Activité analgésique : Inhibition des contorsions induites par acide acétique chez la souris

Méthode de Koster (Koster, R.; Anderson, M. et De Beer, E.J.: Fed. Proc. 18 ; 412 (1959)).

On utilise des souris Swiss, mâles, de 17-22 grammes de poids. On utilise des groupes de 4 animaux pour chaque dose comme minimum.

Les contorsions sont induites par injection i.p. d'acide acétique (25 ml/kg d'une solution de 5,25 mg/ml d'acide acétique dans l'eau distillée, avec du bleu d'Evans en proportion de 0,1 % p/v) et on les compte pendant 15 minutes à partir du moment de l'injection. Chaque produit est mis en suspension ou dissous dans la gomme arabique à 5 % (p/v) dans l'eau distillée et on l'administre par voie orale, à la dose de 160 mg/kg, 60 minutes avant l'injection de l'acide acétique. On détermine l'inhibition des contorsions due à

chaque produit, en prenant comme référence les contorsions d'un groupe d'animaux témoins, qui ne reçoivent que le véhicule par voie orale, 60 minutes avant l'acide acétique.

Les résultats obtenus avec certains des produits décrits sont résumés dans le Tableau XVIII.

Tableau XVIII

Activité analgésique : Inhibition des contorsions induites par l'acide acétique chez la souris

Dose de produit : 160 mg/kg, p.o.

| Produit | % Inhibition des contorsions |
|---|---|
| 6 | 50,0 |
| 7 | 67,5 |
| 12 | 82,5 |
| 31 | 80,0 |
| 37 | 72,5 |
| 59 | 87,5 |
| 60 | 62,5 |
| 64 | 50,0 |
| 86 | 50,0 |
| 115 | 50,0 |
| 119 | 45,0 |
| 128 | 52,5 |
| Acide acétylsalicylique | 72,5 |
| Paracétamol | 20,0 |
| Néfopam | 98,0 |

Compte tenu de leurs bonnes propriétés pharmacodynamiques, les dériviés d'aryl-hétéroarylcarbinols selon l'invention, peuvent être utilisés de façon satisfaisante en thérapeutique humaine et animale, en particulier dans le traitement des douleurs d'intensité modérée à forte comme par exemple : sciatique, lumbago, dorsalgie, entorses, fractures, luxations, douleurs post-opératoires de tout genre, douleurs d'origine dentaire.

En thérapeutique humaine, la dose d'administration des dérivés de la présente invention est bien sûr fonction de la gravité de l'affection à traiter. Elle sera généralement comprise entre environ 90 et environ 270 mg/jour. Les dérivés de l'invention seront par exemple administrés sous la forme de comprimés, de solutions ou suspensions injectables.

On indiquera ci-après, à titre d'exemples, deux formes galéniques particulières des dérivés objet de la présente invention.

| Exemple de formule par comprimé | |
|---|---|
| Citrate de 1H-pyrazole-5-méthanol, 1-méthyl-O-(2-diméthylamino)éthyle | 30,00 mg |
| Lactose | 119,50 mg |
| Amidon de maïs | 46,00 mg |
| Cellulose microcristalline | 23,00 mg |
| Povidone K-90 | 4,60 mg |
| Amidon prégélatinisé | 4,60 mg |
| Dioxyde de silice colloïdale | 1,15 mg |
| Stéarate de magnésium | 1,15 mg |
| Poids comprimé | 230,00 mg |

| Exemple de formule par ampoule injectable | |
|---|---|
| Citrate de 1H-pyrazole-5-méthanol, 1-méthyl-O-(2-diméthylamino)éthyle | 20,00 mg |
| Dextrose | 50,00 mg |
| Eau pour injection q.s.p. | 1,00 mg |

**Revendications**

**1.** Dérivés d'aryl-hétéroaryl carbinols répondant à la formule générale I

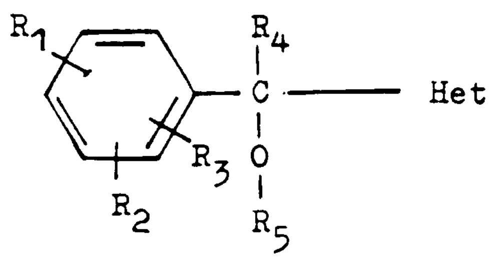

I

ainsi que leurs sels thérapeutiquement acceptables, dans laquelle

| | |
|---|---|
| $R_1$, $R_2$ et $R_3$, | identiques ou différents, représentent un atome d'hydrogène, un halogène, un radical alkyle inférieur, un radical alcoxy inférieur, ou un groupe trifluorométhyle, |
| $R_4$ | représente un atome d'hydrogène, un radical alkyle inférieur en $C_1$ à $C_4$, un cycloalkyle, un radical alkényle inférieur, ou un cycloalkylamino substitué sur l'atome d'azote. |
| $R_5$ | représente un atome d'hydrogène ou un radical de formule |

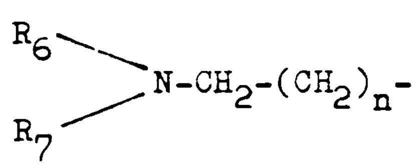

dans laquelle n peut avoir les valeurs 1 ou 2 et le groupement

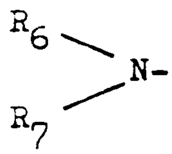

peut avoir l'une des significations suivantes : groupe dialkylamino inférieur, ou un radical d'amine cyclique choisi parmi les groupes pipéridino, morpholino ou pyrrolidino,

$R_5$ représente aussi un groupement de formule générale

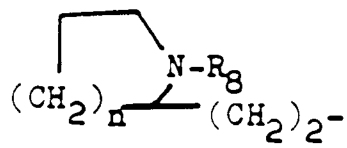

dans laquelle n peut avoir la valeur 1 ou 2 et dans laquelle $R_8$ est un alkyle inférieur.

<u>Het</u> représente un azole choisi parmi les suivants :

a) Pyrazole de formule générale

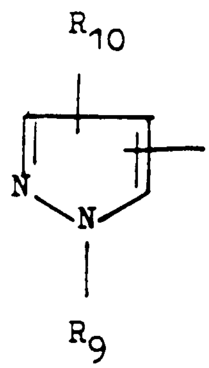

dans laquelle $R_9$ représente l'une des significations suivantes : un radical alkyle inférieur en $C_1$ à $C_4$, le radical dodécyle, le radical benzyle ou un radical du type

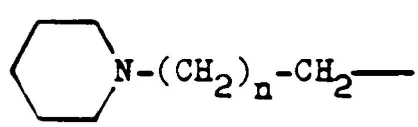

dans laquelle n = 1 ou 2.

$R_{10}$ représente un atone d'hydrogène, un radical méthyle ou un atome d'halogène,

b) imidazole de formule générale

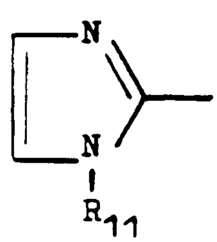

dans laquelle $R_{11}$ représente l'un quelconque des groupements suivants : un atome d'hydrogène, un radical alkyle inférieur en $C_1$ à $C_4$, un radical dodécyle, un radical de formule générale

$A-(CH_2)_n-$

dans laquelle n = 2, 3 ou 4 et A représente l'un quelconque des groupements suivants : un radical pipéridine, un groupement cyano, un radical hydroxy, un radical carboxy, un groupement amino ou un groupement ester méthylique,
à l'exception toutefois des composés de formule générale I dans laquelle Het représente un Imidazole, $R_5$ représente un atome d'hydrogène et $R_{11}$ représente un atome d'hydrogène, une chaîne hydrocarbonnée contenant de 1 à 10 atomes de carbone, un radical aralkyle ou un radical aryle,
et à l'exception des composés suivants :
1H-pyrazole-5-méthanol-1-méthyl-α-phényl, et
1H-pyrazole-5-méthanol-1-méthyl-α-(4-méthyl-phényl),
c) Het peut représenter un imidazole de formule

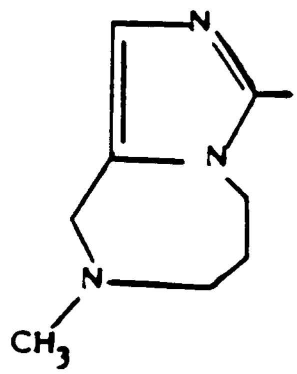

**2.** Les composés répondant à la formule générale I, selon la revendication 1, caractérisés en ce qu'ils sont choisis parmi :

| | |
|---|---|
| 1 | IH—imidazole-2-méthanol, 1-méthyl-α-phényl-O-(2-diméthylamino)éthyle |
| 2 | 1H-imidazole-2-méthanol, α-(4-chlorophényl)-α-méthyl-1-méthyl-O-(2-diméthylamino)éthyle |
| 3 | 1H-imidazole-2-méthanol, α-(4-chlorophényl)-1-méthyl-O-(2-diméthylamino)éthyle |
| 4 | 1H-imidazole-2-méthanol, α-(3-chlorophényl)-1-méthyl-O-(2-diméthylamino)éthyle |
| 5 | 1H-imidazole-2-méthanol, α-(2-chlorophényl)-1-méthyl-O-(2-diméthylamino)éthyle |
| 6 | 1H-imidazole-2-méthanol, α-(4-fluorophényl)-α-méthyl-1-méthyl-O-(2-diméthylamino)éthyle |

| | |
|---|---|
| 7 | 1H-imidazole-2-méthanol, α-méthyl-1-méthyl-α-(3-trifluorométhylphényl)-O-(2-diméthylamino)éthyle |
| 8 | 1H-imidazole-2-méthanol, α-(3-chlorophényl)-α-méthyl-1-méthyl-O-(2-diméthylamino)éthyle |
| 9 | 1H-imidazole-2-méthanol, α-(butyl-α-(3-chlorophényl)-1-méthyl-O-(2-diméthylamino) éthyle |
| 10 | 1H-imidazole-2-méthanol, 1-butyl-α-(4-chlorophényl)-α-méthyl-O-(2-diméthylamino)éthyle |
| 11 | 1H-imidazole-2-méthanol, α-(4-méthoxyphényl)-α-méthyl-1-méthyl-O-(2-diméthylamino)éthyle |
| 12 | 1H-imidazole-2-méthanol-α-(3-chlorophényl)-α-méthyl-1-méthyl-O-éthyl-2-(N-pyrrolidine) |
| 13 | 1H-imidazole-2-méthanol-α-butyl-1-dodécyle-α-(3,4,5-triméthoxyphényl)-O-(2-diméthylamino)-éthyle |
| 14 | 1H-imidazole-2-méthanol, 1-butyl-α-(4-trifluorométhylphényl)-O-(2-diméthylamino)éthyle |
| 15 | 1H-imidazole-2-méthanol, 1-méthyl-α-méthyl-α-(3-trifluorométhylphényl)-O-éthyl-2-(N-pipéridine) |
| 16 | 1H-imidazole-2-méthanol, α-cyclohexyl-α-(3,4-dichlorophényl)-1-méthyl-O-(2-diméthylamino)-éthyle |
| 17 | 1H-imidazole-2-méthanol, α-butyl-α-(3,4-dichlorophényl)-1-méthyl-O-(2-diméthylamino)-éthyle |
| 18 | 1H-imidazole-2-méthanol, α-(3,4-dichlorophényl)-α-méthyl-1-méthyl-O-(2-diméthylamino)-éthyle |
| 19 | 1H-imidazole-2-méthanol-α-(3,4-dichlorophényl)-1-méthyl-O-(2-diméthylamino)éthyle |

| | |
|---|---|
| 20 | 1H-imidazole-2-méthanol, α-(4-chlorophényl)-1-[éthyl-2-(N-pipéridine)]-O-(2-diméthylamino)éthyle |
| 21 | 1H-imidazole-2-méthanol, α-(4-chlorophényl)-α-méthyl-1-[propyl-3-(N-pipéridine)]-O-(2-diméthylamino)éthyle |
| 22 | 1H-imidazole-2-méthanol, 1-(3-cyanopropyl)-α-(4-chlorophényl)-O-(2-diméthylamino)éthyle |
| 23 | 1H-imidazole-2-méthanol, α-(4-chlorophényl)-1-méthyl-α-[(N-méthylpipéridine)-4-yl]-O-(2-diméthylamino)éthyle |
| 24 | 1H-imidazole-2-méthanol-α-(4-chlorophényl)-1-benzyl-O-(N-méthyl-N-benzyl-2-amino)éthyle |
| 25 | 1H-imidazole[1,5-a][1,4]diazépine, 6,7,8,9-tétrahydro-2-méthanol, α-(4-chlorophényl)-α-méthyl-7-méthyl-O-(2-diméthylamino)éthyle |
| 26 | 1H-imidazole[1,5-a][1,4]diazépine, 6,7,8,9-tétrahydro-2-méthanol, α-(4-chlorophényl)-7-méthyl-O-(2-diméthylamino)éthyle |
| 27 | 1H-pyrazole-5-méthanol, 1-butyl-α-(phényl)-O-(2-diméthylamino) éthyle |
| 28 | 1H-pyrazole-5-méthanol, α-(4-chlorophényl)-1-méthyl-α-phényl-O-(2-diméthylamino)éthyle |
| 29 | 1H-pyrazole-5-méthanol, 1-butyl-α-(3,4,5-triméthoxyphényl)-O-(2-diméthylamino)éthyle |
| 30 | 1H-pyrazole-5-méthanol, 1-butyl-α-(4-chlorophényl)-α-méthyl-O-(2-diméthylamino)éthyle |
| 31 | 1H-pyrazole-5-méthanol, 1-méthyl -α-(phényl)-O-(2-diméthylamino)éthyle |
| 32 | 1H-pyrazole-5-méthanol, α-méthyl-1-méthyl-α-(phényl)-O- (2-diméthylamino)éthyle |
| 33 | 1H-pyrazole-5-méthanol, 1-méthyl-α-(3,4,5-triméthoxyphényl)-O-(2-diméthylamino)éthyle |

| | |
|---|---|
| 34 | 1H-pyrazole-5-méthanol, 1-méthyl- α-(phényl)-O-ethyl-2-(1-pyrrolidinyl) |
| 35 | 1H-pyrazole-5-méthanol, 1-méthyl- α-(phényl)-O-éthyl-(2-N-morpholinyl) |
| 36 | 1H-pyrazole-5-méthanol, α-méthyl-1-méthyl-α-(3,4,5-triméthoxyphényl)-O-(2-diméthylamino)éthyle |
| 37 | 1H-pyrazole-5-méthanol, 4-bromo-1-méthyl-α-(phényl)-O-(2-diméthylamino)éthyle |
| 38 | 1H-pyrazole-5-méthanol, 1,3-diméthyl-α-(phényl)-α-méthyl-O-(2-diméthylamino)éthyle |
| 39 | 1H-pyrazole-5-méthanol, 1,3-diméthyl-α-(phényl)-O-(2-diméthylamino)éthyle |
| 40 | 1H-pyrazole-5-méthanol, 1-méthyl-α-(2-méthyl-phényl)-O-(2-diméthylamino)éthyle |
| 41 | 1H-pyrazole-5-méthanol, 1-méthyl-4-chloro-α-(4-chlorophényl)-O-(2-diméthylamino)éthyle |
| 42 | 1H-pyrazole-4-méthanol, 1-méthyl-α-(4-chloro-phényl)-O-(2-diméthylamino)éthyle |
| 43 | 1H-pyrazole-4-méthanol, 1-méthyl-α-méthyl-α-(4-chlorophényl)-O-(2-diméthylamino)éthyle |
| 44 | 1H-pyrazole-5-méthanol, 1-méthyl-α-(4-chloro-phényl)-O-(2-diméthylamino)éthyle |
| 45 | 1H-pyrazole-5-méthanol, 1-méthyl-α-(3-chloro-phényl)-O-(2-diméthylamino)éthyle |
| 46 | 1H-pyrazole-5-méthanol, 1-méthyl-α-(4-méthyl-phényl)-O-(2-diméthylamino)éthyle |
| 47 | 1H-pyrazole-5-méthanol, 1-méthyl-α-(2-chloro-phényl)-O-(2-diméthylamino)éthyle |
| 48 | 1H-pyrazole-5-méthanol, 1-méthyl, α-phényl-O-2(N-pipéridino)éthyle |
| 49 | 1H-pyrazole-5-méthanol, 1-méthyl, α-phényl-O-éthyl-2-(N-propylpipéridine-2-yl) |

| | |
|---|---|
| 50 | 1H-pyrazole-4-méthanol, 1-méthyl-α-(4-chlorophényl)-O-éthyl-2(N-propylpipéridine-2-yl) |
| 51 | 1H-pyrazole-5-méthanol, 1-méthyl-α-phényl-O-éthyl-2(N-éthylpipéridine-2-yl) |
| 52 | 1H-pyrazole-5-méthanol, 1-méthyl-α-phényl-O-éthyl-2(N-méthylpyrrolidine-2-yl) |
| 53 | 1H-pyrazole-5-méthanol, 1-méthyl-α-phényl-O-(2-diisopropylamino)éthyle |
| 54 | 1H-pyrazole-4-méthanol-1-méthyl-α-(4-chlorophényl)-O-éthyl-2(N-méthylpipéridine-2-yl) |
| 55 | 1H-pyrazole-4-méthanol-1-méthyl-α-(4-chlorophényl)-O-éthyl-2(N-éthylpipéridine-2-yl) |
| 56 | 1H-pyrazole-5-méthanol-1-méthyl-α-phényl-O-éthyl-2-(N-méthylpipéridine-2-yl) |
| 57 | 1H-pyrazole-4-méthanol-1-méthyl-α-(4-chlorophényl)-O-(2-diisopropylamino)éthyle |
| 58 | 1H-pyrazole-4-méthanol-1-méthyl-α-(4-chlorophényl)-O-éthyl-2-(N-méthylpyrrolidine-2-yl) |
| 59 | 1H-imidazole-2-méthanol, α-(4-chlorophényl)-α-méthyl-1-méthyl-O-(3-diméthylamino)propyle |
| 60 | 1H-imidazole-2-méthanol, α-(3-chlorophényl)-1-méthyl-O-(3-diméthylamino)propyle |
| 61 | 1H-imidazole-2-méthanol, α-(4-chlorophényl)-α-éthyl-1-méthyl-O-(3-diméthylamino)propyle |
| 62 | 1H-imidazole-2-méthanol, α-butyl-α-(3-chlorophényl)-1-méthyl-O-(3-diméthylamino)propyle |
| 63 | 1H-imidazole-2-méthanol, α-(4-chlorophényl)-α-cyclohexyl-1-méthyl-O-(3-diméthylamino)-propyle |
| 64 | 1H-imidazole-2-méthanol, α-(4-fluorophényl)-α-méthyl-1-méthyl-O-(3-diméthylamino)propyle |
| 65 | 1H-imidazole-2-méthanol, α-méthyl-1-méthyl-α-(3-trifluorométhylphényl)-O-(3-diméthylamino)propyle |

| | |
|---|---|
| 66 | 1H-imidazole-2-méthanol, α-(2-chlorophényl)-α-méthyl-1-méthyl-O-(3-diméthylamino)propyle |
| 67 | 1H-imidazole-2-méthanol-α-(3-chlorophényl)-α-méthyl-1-méthyl-O-(3-diméthylamino)propyle |
| 68 | 1H-imidazole-2-méthanol, α-méthyl-1-méthyl-α-(3,4,5-triméthoxyphényl)-O-(3-diméthyl-amino)propyle |
| 69 | 1H-imidazole-2-méthanol, α-méthyl-1-méthyl-α-(4-méthoxyphényl)-O-(3-diméthylamino)pro-pyle |
| 70 | 1H-imidazole-2-méthanol, α-(4-chlorophényl-1-méthyl-O-(3-diméthylamino)propyle |
| 71 | 1H-imidazole-2-méthanol, 1-méthyl-α-(3,4,5-triméthoxyphényl-O-(3-diméthylamino)propyle |
| 72 | 1H-imidazole-2-méthanol, α-méthyl-1-méthyl-α-(4-trifluorométhylphényl)-O-(3-diméthyl-amino)-propyle |
| 73 | 1H-imidazole-2-méthanol, 1-méthyl-α-(3-trifluorométhylphényl)-O-(3-diméthylamino)-propyle |
| 74 | 1H-imidazole-2-méthanol, 1-méthyl-α-(4-trifluorométhylphényl)-O-(3-diméthylamino)-propyle |
| 75 | 1H-imidazole-2-méthanol, α-(4-méthoxyphényl)-1-méthyl-O-(3-diméthylamino)propyle |
| 76 | 1H-imidazole-2-méthanol, α-butyl-1-méthyl-α-(3-trifluorométhylphényl)-O-(3-diméthyl-amino)propyle |
| 77 | 1H-imidazole-2-méthanol, 1-butyl-α-(4-chlorophényl)-α-méthyl-O-(3-diméthylamino)-propyle |
| 78 | 1H-imidazole-2-méthanol, 1-butyl-α-butyl-α-(3,4,5-triméthoxyphényl)-O-(3-diméthylamino)-propyle |

| | |
|---|---|
| 79 | 1H-imidazole-2-méthanol, 1-butyl-α-butyl-α-(2-chlorophényl)-O-(3-diméthylamino)-propyle |
| 80 | 1H-imidazole-2-méthanol, 1-butyl-α-butyl-α-(2,4-dichlorophényl)-O-(3-diméthylamino)-propyle |
| 81 | 1H-imidazole-2-méthanol, 1-butyl-α-(4-trifluorométhylphényl)-O-(3-diméthylamino)-propyle |
| 82 | 1H-imidazole-2-méthanol, α-(4-chlorophényl)-1-méthyl-O-3(N-pipéridino)propyle |
| 83 | 1H-imidazole-2-méthanol, α-méthyl-1-méthyl-α-(4-trifluorométhylphényl)-O-3-(N-pipéri-dine)-propyle |
| 84 | 1H-imidazole-2-méthanol, α-butyl-α-(2-chloro-phényl)-1-méthyl-O-(3-diméthylamino)propyle |
| 85 | 1H-imidazole-2-méthanol, α-butyl-α-(3,4-dichlorophényl)-1-méthyl-O-(3-diméthylamino)-propyle |
| 86 | 1H-imidazole-2-méthanol, α-(3,4-dichlorophé-nyl)-α-méthyl-1-méthyl-O-(3-diméthylamino)-propyle |
| 87 | 1H-imidazole-2-méthanol, α-(3,4-dichloro-phényl)-1-méthyl-O-(3-diméthylamino)propyle |
| 88 | 1H-imidazole-2-méthanol, α-cyclopropyl-α-(3,4-dichlorophényl)-1-méthyl-O-(3-diméthyl-amino)propyle |
| 89 | 1H-imidazole-2-méthanol, α-(4-chlorophényl)-1-[éthyl-2(N-pipéridino)]-α-méthyl-O-(3-diméthylamino)propyle |
| 90 | 1H-imidazole-2-méthanol, α-(4-chlorophényl)-α-méthyl-1-[propyl-3(N-pipéridino)]-O-(3-diméthylamino)propyle |

| | |
|---|---|
| 91 | 1H-imidazole-2-méthanol, α-(4-chlorophényl)-1-méthyl-[(N-méthylpipéridine)-4-yl]-O-(3-diméthylamino)propyle |
| 92 | 1H-pyrazole-5-méthanol, 1-butyl-α-(phényl)-O-(3-diméthylamino) propyle |
| 93 | 1H-pyrazole-5-méthanol, 1-butyl-α-(4-chlorophényl)-α-méthyl-O-(3-diméthylamino)propyle |
| 94 | 1H-pyrazole-5-méthanol, 1-méthyl-α-(phényl)-O-(3-diméthylamino)propyle |
| 95 | 1H-pyrazole-5-méthanol, 1-méthyl-α-(phényl)-α-méthyl-O-(3-diméthylamino)propyle |
| 96 | 1H-pyrazole-5-méthanol, 1,3-diméthyl-α-(phényl)-α-méthyl-O-(3-diméthylamino)propyle |
| 97 | 1H-pyrazole-5-méthanol, 1,3-diméthyl-α-(phényl)-O-(3-diméthylamino)propyle |
| 98 | 1H-pyrazole-5-méthanol, 1-méthyl-α-(2-méthylphényl)-O-(3-diméthylamino)propyle |
| 99 | 1H-pyrazole-5-méthanol, 1-méthyl-α-(4-chlorophényl)-O-(3-diméthylamino)propyle |
| 100 | 1H-pyrazole-4-méthanol, 1-méthyl-α-phényl-O-(3-diméthylamino)propyle |
| 101 | 1H-pyrazole-4-méthanol, 1-méthyl-α-(4-chlorophényl)-O-(3-N-morpholino)propyle |
| 102 | 1H-pyrazole-4-méthanol, 1-méthyl-α-(4-chlorophényl)-O-(3-N-pyrrolidino)propyle |
| 103 | 1H-pyrazole-5-méthanol, 1-méthyl-α-phényl-O-(3-N-pipéridino)propyle |
| 104 | 1H-pyrazole-5-méthanol, 1-méthyl-α-phényl-O-(3-N-pyrrolidino)propyle |
| 105 | 1H-imidazole-2-méthanol, α-phényle |
| 106 | 1H-imidazole-2-méthanol, α-(4-chlorophényl) |
| 107 | 1H-imidazole-2-méthanol, α-(4-chlorophényl)-1-méthyle |

| | |
|---|---|
| 108 | 1H-imidazole-2-méthanol, α-(3-chlorophényl)-1-méthyle |
| 109 | 1H-imidazole-2-méthanol, α-(4-fluorophényl)-1-méthyle |
| 110 | 1H-imidazole-2-méthanol, 1-méthyl-α-(3-trifluorométhylphényl) |
| 111 | 1H-imidazole-2-méthanol, 1-méthyl-α-(4-trifluorométhylphényl) |
| 112 | 1H-imidazole-2-méthanol, 1-méthyl-α-(3,4,5-triméthoxyphényl) |
| 113 | 1H-imidazole-2-méthanol, α-(3,4-dichlorophényl)-1-méthyl |
| 114 | 1H-imidazole-2-méthanol, 1-butyl-α-(4-trifluorométhylphényl) |
| 115 | 1H-imidazole-2-méthanol, 1-butyl-α-(2,4-dichlorophényl) |
| 116 | 1H-imidazole-2-méthanol, 1-butyl-α-(4-chlorophényl) |
| 117 | 1H-imidazole-2-méthanol-1-butyl-α-(3,4,5-triméthoxyphényl) |
| 118 | 1H-imidazole-2-méthanol, 1-dodécyl-α-(3,4,5-triméthoxyphényl) |
| 119 | 1H-imidazole-2-méthanol, α-butyl-α-(3-chlorophényl)-1-méthyle |
| 120 | 1H-imidazole-2-méthanol, α-(3-chlorophényl)-α-méthyl-1-méthyle |
| 121 | 1H-imidazole-2-méthanol, α-(4-chlorophényl)-α-méthyl-1-méthyle |
| 122 | 1H-imidazole-2-méthanol, α-(4-chlorophényl)-α-[(N-méthyl-péridine)4-yl]-1-méthyle |
| 123 | 1H-imidazole-2-méthanol, α-(4-chlorophényl)-α-éthyl-1-méthyle |

| | |
|---|---|
| 124 | 1H-imidazole-2-méthanol, α-butyl-α-(4-chlorophényl)-1-méthyle |
| 125 | 1H-imidazole-2-méthanol, α-(4-chlorophényl)-α-cyclohexyl-1-méthyle |
| 126 | 1H-imidazole-2-méthanol, α-(2-chlorophényl)-α-méthyl-1-méthyle |
| 127 | 1H-imidazole-2-méthanol, α-butyl-α-(2-chlorophényl)-1-méthyle |
| 128 | 1H-imidazole-2-méthanol, α-méthyl-1-méthyl-α-(3-trifluorométhylphényl) |
| 129 | 1H-imidazole-2-méthanol, α-butyl-1-méthyl-α-(3-trifluorométhylphényl) |
| 130 | 1H-imidazole-2-méthanol, α-cyclohexyl-1-méthyl-α-(3-trifluorométhylphényl) |
| 131 | 1H-imidazole-2-méthanol, α-méthyl-1-méthyl-α-(4-trifluorométhylphényl) |
| 132 | 1H-imidazole-2-méthanol, α-(4-fluorophényl)-α-méthyl-1-méthyle |
| 133 | 1H-imidazole-2-méthanol, α-(4-méthoxyphényl)-α-méthyl-1-méthyle |
| 134 | 1H-imidazole-2-méthanol, α-(3,4-dichlorophényl)-α-méthyl-1-méthyle |
| 135 | 1H-imidazole-2-méthanol, α-butyl-α-(3,4-dichlorophényl)-1-méthyle |
| 136 | 1H-imidazole-2-méthanol, α-cyclohexyl-α-(3,4-dichlorophényl)-1-méthyle |
| 137 | 1H-imidazole-2-méthanol, α-méthyl-1-méthyl-α-(3,4,5-triméthoxyphényl) |
| 138 | 1H-imidazole-2-méthanol, 1-butyl-α-(4-chlorophényl)-α-méthyle |
| 139 | 1H-imidazole-2-méthanol, 1-butyl-α-butyl-α-(4-chlorophényl) |

| | |
|---|---|
| 140 | 1H-imidazole-2-méthanol, 1-butyl-α-(4-chlorophényl)-α-[(N-méthylpipéridine)-4-yl] |
| 141 | 1H-imidazole-2-méthanol, 1-butyl-α-butyl-α-(3,4,5-triméthoxyphényl) |
| 142 | 1H-imidazole-2-méthanol, 1-butyl-α-butyl-α-(2-chlorophényl) |
| 143 | 1H-imidazole-2-méthanol, 1-butyl-α-éthyl-α-(3-trifluorométhylphényl) |
| 144 | 1H-imidazole-2-méthanol, 1-butyl-α-butyl-α-(2,4-dichlorophényl) |
| 145 | 1H-imidazole-2-méthanol, α-(4-chlorophényl)-α-méthyl-1-(N-pipéridino)propyle |
| 146 | 1H-imidazole-2-méthanol, α-(4-chlorophényl)-1-(3-diméthylamino)propyl-α-méthyle |
| 147 | 1H-imidazole-2-méthanol, α-butyl-1-dodécyl-α-(3,4,5-triméthoxyphényl) |
| 148 | 1H-imidazole-2-méthanol, 1-benzyl-α-butyl-α-(3-trifluorométhylphényl) |
| 149 | 1H-imidazole-2-méthanol, 1-benzyl-α-(4-chlorophényl)-α-méthyle |
| 150 | 1H-imidazole-2-méthanol-1-(2-cyanoéthyl)-α-(4-chlorophényl) |
| 151 | 1H-imidazole-2-méthanol-1-(3-aminopropyl)-α-(4-chlorophényl) |
| 152 | 1H-imidazole-2-méthanol-1-(propane-3-carboxylique)-α-(3-chlorophényl) |
| 153 | 1H-imidazole-2-méthanol-1-(3-hydroxypropyl)-α-(4-chlorophényl) |
| 154 | (1H-imidazole-2-méthanol-1-(3-méthylpropanoate)-α-(4-chlorophényl) |
| 155 | 1H-imidazole-2-méthanol-1-(3-hydroxypropyl)-α-phényle |

| | |
|---|---|
| 156 | 1H-imidazole-2-méthanol-1-(3-hydroxypropyl)-α-(4-méthylphényl) |
| 157 | 1H-imidazole-2-méthanol-1-(3-hydroxypropyl)-α-(4-méthoxyphényl) |
| 158 | 1H-imidazole-2-méthanol-1-(3-hydroxypropyl)-α-(3,4-dichlorophényl) |
| 159 | 1H-imidazole-2-méthanol-1-(3-méthylpropanoate)-α-phényle |
| 160 | 1H-imidazole-2-méthanol-1-(4-hydroxybutyl)-α-(4-chlorophényl) |
| 161 | 1H-imidazole-2-méthanol-1-(3-cyanopropyl)-α-(4-chlorophényl) |
| 162 | 1H-imidazole-2-méthanol-1-(4-butyrique)-α-(4-chlorophényl) |
| 163 | 1H-imidazole-2-méthanol-1-(4-méthylbutyrate)-α-(4-chlorophényl) |
| 164 | 1H-pyrazole-5-méthanol, 1-butyl-α-phényle |
| 165 | 1H-pyrazole-5-méthanol, α-(4-chlorophényl)-1-méthyle |
| 166 | 1H-pyrazole-5-méthanol, 1-méthyl-α-(3,4,5-triméthoxyphényl) |
| 167 | 1H-pyrazole-5-méthanol, 1-butyl-α-(3,4,5-triméthoxyphényl) |
| 168 | 1H-pyrazole-5-méthanol, 4-bromo-1-méthyl-α-phényle |
| 169 | 1H-pyrazole-5-méthanol, α-(4-chlorophényl)-1-méthyl-α-phényle |
| 170 | 1H-pyrazole-5-méthanol, 1-butyl-α-(4-chlorophényl)-α-méthyle |
| 171 | 1H-pyrazole-5-méthanol, 1-méthyl-α-méthyl-α-phényle |
| 172 | 1H-pyrazole-5-méthanol, 1-méthyl-α-méthyl-α-(3,4,5-triméthoxyphényl) |
| 173 | 1H-pyrazole-5-méthanol, 1,3-diméthyl-α-méthyl-α-phényle |

| | |
|---|---|
| 174 | 1H-pyrazole-5-méthanol, α-éthényl-1-méthyl-α-phényle |
| 175 | 1H-pyrazole-5-méthanol, 1-butyl-α-(4-chlorophényl)-α-éthényle |
| 176 | 1H-pyrazole-5-méthanol, 4-chloro-1-méthyl-α-phényle |
| 177 | 1H-pyrazole-5-méthanol, 1-méthyl-α-(2-méthylphényl) |
| 178 | 1H-pyrazole-5-méthanol-1-méthyl-α-(3-chlorophényl) |
| 180 | 1H-pyrazole-5-méthanol-1-méthyl-α-(2-chlorophényl) |
| 181 | 1H-pyrazole-5-méthanol-1-méthyl-α-(4-méthoxyphényl) |
| 182 | 1H-imidazole-2-méthanol, α-(4-chlorophényl)-α-méthyl-1-méthyl-O-(2-triméthylammonium) éthyl, iodure |
| 183 | 1H-imidazole-2-méthanol, α-(4-chlorophényl)-α-méthyl-1-méthyl-O-(3-triméthylammonium) propyl, iodure |
| 184 | 1H-imidazole-2-méthanol, α-(4-méthoxyphényl)-α-méthyl-1-méthyl-O-(2-triméthylammonium) éthyl, iodure |
| 185 | 1H-imidazole-2-méthanol, α-(4-méthoxyphényl)-α-méthyl-1-méthyl-O-(3-triméthylammonium) propyl, iodure |
| 186 | 1,1-diméthylimidazolium-2-méthanol, α-(4-méthoxyphényl)-α-méthyl, iodure |

**3.** Procédé de préparation des composés selon l'une des revendications 1 ou 2, caractérisé par la mise en oeuvre d'au moins l'une des opérations suivantes :

3.1. Par réaction d'un composé de formule générale II

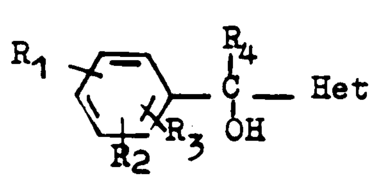

II

avec un composé de formule générale III

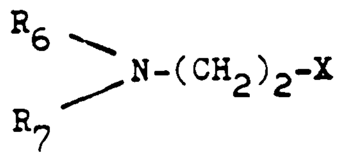 III

dans lesquelles $R_1$ à $R_4$, $R_6$ et $R_7$, X et Het ont les significations mentionnées dans la revendication 1.

3.2. Par réaction d'un composé de formule générale II avec un composé de formule générale IV

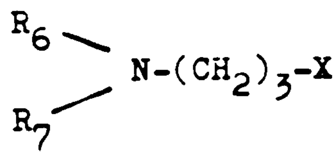 IV

dans laquelle $R_1$ à $R_4$, $R_6$ et $R_7$, X et Het ont les significations mentionnées dans la revendication 1.

**4.** Procédé de préparation de composés de formule générale II caractérisé par la mise en oeuvre d'au moins l'une des opérations suivantes :

4.1. Par réduction d'un composé de formule générale V

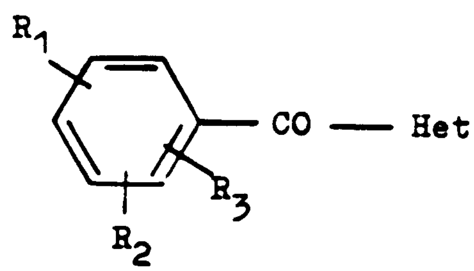 V

dans laquelle $R_1$, $R_2$, $R_3$ et Het ont les significations mentionnées dans la revendication 1.

4.2. Par réaction d'un composé de formule générale V avec des réactifs de Grignard du type

$R_4$ Mg X

dans laquelle $R_4$ a les significations mentionnées dans la revendication 1.

**5.** Procédé de préparation de composés de formule générale II caractérisé par la mise en oeuvre de l'opération suivante :

Réaction d'un aldéhyde de formule générale VI

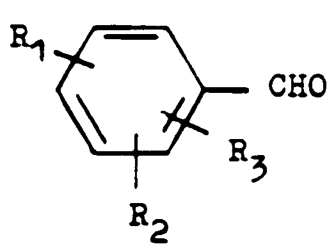 VI

avec des composés du type Het-Li dans laquelle $R_1$, $R_2$, $R_3$ ont les significations mentionnées dans la revendication 1, Het represente un pyrazole de formule générale mentionnée dans la revendication 1 et Li représente un atome de lithium.

**6.** Procédé de préparation de composés de formule générale VII

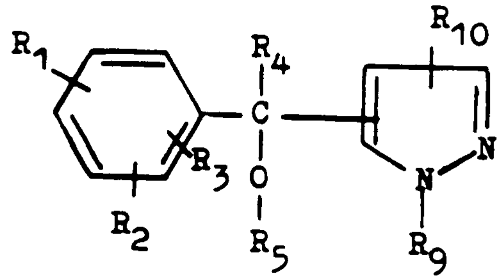

VII

dans laquelle $R_1$ à $R_9$ ont les significations mentionnées dans la revendication 1 et $R_{10}$ est du chlore ou du brome, par halogénation du composé précurseur de formule générale VII dans laquelle $R_1$ à $R_9$ ont les significations mentionnnées dans les revendications 1 à 10 et $R_{10}$ représente un atome d'hydrogène.

**7.** Procédé de préparation de composés de formule générale VIII

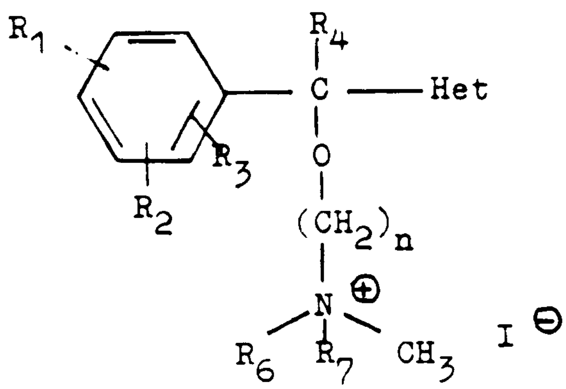

VIII

par réaction de composés de formule générale I

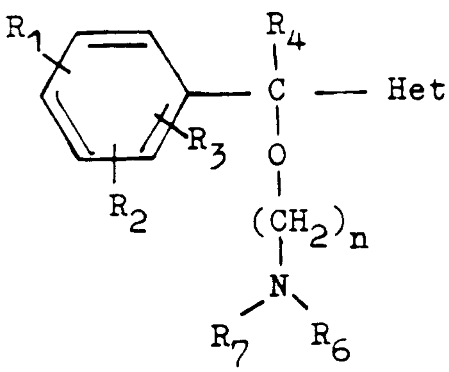

I

dans lesquelles $R_1$ à $R_4$, $R_6$, $R_7$ et Het ont les significations mentionnées dans la revendication 1, avec l'iodure de méthyle.

**8.** Procédé de préparation de composés de formule générale IX

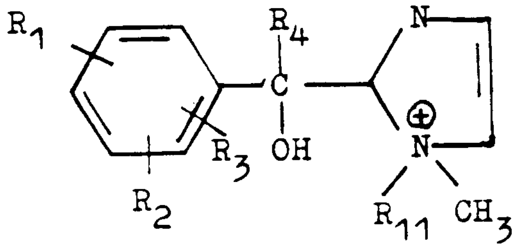

I ⊖ IX

par réaction de composés de formule générale X

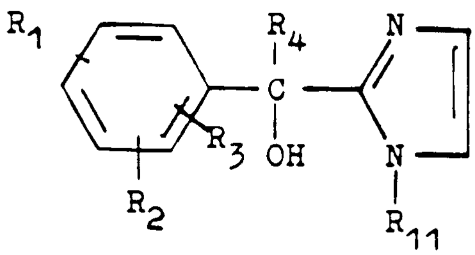

X

dans lesquelles $R_1$ à $R_2$ et $R_{11}$ ont les significations mentionnées dans la revendication 1, avec l'iodure de méthyle.

**9.** A titre de médicaments, les dérivés de formule générale I et leurs sels thérapeutiquement acceptables selon l'une des revendications 1 et 2, en particulier à titre de médicaments destinés au traitement de la douleur.

**10.** Compositions pharmaceutiques, caractérisées par le fait qu'elles contiennent, outre un support pharmaceutiquement acceptable, au moins un dérivé de formule générale I ou l'un de ses sels physiologiquement acceptables, selon l'une des revendications 1 et 2.

**11.** Utilisation des dérivés d'aryl-hétéroaryl carbinols répondant à la formule générale I

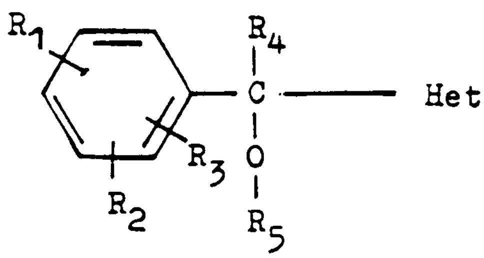

I

ainsi que leurs sels thérapeutiquement acceptablss, dans laquelle

| | |
|---|---|
| $R_1$, $R_2$ et $R_3$, | identiques ou différents, représentent un atome d'hydrogène, un halogène, un radical alkyle inférieur, un radical alcoxy inférieur, ou un groupe trifluorométhyle, |
| $R_4$ | représente un atome d'hydrogène, un radical alkyle inférieur en $C_1$ à $C_4$, un cycloalkyle, un radical alkényle inférieur, ou un cycloalkylamino substitué sur l'atome d'azote, |
| $R_5$ | représente un atome d'hydrogène ou un radical de formule |

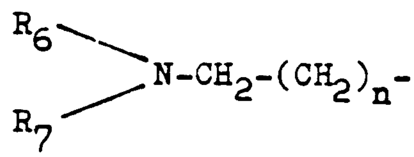

dans laquelle n peut avoir les valeurs 1 ou 2 et le groupement

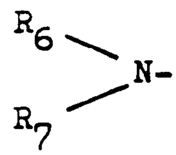

peut avoir l'uns des significations suivantes : groupe dialkylamino inférieur, ou un radical d'amine cyclique choisi parmi les groupes pipéridino, morpholino ou pyrrolidino,

$R_5$ représente aussi un groupement de formule générale

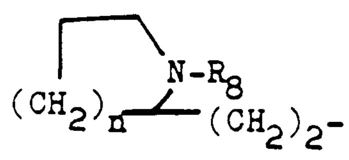

dans laquelle n peut avoir la valeur 1 ou 2 et dans laquelle $R_8$ est un alkyle inférieur.

Het représente un azole choisi parmi les suivants :

a) Pyrazole de formule générale

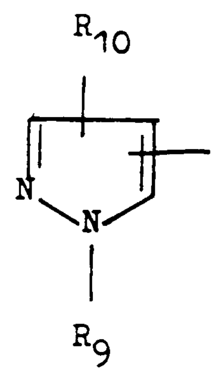

dans laquelle $R_9$ représents l'une des significations suivantes : un atome d'hydrogène, un radical alkyle inférieur en $C_1$ à $C_4$, le radical dodécyle, le radical benzyle ou un radical du type

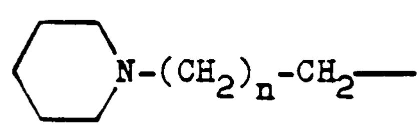

dans laquelle n = 1 ou 2.

$R_{10}$ représente un atome d'hydrogène un radical méthyle ou un atome d'halogène,

b) imidazole de formule générale

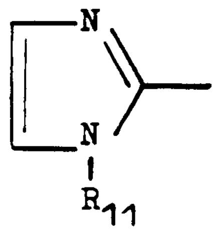

dans laquelle $R_{11}$ représente l'un quelconque des groupements suivants : un atome d'hydrogène, un radical alkyle inférieur en $C_1$ à $C_4$, un radical dodécyle, un radical de formule générale

$A-(CH_2)_n-$

dans laquelle n = 2, 3 ou 4 et A représente l'un quelconque des groupements suivants : un radical pipéridine, un groupement cyano, un radical hydroxy, un radical carboxy, un groupement amino ou un groupement ester méthylique,
c) Het peut aussi représenter un imidazols de formule

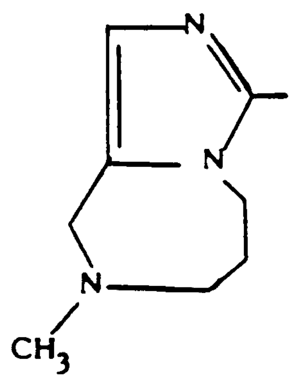

pour la fabrication de médicaments destinés au traitement de la douleur, en particulier pour la fabrication d'analgésiques.

**12.** A titre de médicament, le 1H-pyrazole-5-méthanol-1-méthyl-α-(4-méthyl-phényl)

**Claims**

**1.** Arylheteroarylcarbinol derivatives corresponding to the general formula I:

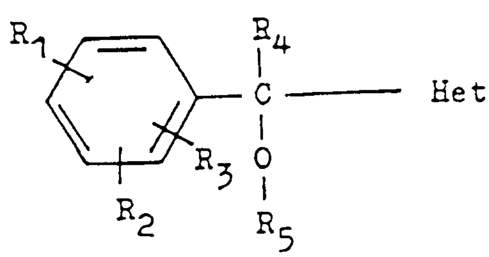

I

and their therapeutically acceptable salts, in which formula:
$R_1$, $R_2$ and $R_3$, which are identical or different, represent a hydrogen atom, a halogen, a lower

alkyl radical, a lower alkoxy radical or a trifluoromethyl group,

$R_4$ represents a hydrogen atom, a $C_1$ to $C_4$ lower alkyl radical, a cycloalkyl, a lower alkenyl radical or a cycloalkylamino substituted on the nitrogen atom,

$R_5$ represents a hydrogen atom or a radical of the formula:

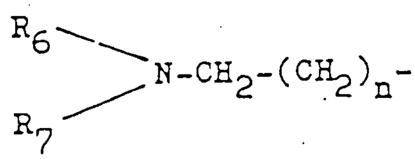

in which n can have the value 1 or 2 and the group:

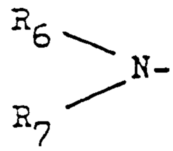

can have one of the following meanings: a lower dialkylamino group or a cyclic amine radical selected from piperidino, morpholino and pyrrolidino groups, or

$R_5$ represents a group of the general formula:

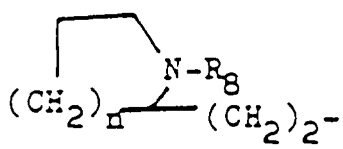

in which n can have the value 1 or 2 and in which $R_8$ is a lower alkyl, and

<u>Het</u> represents an azole selected from the following:

a) a pyrazole of the general formula:

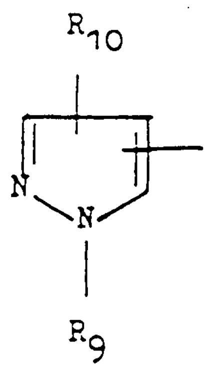

in which $R_9$ has one of the following meanings: a $C_1$ to $C_4$ lower alkyl radical, the dodecyl radical, the benzyl radical or a radical of the type:

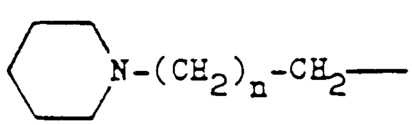

in which n = 1 or 2, and

$R_{10}$ represents a hydrogen atom, a methyl radical or a halogen atom,

b) an imidazole of the general formula:

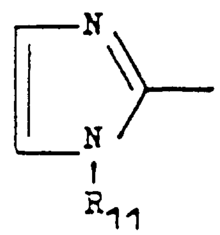

in which $R_{11}$ represents any one of the following groups: a hydrogen atom, a $C_1$ to $C_4$ lower alkyl radical, a dodecyl radical or a radical of the general formula:

$A-(CH_2)_n-$

in which n = 2, 3 or 4 and A represents any one of the following groups: a piperidine radical, a cyano group, a hydroxyl radical, a carboxyl radical, an amino group or a methyl ester group,
with the exception, however, of the compounds of general formula I in which Het represents an Imidazole, $R_5$ represents a hydrogen atom and $R_{11}$ represents a hydrogen atom, a hydrocarbon chain containing 1 to 10 carbon atoms, an aralkyl radical or an aryl radical, and with the exception of the following compounds:
1-methyl-α-phenyl-1H-pyrazole-5-methanol, and
1-methyl-α-(4-methyl-phenyl)-1H-pyrazole-5-methanol,

c) Het may represent an imidazole of formula:

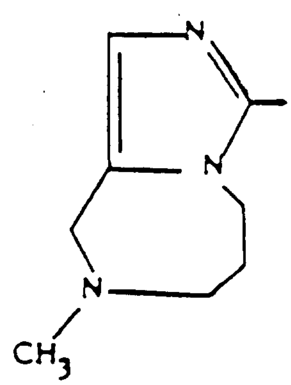

**2.** Compounds corresponding to the general formula I according to Claim 1, characterized in that they are selected from:

1 1-methyl-α-phenyl-O-(2-dimethylaminoethyl)-1H-imidazole-2-methanol
2 α-(4-chlorophenyl)-α-methyl-1-methyl-O-(2-dimethylaminoethyl)-1H-imidazole-2-methanol
3 α-(4-chlorophenyl)-1-methyl-O-(2-dimethylaminoethyl)-1H-imidazole-2-methanol
4 α-(3-chlorophenyl)-1-methyl-O-(2-dimethylaminoethyl)-1H-imidazole-2-methanol
5 α-(2-chlorophenyl)-1-methyl-O-(2-dimethylaminoethyl)-1H-imidazole-2-methanol
6 α-(4-fluorophenyl)α-methyl-1-methyl-O-(2-dimethylaminoethyl)-1H-imidazole-2-methanol
7 α-methyl-1-methyl-α-(3-trifluoromethylphenyl)-O-(2-dimethylaminoethyl)-1H-imidazole-2-methanol
8 α-(3-chlorophenyl)-α-methyl-1-methyl-O-(2-dimethylaminoethyl)-1H-imidazole-2-methanol
9 α-butyl-α-(3-chlorophenyl)-1-methyl-O-(2-dimethylaminoethyl)-1H-imidazole-2-methanol
10 1-butyl-α-(4-chlorophenyl)-α-methyl-O-(2-dimethylaminoethyl)-1H-imidazole-2-methanol

11 α-(4-methoxyphenyl)-α-methyl-1-methyl-O-(2-dimethylaminoethyl)-1H-imidazole-2-methanol
12 α-(3-chlorophenyl)-α-methyl-1-methyl-O-(ethyl-2-N-pyrrolidine)-1H-imidazole-2-methanol
13 α-butyl-1-dodecyl-α-(3,4,5-trimethoxyphenyl)-O-(2-dimethylaminoethyl)-1H-imidazole-2-methanol
14 1-butyl-α-(4-trifluoromethylphenyl)-O-(2-dimethylaminoethyl)-1H-imidazole-2-methanol
15 1-methyl-α-methyl-α-(3-trifluoromethylphenyl)-O-(ethyl-2-N-piperidine)-1H-imidazole-2-methanol
16 α-cyclohexyl-α-(3,4-dichlorophenyl)-1-methyl-O-(2-dimethylaminoethyl)-1H-imidazole-2-methanol
17 α-butyl-α-(3,4-dichlorophenyl)-1-methyl-O-(2-dimethylaminoethyl)-1H-imidazole-2-methanol
18 α-(3,4-dichlorophenyl)-α-methyl-1-methyl-O-(2-dimethylaminoethyl)-1H-imidazole-2-methanol
19 α-(3,4-dichlorophenyl)-1-methyl-O-(2-dimethylaminoethyl)-1H-imidazole-2-methanol
20 α-(4-chlorophenyl)-1-(ethyl-2-N-piperidine)-O-(2-dimethylaminoethyl)-1H-imidazole-2-methanol
21 α-(4-chlorophenyl)-α-methyl-1-(propyl-3-N-piperidine)-O-(2-dimethylaminoethyl)-1H-imidazole-2-methanol
22 1-(3-cyanopropyl)-α-(4-chlorophenyl)-O-(2-dimethylaminoethyl)-1H-imidazole-2-methanol
23 α-(4-chlorophenyl)-1-methyl-α-(N-methylpiperidin-4-yl)-O-(2-dimethylaminoethyl)-1H-imidazole-2-methanol
24 α-(4-chlorophenyl)-1-benzyl-O-(2-N-methyl-N-benzylaminoethyl)-1H-imidazole-2-methanol
25 α-(4-chlorophenyl)-α-methyl-7-methyl-O-(2-dimethylaminoethyl)-6,7,8,9-tetrahydro-1H-imidazole-[1,5-a][1,4]diazepine-2-methanol
26 α-(4-chlorophenyl)-7-methyl-O-(2-dimethylaminoethyl)-6,7,8,9-tetrahydro-1H-imidazole[1,5-a][1,4]-diazepine-2-methanol
27 1-butyl-α-(phenyl)-O-(2-dimethylaminoethyl)-1H-pyrazole-5-methanol
28 α-(4-chlorophenyl)-1-methyl-α-phenyl-O-(2-dimethylaminoethyl)-1H-pyrazole-5-methanol
29 1-butyl-α-(3,4,5-trimethoxyphenyl)-O-(2-dimethylaminoethyl)-1H-pyrazole-5-methanol
30 1-butyl-α-(4-chlorophenyl)-α-methyl-O-(2-dimethylaminoethyl)-1H-pyrazole-5-methanol
31 1-methy1-α-(phenyl)-O-(2-dimethylaminoethyl)-1H-pyrazole-5-methanol
32 α-methyl-1-methyl-α-(phenyl)-O-(2-dimethylaminoethyl)-1H-pyrazole-5-methanol
33 1-methyl-α-(3,4,5-trimethoxyphenyl)-O-(2-dimethylaminoethyl)-1H-pyrazole-5-methanol
34 1-methyl-α-(phenyl)-O-ethyl-2-(1-pyrrolidinyl)-1H-pyrazole-5-methanol
35 1-methyl-α-(phenyl)-O-ethyl-(2-N-morpholinyl)-1H-pyrazole-5-methanol
36 α-methyl-1-methyl-α-(3,4,5-trimethoxyphenyl)-O-(2-dimethylaminoethyl)-1H-pyrazole-5-methanol
37 4 - bromo-1-methyl-α-(phenyl)-O-(2-dimethylaminoethyl)-1H-pyrazole-5-methanol
38 1,3-dimethyl-α-(phenyl)-α-methyl-O-(2-dimethylaminoethyl)-1H-pyrazole-5-methanol
39 1,3-dimethyl-α-(phenyl)-O-(2-dimethylaminoethyl)-1H-pyrazole-5-methanol
40 1-methyl-α-(2-methylphenyl)-O-(2-dimethylaminoethyl)-1H-pyrazole-5-methanol
41 1-methyl-4-chloro-α-(4-chlorophenyl)-O-(2-dimethylaminoethyl)-1H-pyrazole-5-methanol
42 1-methyl-α-(4-chlorophenyl)-O-(2-dimethylaminoethyl)-1H-pyrazole-4-methanol
43 1-methy1-α-methyl-α-(4-chlorophenyl)-O-(2-dimethylaminoethyl)-1H-pyrazole-4-methanol
44 1-methyl-α-(4-chlorophenyl)-O-(2-dimethylaminoethyl-1H-pyrazole-5-methanol
45 1-methyl-α-(3-chlorophenyl)-O-(2-dimethylaminoethyl)-1H-pyrazole-5-methanol
46 1-methyl-α-(4-methylphenyl)-O-(2-dimethylaminoethyl)-1H-pyrazole-5-methanol
47 1-methyl-α-(2-chlorophenyl)-O-(2-dimethylaminoethyl)-1H-pyrazole-5-methanol
48 1-methyl-α-phenyl-O-(ethyl-2-N-piperidine)-1H-pyrazole-5-methanol
49 1-methyl-α-phenyl-O-[2-(N-propylpiperidin-2-yl)-ethyl]-1H-pyrazole-5-methanol
50 1-methyl-α-(4-chlorophenyl)-O-[2-(N-propylpiperidin-2-yl)ethyl]-1H-pyrazole-4-methanol
51 1-methyl-α-phenyl-O-[2-(N-ethylpiperidin-2-yl)-ethyl]-1H-pyrazole-5-methanol
52 1-methyl-α-phenyl-O-[2-(N-methylpyrrolidin-2-yl)-ethyl]-1H-pyrazole-5-methanol
53 1-methyl-α-phenyl-O-(2-diisopropylaminoethyl)-1H-pyrazole-5-methanol
54 1-methyl-α-(4-chlorophenyl)-O-[2-(N-methylpiperidin-2-yl)ethyl]-1H-pyrazole-4-methanol
55 1-methyl-α-(4-chlorophenyl)-O-[2-(N-ethylpiperidin-2-yl)ethyl]-1H-pyrazole-4-methanol
56 1-methyl-α-phenyl-O-[2-(N-methylpiperidin-2-yl)-ethyl]-1H-pyrazole-5-methanol
57 1-methyl-α-(4-chlorophenyl)-O-(2-diisopropylaminoethyl)-1H-pyrazole-4-methanol
58 1-methyl-α-(4-chlorophenyl)-O-[2-(N-methylpyrrolidin-2-yl)ethyl]-1H-pyrazole-4-methanol
59 α-(4-chlorophenyl)-α-methyl-1-methyl-O-(3-dimethylaminopropyl)-1H-imidazole-2-methanol
60 α-(3-chlorophenyl)-1-methyl-O-(3-dimethylaminopropyl)-1H-imidazole-2-methanol
61 α-(4-chlorophenyl)-α-ethyl-1-methyl-O-(3-dimethylaminopropyl)-1H-imidazole-2-methanol
62 α-butyl-α-(3-chlorophenyl)-1-methyl-O-(3-dimethylaminopropyl)-1H-imidazole-2-methanol
63 α-(4-chlorophenyl)-α-cyclohexyl-1-methyl-O-(3-dimethylaminopropyl)-1H-imidazole-2-methanol
64 α-(4-fluorophenyl)-α-methyl-1-methyl-O-(3-dimethylaminopropyl)-1H-imidazole-2-methanol

65 α-methyl-1-methyl-α-(3-trifluoromethylphenyl)-O-(3-dimethylaminopropyl)-1H-imidazole-2-methanol
66 α-(2-chlorophenyl)-α-methyl-1-methyl-O-(3-dimethylaminopropyl)-1H-imidazole-2-methanol
67 α-(3-chlorophenyl)-α-methyl-1-methyl-O-(3-dimethylaminopropyl)-1H-imidazole-2-methanol
68 α-methyl-1-methyl-α-(3,4,5-trimethoxyphenyl)-O-(3-dimethylaminopropyl)-1H-imidazole-2-methanol
69 α-methyl-1-methyl-α-(4-methoxyphenyl)-O-(3-dimethylaminopropyl)-1H-imidazole-2-methanol
70 α-(4-chlorophenyl)-1-methyl-O-(3-dimethylaminopropyl)-1H-imidazole-2-methanol
71 1-methyl-α-(3,4,5-trimethoxyphenyl)-O-(3-dimethylaminopropyl)-1H-imidazole-2-methanol
72 α-methyl-1-methyl-α-(4-trifluoromethylphenyl)-O-(3-dimethylaminopropyl)-1H-imidazole-2-methanol
73 1-methyl-α-(3-trifluoromethylphenyl)-O-(3-dimethylaminopropyl)-1H-imidazole-2-methanol
74 1-methyl-α-(4-trifluoromethylphenyl)-O-(3-dimethylaminopropyl)-1H-imidazole-2-methanol
75 α-(4-methoxyphenyl)-1-methyl-O-(3-dimethylaminopropyl)-1H-imidazole-2-methanol
76 α-butyl-1-methyl-α-(3-trifluoromethylphenyl)-O-(3-dimethylaminopropyl)-1H-imidazole-2-methanol
77 1-butyl-α-(4-chlorophenyl)-α-methyl-O-(3-dimethylaminopropyl)-1H-imidazole-2-methanol
78 1-butyl-α-butyl-α-(3,4,5-trimethoxyphenyl)-O-(3-dimethylaminopropyl)-1H-imidazole-2-methanol
79 1-butyl-α-butyl-α-(2-chlorophenyl)-O-(3-dimethylaminopropyl)-1H-imidazole-2-methanol
80 1-butyl-α-butyl-α-(2,4-dichlorophenyl)-O-(3-dimethylaminopropyl)-1H-imidazole-2-methanol
81 1-butyl-α-(4-trifluoromethylphenyl)-O-(3-dimethylaminopropyl)-1H-imidazole-2-methanol
82 α-(4-chlorophenyl)-1-methyl-O-(propyl-3-N-piperidino-1H-imidazole-2-methanol
83 α-methyl-1-methyl-α-(4-trifluoromethylphenyl)-O-(propyl-3-N-piperidine-1H-imidazole-2-methanol
84 α-butyl-α-(2-chlorophenyl)-1-methyl-O-(3-dimethylaminopropyl)-1H-imidazole-2-methanol
85 α-butyl-α-(3,4-dichlorophenyl)-1-methyl-O-(3-dimethylaminopropyl)-1H-imidazole-2-methanol
86 α-(3,4-dichlorophenyl)-α-methyl-1-methyl-O-(3-dimethylaminopropyl)-1H-imidazole-2-methanol
87 α-(3,4-dichlorophenyl)-1-methyl-O-(3-dimethylaminopropyl)-1H-imidazole-2-methanol
88 α-cyclopropyl-α-(3,4-dichlorophenyl)-1-methyl-O-(3-dimethylaminopropyl)-1H-imidazole-2-methanol
89 α-(4-chlorophenyl)-1-(ethyl-2-N-piperidino)-α-methyl-O-(3-dimethylaminopropyl)-1H-imidazole-2-methanol
90 α-(4-chlorophenyl)-α-methyl-1-(propyl-3-N-piperidino)-O-(3-dimethylaminopropyl)-1H-imidazole-2-methanol
91 α-(4-chlorophenyl)-1-methyl-(N-methylpiperidin-4-yl)-O-(3-dimethylaminopropyl)-1H-imidazole-2-methanol
92 1-butyl-α-(phenyl)-O-(3-dimethylaminopropyl)-1H-pyrazole-5-methanol
93 1-butyl-α-(4-chlorophenyl)-α-methyl-O-(3-dimethylaminopropyl)-1H-pyrazole-5-methanol
94 1-methyl-α-(phenyl)-O-(3-dimethylaminopropyl)-1H-pyrazole-5-methanol
95 1-methyl-α-(phenyl)-α-methyl-O-(3-dimethylaminopropyl)-1H-pyrazole-5-methanol
96 1,3-dimethyl-α-(phenyl)-α-methyl-O-(3-dimethylaminopropyl)-1H-pyrazole-5-methanol
97 1,3-dimethyl-α-(phenyl)-O-(3-dimethylaminopropyl)-1H-pyrazole-5-methanol
98 1-methyl-α-(2-methylphenyl)-O-(3-dimethylaminopropyl)-1H-pyrazole-5-methanol
99 1-methyl-α-(4-chlorophenyl)-O-(3-dimethylaminopropyl)-1H-pyrazole-5-methanol
100 1-methyl-α-phenyl-O-(3-dimethylaminopropyl)-1H-pyrazole-4-methanol
101 1-methyl-α-(4-chlorophenyl)-O-(propyl-3-N-morpholine)-1H-pyrazole-4-methanol
102 1-methyl-α-(4-chlorophenyl)-O-(propyl-3-N-pyrrolidine)-1H-pyrazole-4-methanol
103 1-methyl-α-phenyl-O-(propyl-3-N-piperidine)-1H-pyrazole-5-methanol
104 1-methyl-α-phenyl-O-(propyl-3-N-pyrrolidine)-1H-pyrazole-5-methanol
105 α-phenyl-1H-imidazole-2-methanol
106 α-(4-chlorophenyl)-1H-imidazole-2-methanol
107 α-(4-chlorophenyl)-1-methyl-1H-imidazole-2-methanol
108 α-(3-chlorophenyl)-1-methyl-1H-imidazole-2-methanol
109 α-(4-fluorophenyl)-1-methyl-1H-imidazole-2-methanol
110 1-methyl-α-(3-trifluoromethylphenyl)-1H-imidazol-2-methanol
111 1-methyl-α-(4-trifluoromethylphenyl)-1H-imidazole-2-methanol
112 1-methyl-α-(3,4,5-trimethoxyphenyl)-1H-imidazole-2-methanol
113 α-(3,4-dichlorophenyl)-1-methyl-1H-imidazole-2-methanol
114 1-butyl-α-(4-trifluoromethylphenyl)-1H-imidazole-2-methanol
115 1-butyl-α-(2,4-dichlorophenyl)-1H-imidazole-2-methanol

116 1-butyl-α-(4-chlorophenyl)-1H-imidazole-2-methanol
117 1-butyl-α-(3,4,5-trimethoxyphenyl)-1H-imidazole-2-methanol
118 1-dodecyl-α-(3,4,5-trimethoxyphenyl)-1H-imidazole-2-methanol
119 α-butyl-α-(3-chlorophenyl)-1-methyl-1H-imidazole-2-methanol
120 α-(3-chlorophenyl)-α-methyl-1-methyl-1H-imidazole-2-methanol
121 α-(4-chlorophenyl)-α-methyl-1-methyl-1H-imidazole-2-methanol
122 α-(4-chlorophenyl)-α-(N-methylpiperidin-4-yl)-1-methyl-1H-imidazole-2-methanol
123 α-(4-chlorophenyl)-α-ethyl-1-methyl-1H-imidazole-2-methanol
124 α-butyl-α-(4-chlorophenyl)-1-methyl-1H-imidazole-2-methanol
125 α-(4-chlorophenyl)-α-cyclohexyl-1-methyl-1H-imidazole-2-methanol
126 α-(2-chlorophenyl)-α-methyl-1-methyl-1H-imidazole-2-methanol
127 α-butyl-α-(2-chlorophenyl)-1-methyl-1H-imidazole-2-methanol
128 α-methyl-1-methyl-α-(3-trifluoromethylphenyl)-1H-imidazole-2-methanol
129 α-butyl-1-methyl-α-(3-trifluoromethylphenyl)-1H-imidazole-2-methanol
130 α-cyclohexyl-1-methyl-α-(3-trifluoromethylphenyl)-IH-imidazole-2-methanol
131 α-methyl-1-methyl-α-(4-trifluoromethylphenyl)-1H-imidazole-2-methanol
132 α-(4-fluorophenyl)-α-methyl-1-methyl-1H-imidazole-2-methanol
133 α-(4-methoxyphenyl)-α-methyl-1-methyl-1H-imidazole-2-methanol
134 α-(3,4-dichlorophenyl)-α-methyl-1-methyl-1H-imidazole-2-methanol
135 α-butyl-α-(3,4-dichlorophenyl)-1-methyl-1H-imidazole-2-methanol
136 α-cyclohexyl-α-(3,4-dichlorophenyl)-1-methyl-1H-imidazole-2-methanol
137 α-methyl-1-methyl-α-(3,4,5-trimethoxyphenyl)-1H-imidazole-2-methanol
138 1-butyl-α-(4-chlorophenyl)-α-methyl-1H-imidazole-2-methanol
139 1-butyl-α-butyl-α-(4-chlorophenyl)-1H-imidazole-2-methanol
140 1-butyl-α-(4-chlorophenyl)-α-(N-methylpiperidin-4-yl)-1H-imidazole-2-methanol
141 1-butyl-α-butyl-α-(3,4,5-trimethoxyphenyl)-1H-imidazole-2-methanol
142 1-butyl-α-butyl-α-(2-chlorophenyl)-1H-imidazole-2-methanol
143 1-butyl-α-ethyl-α-(3-trifluoromethylphenyl)-1H-imidazole-2-methanol
144 1-butyl-α-butyl-α-(2,4-dichlorophenyl)-1H-imidazole-2-methanol
145 α-(4-chlorophenyl)-α-methyl-1-(propyl-N-piperidine)-1H-imidazole-2-methanol
146 α-(4-chlorophenyl)-1-(3-dimethylaminopropyl)-α-methyl-1H-imidazole-2-methanol
147 α-butyl-1-dodecyl-α-(3,4,5-trimethoxyphenyl)-1H-imidazole-2-methanol
148 1-benzyl-α-butyl-α-(3-trifluoromethylphenyl)-1H-imidazole-2-methanol
149 1-benzyl-α-(4-chlorophenyl)-α-methyl-1H-imidazole-2-methanol
150 1-(2-cyanoethyl)-α-(4-chlorophenyl)-1H-imidazole-2-methanol
151 1-(3-aminopropyl)-α-(4-chlorophenyl)-1H-imidazole-2-methanol
152 1-(propane-3-carboxylic)-α-(3-chlorophenyl)-1H imidazole-2-methanol
153 1-(3-hydroxypropyl)-α-(4-chlorophenyl)-1H-imidazole-2-methanol
154 1-(3-methylpropanoate)-α-(4-chlorophenyl)-1H-imidazole-2-methanol
155 1-(3-hydroxypropyl)-α-phenyl-1H-imidazole-2-methanol
156 1-(3-hydroxypropyl)-α-(4-methylphenyl)-1H-imidazole-2-methanol
157 1-(3-hydroxypropyl)-α-(4-methoxyphenyl)-1H-imidazole-2-methanol
158 1-(3-hydroxypropyl)-α-(3,4-dichlorophenyl)-1H-imidazole-2-methanol
159 1-(3-methylpropanoate)-α-phenyl-1H-imidazole-2-methanol
160 1-(4-hydroxybutyl)-α-(4-chlorophenyl)-1H-imidazole-2-methanol
161 1-(3-cyanopropyl)-α-(4-chlorophenyl)-1H-imidazole-2-methanol
162 1-(4-butyric)-α-(4-chlorophenyl)-1H-imidazole-2-methanol
163 1-(4-methylbutyrate)-α-(4-chlorophenyl)-1H-imidazole-2-methanol
164 1-butyl-α-phenyl-1H-pyrazole-5-methanol
165 α-(4-chlorophenyl)-1-methyl-1H-pyrazole-5-methanol
166 1-methyl-α-(3,4,5-trimethoxyphenyl)-1H-pyrazole-5-methanol
167 1-butyl-α-(3,4,5-trimethoxyphenyl)-1H-pyrazole-5-methanol
168 4-bromo-1-methyl-α-phenyl-1H-pyrazole-5-methanol
169 α-(4-chlorophenyl)-1-methyl-α-phenyl-1H-pyrazole-5-methanol
170 1-butyl-α-(4-chlorophenyl)-α-methyl-1H-pyrazole-5-methanol
171 1-methyl-α-methyl-α-phenyl-1H-pyrazole-5-methanol
172 1-methyl-α-methyl-α-(3,4,5-trimethoxyphenyl)-1H-pyrazole-5-methanol
173 1,3-dimethyl-α-methyl-α-phenyl-1H-pyrazole-5-methanol

174 α-ethenyl-1-methyl-α-phenyl-1H-pyrazole-5-methanol
175 1-butyl-α-(4-chlorophenyl)-α-ethenyl-1H-pyrazole-5-methanol
176 4-chloro-1-methyl-α-phenyl-1H-pyrazole-5-methanol
177 1-methyl-α-(2-methylphenyl)-1H-pyrazole-5-methanol
178 1-methyl-α-(3-chlorophenyl)-1H-pyrazole-5-methanol [lacuna]
180 1-methyl-α-(2-chlorophenyl)-1H-pyrazole-5-methanol
181 1-methyl-α-(4-methoxyphenyl)-1H-pyrazole-5-methanol
182 α-(4-chlorophenyl)-α-methyl-1-methyl-O-(2-trimethylammoniumethyl)-1H-imidazole-2-methanol iodide
183 α-(4-chlorophenyl)-α-methyl-1-methyl-O-(3-trimethylammoniumpropyl)-1H-imidazole-2-methanol iodide
184 α-(4-methoxyphenyl)-α-methyl-1-methyl-O-(2-trimethylammoniumethyl)-1H-imidazole-2-methanol iodide
185 α-(4-methoxyphenyl)-α-methyl-1-methyl-O-(3-trimethylammoniumpropyl)-1H-imidazole-2-methanol iodide
186 α-(4-methoxyphenyl)-α-methyl-1,1-dimethylimidazolio-2-methanol iodide

**3.** Method for the preparation of the compounds according to either of Claims 1 or 2, characterized in that at least one of the following operations is carried out:

3.1. Reaction of a compound of the general formula II:

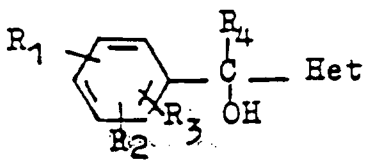

II

with a compound of the general formula III:

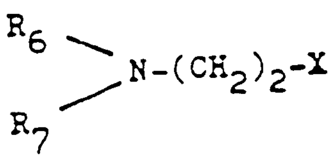

III

in which formulae $R_1$ to $R_4$, $R_6$, $R_7$, X and Het have the meanings mentioned in Claim 1.

3.2. Reaction of a compound of the general formula II with a compound of the general formula IV:

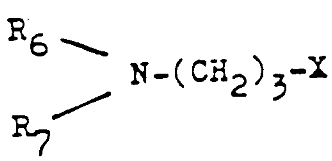

IV

in which $R_1$ to $R_4$, $R_6$, $R_7$, X and Het have the meanings mentioned in Claim 1.

**4.** Method for the preparation of compounds of the general formula II, characterized in that at least one of the following operations is carried out:

4.1. Reduction of a compound of the general formula V:

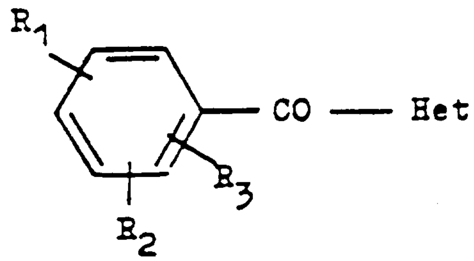

V

in which $R_1$, $R_2$, $R_3$ and Het have the meanings mentioned in Claim 1.

4.2. Reaction of a compound of the general formula V with Grignard reagents of the type:

$R_4MgX$

in which $R_4$ has the meanings mentioned in Claim 1.

**5.** Method for the preparation of compounds of the general formula II, characterized in that the following operation is carried out:
Reaction of an aldehyde of the general formula VI:

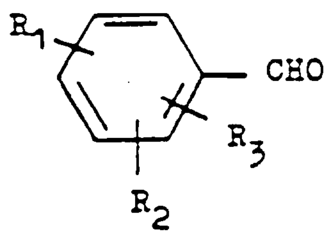

VI

with compounds of the type Het-Li, in which formula $R_1$, $R_2$ and $R_3$ have the meanings mentioned in Claim 1, Het represents a pyrazole of the general formula mentioned in Claim 1 and Li represents a lithium atom.

**6.** Method for the preparation of compounds of the general formula VII:

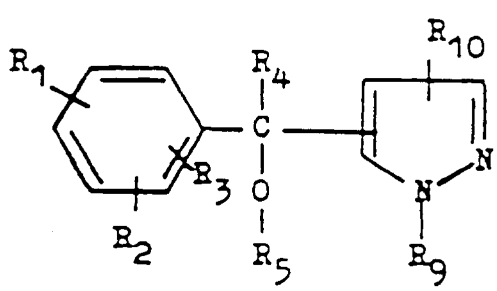

VII

in which $R_1$ to $R_9$ have the meanings mentioned in Claim 1 and $R_{10}$ is chlorine or bromine, by halogenation of the precursor compound of general formula VII in which $R_1$ to $R_9$ have the meanings mentioned in Claims 1 to 10 and $R_{10}$ represents a hydrogen atom.

7. Method for the preparation of compounds of the general formula VIII:

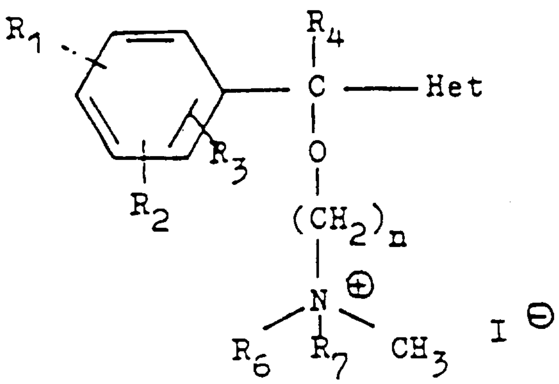

VIII

from compounds of the general formula I:

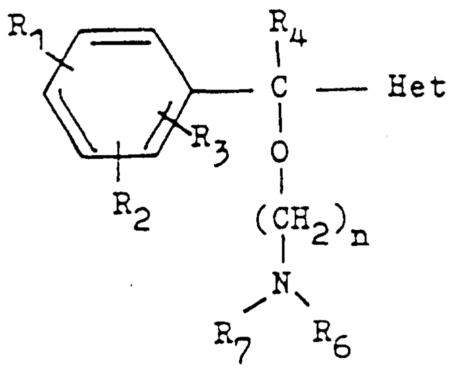

I

in which formulae $R_1$ to $R_4$, $R_6$, $R_7$ and Het have the meanings mentioned in Claim 1, by reaction with methyl iodide.

8. Method for the preparation of compounds of the general formula IX:

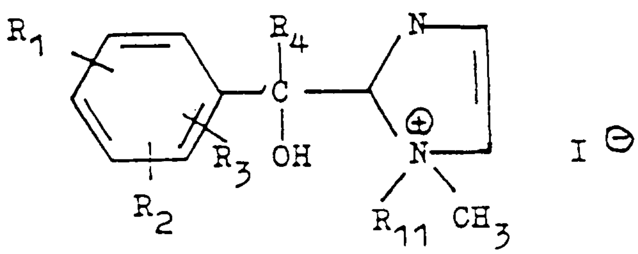

IX

from compounds of the general formula X:

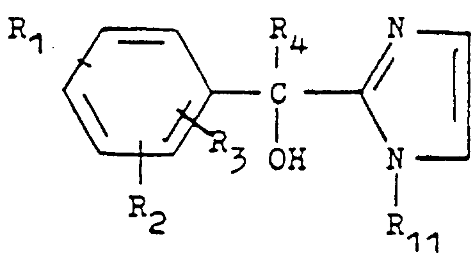

X

in which formulae $R_1$ to $R_2$ and $R_{11}$ have the meanings mentioned in Claim 1, by reaction with methyl iodide.

**9.** As drugs, the derivatives of the general formula I and their therapeutically acceptable salts according to one of Claims 1 and 2, in particular as drugs for the treatment of pain.

**10.** Pharmaceutical compositions characterized in that they contain, in addition to a pharmaceutically acceptable excipient, at least one derivative of the general formula I or one of its physiologically acceptable salts according to one of Claims 1 and 2.

**11.** Use of the arylheteroarylcarbinol derivatives corresponding to the general formula I:

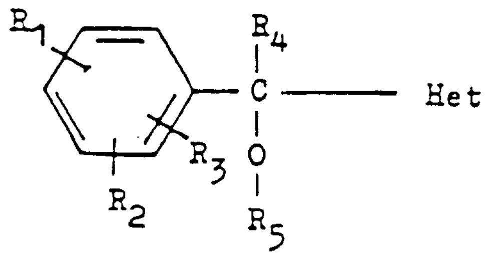

I

and their therapeutically acceptable salts, in which formula:

$R_1$, $R_2$ and $R_3$, which are identical or different, represent a hydrogen atom, a halogen, a lower alkyl radical, a lower alkoxy radical or a trifluoromethyl group,

$R_4$ represents a hydrogen atom, a $C_1$ to $C_4$ lower alkyl radical, a cycloalkyl, a lower alkenyl radical or a cycloalkylamino substituted on the nitrogen atom,

$R_5$ represents a hydrogen atom or a radical of the formula:

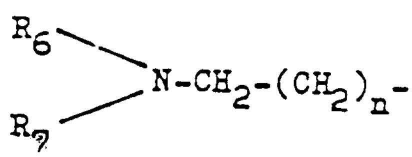

in which n can have the value 1 or 2 and the group:

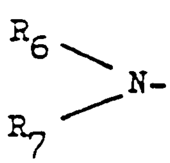

can have one of the following meanings:
a lower dialkylamino group or a cyclic amine radical selected from piperidino, morpholino and pyrrolidino groups, or

$R_5$ represents a group of the general formula:

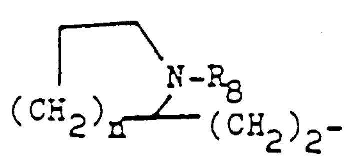

in which n can have the value 1 or 2 and in which $R_8$ is a lower alkyl, and

Het represents an azole selected from the following:

a) a pyrazole of the general formula:

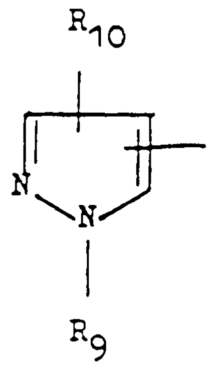

in which $R_9$ has one of the following meanings:
a $C_1$ to $C_4$ lower alkyl radical, the dodecyl radical, the benzyl radical or a radical of the type:

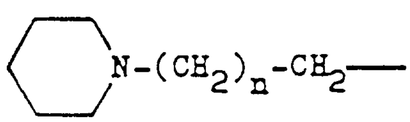

in which n = 1 or 2, and

$R_{10}$ represents a hydrogen atom, a methyl radical or a halogen atom,

b) an imidazole of the general formula:

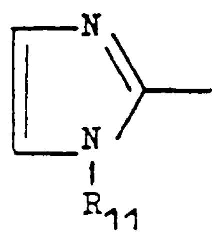

in which $R_{11}$ represents any one of the following groups: a hydrogen atom, a $C_1$ to $C_4$ lower alkyl radical, a dodecyl radical or a radical of the general formula:

$A-(CH_2)_n-$

in which n = 2, 3 or 4 and A represents any one of the following groups: a piperidine radical, a cyano group, a hydroxyl radical, a carboxyl radical, an amino group or a methyl ester group,

c) Het may also represent an imidazole of formula:

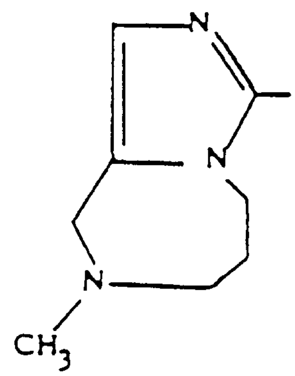

for the manufacture of drugs intended for the treatment of pain, in particular for the manufacture of analgesics.

**12.** As drug, 1-methyl-$\alpha$-(4-methyl-phenyl)-1H-pyrazole-5-methanol.

**Patentansprüche**

**1.** Aryl-heteroaryl-carbinol-Derivate der allgemeinen Formel (I)

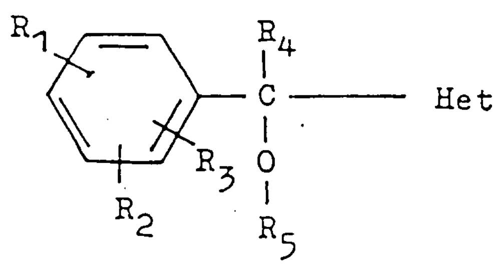

I

sowie ihre therapeutisch akzeptablen Salze, worin bedeuten:

| | |
|---|---|
| $R_1$, $R_2$ und $R_3$, | die gleich oder verschieden sind, jeweils ein Wasserstoffatom, ein Halogenatom, einen niederen Alkylrest, einen niederen Alkoxyrest oder eine Trifluoromethylgruppe, |
| $R_4$ | ein Wasserstoffatom, einen $C_1$-$C_4$-Niedrigalkylrest, einen Cycloalkylrest, einen niederen Alkenylrest oder einen durch ein Stickstoffatom substituierten Cycloalkylaminorest, |
| $R_5$ | ein Wasserstoffatom oder einen Rest der Formel |

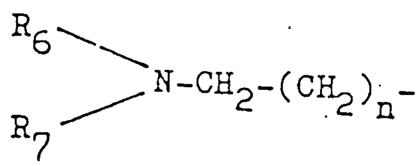

in der n die Werte 1 oder 2 haben kann und die Gruppierung

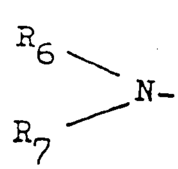

| | |
|---|---|
| $R_5$ | eine der folgenden Bedeutungen haben kann: eine niedere Dialkylaminogruppe oder einen cyclischen Aminorest, ausgewählt unter den Piperidino-, Morpholino- oder Pyrrolidino-Gruppen, außerdem auch eine Gruppe der allgemeinen Formel |

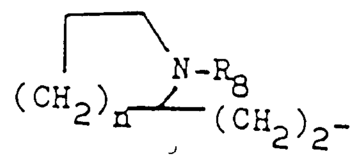

in der n den Wert 1 oder 2 haben kann und in der $R_8$ einen niederen Alkylrest darstellt,

| | |
|---|---|
| Het | ein Azol, ausgewählt unter den folgenden: |

a) ein Pyrazol der allgemeinen Formel

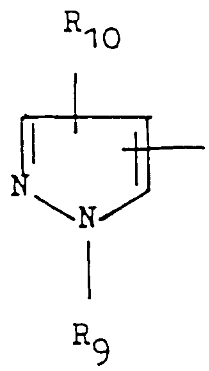

in der $R_9$ eine der folgenden Bedeutungen hat:

— einen $C_1$-$C_4$-Niedrigalkrest, den Dodecylrest, den Benzylrest oder einen Rest des Typs

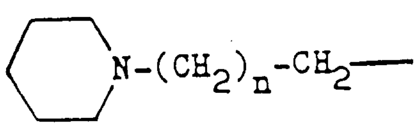

in der n = 1 oder 2, und

in der $R_{10}$ ein Wasserstoffatom, einen Methylrest oder ein Halogenatom darstellt,

b) ein Imidazol der allgemeinen Formel

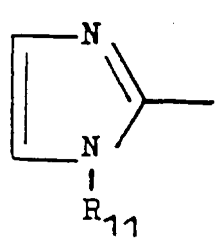

in der $R_{11}$ irgendeine der folgenden Gruppen darstellt: ein Wasserstoffatom, einen $C_1$-$C_4$-Niedrigalkylrest, einen Dodecylrest, einen Rest der Formel A-$(CH_2)_n$-, in der n = 2, 3 oder 4 und A eine beliebige der folgenden Gruppen darstellt: einen Piperidinrest, eine Cyanogruppe, einen Hydroxyrest, einen

Carboxyrest, eine Aminogruppe oder eine Methylestergruppe,
jedoch mit Ausnahme der Verbindungen der allgemeinen Formel (I), in denen Het steht für ein Imidazol, $R_5$ steht für ein Wasserstoffatom und $R_{11}$ steht für ein Wasserstoffatom, eine Kohlenwasserstoffkette mit 1 bis 10 Kohlenstoffatomen, einen Aralkylrest oder einen Arylrest und
mit Ausnahme der folgenden Verbindungen:
1H-Pyrazol-5-methanol-1-methyl-α-phenyl und
1H-Pyrazol-5-methanol-1-methyl-α-(4-methylphenyl),
c) ein Imidazol der Formel

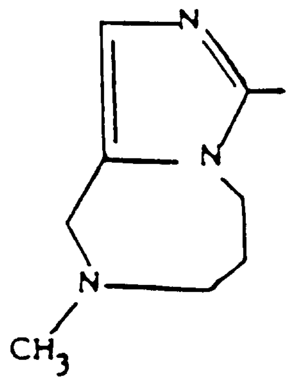

**2.** Verbindungen der allgemeinen Formel (I) nach Anspruch 1, dadurch gekennzeichnet, daß sie ausgewählt werden aus:

| | |
|---|---|
| 1 | IH-Imidazol -2-methanol, 1-methyl-α-phenyl-O-(2-dimethylamino)ethyl |
| 2 | 1H-Imidazol -2-methanol, α-(4-chlorophenyl)-α-methyl-1-methyl-O-(2-dimethylamino)ethyl |
| 3 | 1H-Imidazol -2-methanol, α-(4-chlorophenyl)-1-methyl-O-(2-dimethylamino)ethyl |
| 4 | 1H-Imidazol -2-methanol, α-(3-chlorophenyl)-1-methyl-O-(2-dimethylamino)ethyl |
| 5 | 1H-Imidazol -2-methanol, α-(2-chlorophenyl)-1-methyl-O-(2-dimethylamino)ethyl |
| 6 | 1H-Imidazol -2-methanol, α-(4-fluorophenyl)-α-methyl-1-methyl-O-(2-dimethylamino)ethyl |

| | |
|---|---|
| 7 | 1H-Imidazol -2-methanol, α-methyl-1-methyl-α-(3-trifluoromethylphenyl)-O-(2-dimethyl-amino)ethyl |
| 8 | 1H-Imidazol -2-methanol, α-(3-chlorophenyl)-α-methyl-1-methyl-O-(2-dimethylamino)ethyl |
| 9 | 1H-Imidazol -2-methanol, α-(butyl-α-(3-chlorophenyl)-1-methyl-O-(2-dimethylamino) ethyl |
| 10 | 1H-Imidazol -2-methanol, 1-butyl-α-(4-chlorophenyl)-α-methyl-O-(2-dimethylamino)ethyl |
| 11 | 1H-Imidazol -2-methanol, α-(4-methoxyphenyl)-α-methyl-1-methyl-O-(2-dimethylamino)ethyl |
| 12 | 1H-Imidazol -2-methanol-α-(3-chlorophenyl)-α-methyl-1-methyl-O-ethyl-2-(N-pyrrolidin ) |
| 13 | 1H-Imidazol -2-methanol-α-butyl-1-dodecyle-α-(3,4,5-trimethoxyphenyl)-O-(2-dimethylamino)-ethyl |
| 14 | 1H-Imidazol -2-methanol, 1-butyl-α-(4-tri-fluoromethylphenyl)-O-(2-dimethylamino)ethyl |
| 15 | 1H-Imidazol -2-methanol, 1-methyl-α-methyl-α-(3-trifluoromethylphenyl)-O-ethyl-2-(N-piperidin ) |
| 16 | 1H-Imidazol -2-methanol, α-cyclohexyl-α-(3,4-dichlorophenyl)-1-methyl-O-(2-dimethylamino)-ethyl |
| 17 | 1H-Imidazol -2-methanol, α-butyl-α-(3,4-di-chlorophenyl)-1-methyl-O-(2-dimethylamino)-ethyl |
| 18 | 1H-Imidazol -2-methanol, α-(3,4-dichlorophe-nyl)-α-methyl-1-methyl-O-(2-dimethylamino)-ethyl |
| 19 | 1H-Imidazol -2-methanol-α-(3,4-dichlorophe-nyl)-1-methyl-O-(2-dimethylamino)ethyl |

| | |
|---|---|
| 20 | 1H-Imidazol -2-methanol, α-(4-chlorophenyl)-1-[ethyl-2-(N-piperidin )]-O-(2-dimethyl-amino)ethyl |
| 21 | 1H-Imidazol -2-methanol, α-(4-chlorophenyl)-α-methyl-1-[propyl-3-(N-piperidin )]-O-(2-dimethylamino)ethyl |
| 22 | 1H-Imidazol -2-methanol, 1-(3-cyanopropyl)-α-(4-chlorophenyl)-O-(2-dimethylamino)ethyl |
| 23 | 1H-Imidazol -2-methanol, α-(4-chlorophenyl)-1-methyl-α-[(N-methylpiperidin )-4-yl]-O-(2-dimethylamino)ethyl |
| 24 | 1H-Imidazol -2-methanol-α-(4-chlorophenyl)-1-benzyl-O-(N-methyl-N-benzyl-2-amino)ethyl |
| 25 | 1H-Imidazol [1,5-a][1,4]diazepin , 6,7,8,9-tetrahydro-2-methanol, α-(4-chlorophenyl)-α-methyl-7-methyl-O-(2-dimethylamino)ethyl |
| 26 | 1H-Imidazol [1,5-a][1,4]diazepin , 6,7,8,9-tetrahydro-2-methanol, α-(4-chlorophenyl)-7-methyl-O-(2-dimethylamino)ethyl |
| 27 | 1H-Pyrazol -5-methanol, 1-butyl-α-(phenyl)-O-(2-dimethylamino) ethyl |
| 28 | 1H-Pyrazol -5-methanol, α-(4-chlorophenyl)-1-methyl-α-phenyl-O-(2-dimethylamino)ethyl |
| 29 | 1H-Pyrazol -5-methanol, 1-butyl-α-(3,4,5-trimethoxyphenyl)-O-(2-dimethylamino)ethyl |
| 30 | 1H-Pyrazol -5-methanol, 1-butyl-α-(4-chlorophenyl)-α-methyl-O-(2-dimethylamino)ethyl |
| 31 | 1H-Pyrazol -5-methanol, 1-méthyl -α-(phényl)-O-(2-diméthylamino)éthyl |
| 32 | 1H-Pyrazol -5-methanol, α-methyl-1-methyl-α-(phenyl)-O- (2-dimethylamino)ethyl |
| 33 | 1H-Pyrazol -5-methanol, 1-methyl-α-(3,4,5-trimethoxyphenyl)-O-(2-dimethylamino)ethyl |

| | |
|---|---|
| 34 | 1H-Pyrazol -5-methanol, 1-methyl- α-(phenyl)-O-ethyl-2-(1-pyrrolidinyl) |
| 35 | 1H-Pyrazol -5-methanol, 1-methyl- α-(phenyl)-O-ethyl-(2-N-morpholinyl) |
| 36 | 1H-Pyrazol -5-methanol, α-methyl-1-methyl-α-(3,4,5-trimethoxyphenyl)-O-(2-dimethylamino)ethyl |
| 37 | 1H-Pyrazol -5-methanol, 4-bromo-1-methyl-α-(phenyl)-O-(2-dimethylamino)ethyl |
| 38 | 1H-pyrazol -5-methanol, 1,3-dimethyl-α-(phenyl)-α-methyl-O-(2-dimethylamino)ethyl |
| 39 | 1H-Pyrazol -5-methanol, 1,3-dimethyl-α-(phenyl)-O-(2-dimethylamino)ethyl |
| 40 | 1H-Pyrazol -5-methanol, 1-methyl-α-(2-methyl-phenyl)-O-(2-dimethylamino)ethyl |
| 41 | 1H-Pyrazol -5-methanol, 1-methyl-4-chloro-α-(4-chlorophenyl)-O-(2-dimethylamino)ethyl |
| 42 | 1H-Pyrazol -4-methanol, 1-methyl-α-(4-chloro-phenyl)-O-(2-dimethylamino)ethyl |
| 43 | 1H-Pyrazol -4-methanol, 1-methyl-α-methyl-α-(4-chlorophenyl)-O-(2-dimethylamino)ethyl |
| 44 | 1H-Pyrazol -5-methanol, 1-methyl-α-(4-chloro-phenyl)-O-(2-dimethylamino)ethyl |
| 45 | 1H-Pyrazol -5-methanol, 1-methyl-α-(3-chloro-phenyl)-O-(2-dimethylamino)ethyl |
| 46 | 1H-pyrazol -5-methanol, 1-methyl-α-(4-methyl-phenyl)-O-(2-dimethylamino)ethyl |
| 47 | 1H-Pyrazol -5-methanol, 1-methyl-α-(2-chloro-phenyl)-O-(2-dimethylamino)ethyl |
| 48 | 1H-Pyrazol -5-methanol, 1-methyl, α-phenyl-O-2(N-piperidino)ethyl |
| 49 | 1H-Pyrazol -5-methanol, 1-methyl, α-phenyl-O-ethyl-2-(N-propylpiperidin -2-yl) |

| | |
|---|---|
| 50 | 1H-Pyrazol -4-methanol, 1-methyl-α-(4-chlorophenyl)-O-ethyl-2(N-propylpiperidin -2-yl) |
| 51 | 1H-Pyrazol -5-methanol, 1-methyl-α-phenyl-O-ethyl-2(N-ethylpiperidin -2-yl) |
| 52 | 1H-Pyrazol -5-methanol, 1-methyl-α-phenyl-O-ethyl-2(N-methylpyrrolidin -2-yl) |
| 53 | 1H-Pyrazol -5-methanol, 1-methyl-α-phenyl-O-(2-diisopropylamino)ethyl |
| 54 | 1H-Pyrazol -4-methanol-1-methyl-α-(4-chlorophenyl)-O-ethyl-2(N-methylpiperidin -2-yl) |
| 55 | 1H-Pyrazol -4-methanol-1-methyl-α-(4-chlorophenyl)-O-ethyl-2(N-ethylpiperidin -2-yl) |
| 56 | 1H-Pyrazol -5-methanol-1-methyl-α-phenyl-O-ethyl-2-(N-methylpiperidin -2-yl) |
| 57 | 1H-Pyrazol -4-methanol-1-methyl-α-(4-chlorophenyl)-O-(2-diisopropylamino)ethyl |
| 58 | 1H-Pyrazol -4-methanol-1-methyl-α-(4-chlorophenyl)-O-ethyl-2-(N-methylpyrrolidin -2-yl) |
| 59 | 1H-Imidazol -2-methanol, α-(4-chlorophenyl)-α-methyl-1-methyl-O-(3-dimethylamino)propyl |
| 60 | 1H-Imidazol -2-methanol, α-(3-chlorophenyl)-1-methyl-O-(3-dimethylamino)propyl |
| 61 | 1H-Imidazol -2-methanol, α-(4-chlorophenyl)-α-ethyl-1-methyl-O-(3-dimethylamino)propyl |
| 62 | 1H-Imidazol -2-methanol, α-butyl-α-(3-chlorophenyl)-1-methyl-O-(3-dimethylamino)propyl |
| 63 | 1H-Imidazol -2-methanol, α-(4-chlorophenyl)-α-cyclohexyl-1-methyl-O-(3-dimethylamino)-propyl |
| 64 | 1H-Imidazol -2-methanol, α-(4-fluorophenyl)-α-methyl-1-methyl-O-(3-dimethylamino)propyl |
| 65 | 1H-Imidazol -2-methanol, α-methyl-1-methyl-α-(3-trifluoromethylphenyl)-O-(3-dimethylamino)propyl |

| | |
|---|---|
| 66 | 1H-Imidazol -2-methanol, α-(2-chlorophenyl)-α-methyl-1-methyl-O-(3-dimethylamino)propyl |
| 67 | 1H-Imidazol -2-methanol-α-(3-chlorophényl)-α-méthyl-1-méthyl-O-(3-dimethylamino)propyl |
| 68 | 1H-Imidazol -2-methanol, α-méthyl-1-méthyl-α-(3,4,5-trimethoxyphenyl)-O-(3-dimethyl-amino)propyl |
| 69 | 1H-Imidazol -2-methanol, α-méthyl-1-méthyl-α-(4-methoxyphenyl)-O-(3-dimethylamino)propyl |
| 70 | 1H-Imidazol -2-methanol, α-(4-chlorophenyl-1-methyl-O-(3-dimethylamino)propyl |
| 71 | 1H-Imidazol -2-methanol, 1-methyl-α-(3,4,5-trimethoxyphenyl-O-(3-dimethylamino)propyl |
| 72 | 1H-Imidazol -2-methanol, α-methyl-1-methyl-α-(4-trifluorométhylphenyl)-O-(3-dimethyl-amino)-propyl |
| 73 | 1H-Imidazol -2-methanol, 1-methyl-α-(3-trifluorométhylphenyl)-O-(3-diméthylamino)-propyl |
| 74 | 1H-Imidazol -2-méthanol, 1-méthyl-α-(4-trifluoromethylphenyl)-O-(3-dimethylamino)-propyl |
| 75 | 1H-Imidazol -2-methanol, α-(4-methoxyphenyl)-1-méthyl-O-(3-dimethylamino)propyl |
| 76 | 1H-Imidazol -2-methanol, α-butyl-1-méthyl-α-(3-trifluoromethylphenyl)-O-(3-dimethyl-amino)propyl |
| 77 | 1H-Imidazol -2-méthanol, 1-butyl-α-(4-chlorophenyl)-α-methyl-O-(3-dimethylamino)-propyl |
| 78 | 1H-Imidazol -2-methanol, 1-butyl-α-butyl-α-(3,4,5-trimethoxyphenyl)-O-(3-dimethylamino)-propyl |

| | |
|---|---|
| 79 | 1H-Imidazol -2-methanol, 1-butyl-α-butyl-α-(2-chlorophenyl)-O-(3-dimethylamino)-propyl |
| 80 | 1H-Imidazol -2-methanol, 1-butyl-α-butyl-α-(2,4-dichlorophenyl)-O-(3-dimethylamino)-propyl |
| 81 | 1H-Imidazol -2-methanol, 1-butyl-α-(4-trifluoromethylphenyl)-O-(3-dimethylamino)-propyl |
| 82 | 1H-Imidazol -2-methanol, α-(4-chlorophenyl)-1-methyl-O-3(N-piperidino)propyl |
| 83 | 1H-Imidazol -2-methanol, α-methyl-1-methyl-α-(4-trifluoromethylphenyl)-O-3-(N-piperidin )-propyl |
| 84 | 1H-Imidazol -2-methanol, α-butyl-α-(2-chlorophenyl)-1-methyl-O-(3-dimethylamino)propyl |
| 85 | 1H-Imidazol -2-methanol, α-butyl-α-(3,4-dichlorophenyl)-1-methyl-O-(3-dimethylamino)-propyl |
| 86 | 1H-Imidazol -2-methanol, α-(3,4-dichlorophenyl)-α-methyl-1-methyl-O-(3-dimethylamino)-propyl |
| 87 | 1H-Imidazol -2-methanol, α-(3,4-dichlorophenyl)-1-methyl-O-(3-dimethylamino)propyl |
| 88 | 1H-Imidazol -2-methanol, α-cyclopropyl-α-(3,4-dichlorophenyl)-1-methyl-O-(3-dimethylamino)propyl |
| 89 | 1H-Imidazol -2-methanol, α-(4-chlorophenyl)-1-[ethyl-2(N-piperidino)]-α-methyl-O-(3-dimethylamino)propyl |
| 90 | 1H-Imidazol -2-methanol, α-(4-chlorophenyl)-α-methyl-1-[propyl-3(N-piperidino)]-O-(3-dimethylamino)propyl |

| | |
|---|---|
| 91 | 1H-Imidazol -2-methanol, α-(4-chlorophenyl)-1-methyl-[(N-methylpiperidin)-4-yl]-O-(3-dimethylamino)propyl |
| 92 | 1H-Pyrazol -5-methanol, 1-butyl-α-(phényl)-O-(3-dimethylamino) propyl |
| 93 | 1H-Pyrazol -5-methanol, 1-butyl-α-(4-chlorophenyl)-α-methyl-O-(3-dimethylamino)propyl |
| 94 | 1H-Pyrazol -5-methanol, 1-methyl-α-(phényl)-O-(3-dimethylamino)propyl |
| 95 | 1H-Pyrazol -5-methanol, 1-methyl-α-(phenyl)-α-methyl-O-(3-dimethylamino)propyl |
| 96 | 1H-pyrazol -5-methanol, 1,3-dimethyl-α-(phenyl)-α-methyl-O-(3-dimethylamino)propyl |
| 97 | 1H-Pyrazol -5-methanol, 1,3-dimethyl-α-(phenyl)-O-(3-dimethylamino)propyl |
| 98 | 1H-Pyrazol -5-methanol, 1-methyl-α-(2-methylphenyl)-O-(3-dimethylamino)propyl |
| 99 | 1H-Pyrazol -5-methanol, 1-methyl-α-(4-chlorophenyl)-O-(3-dimethylamino)propyl |
| 100 | 1H-Pyrazol -4-methanol, 1-methyl-α-phenyl-O-(3-dimethylamino)propyl |
| 101 | 1H-Pyrazol -4-methanol, 1-methyl-α-(4-chlorophenyl)-O-(3-N-morpholino)propyle |
| 102 | 1H-Pyrazol -4-methanol, 1-methyl-α-(4-chlorophenyl)-O-(3-N-pyrrolidino)propyl |
| 103 | 1H-Pyrazol -5-methanol, 1-methyl-α-phenyl-O-(3-N-piperidino)propyl |
| 104 | 1H-Pyrazol -5-methanol, 1-méthyl-α-phenyl-O-(3-N-pyrrolidino)propyl |
| 105 | 1H-Imidazol -2-methanol, α-phenyl |
| 106 | 1H-Imidazol -2-methanol, α-(4-chlorophenyl) |
| 107 | 1H-Imidazol -2-methanol, α-(4-chlorophenyl)-1-methyl |

| | |
|---|---|
| 108 | 1H-Imidazol -2-methanol, α-(3-chlorophenyl)-1-methyl |
| 109 | 1H-Imidazol -2-methanol, α-(4-fluorophenyl)-1-methyl |
| 110 | 1H-Imidazol -2-methanol, 1-methyl-α-(3-trifluoromethylphenyl) |
| 111 | 1H-Imidazol -2-methanol, 1-methyl-α-(4-trifluoromethylphenyl) |
| 112 | 1H-Imidazol -2-methanol, 1-methyl-α-(3,4,5-trimethoxyphenyl) |
| 113 | 1H-Imidazol -2-methanol, α-(3,4-dichlorophenyl)-1-methyl |
| 114 | 1H-Imidazol -2-methanol, 1-butyl-α-(4-trifluoromethylphenyl) |
| 115 | 1H-Imidazol -2-methanol, 1-butyl-α-(2,4-dichlorophenyl) |
| 116 | 1H-Imidazol -2-methanol, 1-butyl-α-(4-chlorophenyl) |
| 117 | 1H-Imidazol -2-methanol-1-butyl-α-(3,4,5-trimethoxyphenyl) |
| 118 | 1H-Imidazol -2-methanol, 1-dodecyl-α-(3,4,5-trimethoxyphenyl) |
| 119 | 1H-Imidazol -2-methanol, α-butyl-α-(3-chlorophenyl)-1-methyl |
| 120 | 1H-Imidazol -2-methanol, α-(3-chlorophenyl)-α-methyl-1-methyl |
| 121 | 1H-Imidazol -2-methanol, α-(4-chlorophenyl)-α-methyl-1-methyl |
| 122 | 1H-Imidazol -2-methanol, α-(4-chlorophenyl)-α-[(N-methyl-peridin )4-yl]-1-methyl |
| 123 | 1H-Imidazol -2-methanol, α-(4-chlorophenyl)-α-ethyl-1-methyl |

| | |
|---|---|
| 124 | 1H-Imidazol -2-methanol, α-butyl-α-(4-chlorophenyl)-1-methyl |
| 125 | 1H-Imidazol -2-methanol, α-(4-chlorophenyl)-α-cyclohexyl-1-methyl |
| 126 | 1H-Imidazol -2-methanol, α-(2-chlorophenyl)-α-methyl-1-methyl |
| 127 | 1H-Imidazol -2-methanol, α-butyl-α-(2-chlorophenyl)-1-methyl |
| 128 | 1H-Imidazol -2-methanol, α-methyl-1-methyl-α-(3-trifluoromethylphenyl) |
| 129 | 1H-Imidazol -2-methanol, α-butyl-1-methyl-α-(3-trifluoromethylphenyl) |
| 130 | 1H-Imidazol -2-methanol, α-cyclohexyl-1-methyl-α-(3-trifluoromethylphenyl) |
| 131 | 1H-Imidazol -2-methanol, α-methyl-1-methyl-α-(4-trifluoromethylphenyl) |
| 132 | 1H-Imidazol -2-methanol, α-(4-fluorophenyl)-α-methyl-1-methyl |
| 133 | 1H-Imidazol -2-methanol, α-(4-methoxyphenyl)-α-methyl-1-methyl |
| 134 | 1H-Imidazol -2-methanol, α-(3,4-dichlorophenyl)-α-methyl-1-methyl |
| 135 | 1H-Imidazol -2-methanol, α-butyl-α-(3,4-dichlorophenyl)-1-methyl |
| 136 | 1H-Imidazol -2-methanol, α-cyclohexyl-α-(3,4-dichlorophenyl)-1-methyl |
| 137 | 1H-Imidazol -2-methanol, α-methyl-1-methyl-α-(3,4,5-trimethoxyphenyl) |
| 138 | 1H-Imidazol -2-methanol, 1-butyl-α-(4-chlorophenyl)-α-methyl |
| 139 | 1H-Imidazol -2-methanol, 1-butyl-α-butyl-α-(4-chlorophenyl) |

| | |
|---|---|
| 140 | 1H-Imidazol -2-methanol, 1-butyl-α-(4-chlorophenyl)-α-[(N-methylpiperidin )-4-yl] |
| 141 | 1H-Imidazol -2-methanol, 1-butyl-α-butyl-α-(3,4,5-trimethoxyphenyl) |
| 142 | 1H-Imidazol -2-methanol, 1-butyl-α-butyl-α-(2-chlorophenyl) |
| 143 | 1H-Imidazol -2-methanol, 1-butyl-α-ethyl-α-(3-trifluoromethylphenyl) |
| 144 | 1H-Imidazol -2-methanol, 1-butyl-α-butyl-α-(2,4-dichlorophenyl) |
| 145 | 1H-Imidazol -2-methanol, α-(4-chlorophenyl)-α-methyl-1-(N-piperidino)propyl |
| 146 | 1H-Imidazol -2-methanol, α-(4-chlorophenyl)-1-(3-dimethylamino)propyl-α-methyl |
| 147 | 1H-Imidazol -2-methanol, α-butyl-1-dodecyl-α-(3,4,5-trimethoxyphenyl) |
| 148 | 1H-Imidazol -2-methanol, 1-benzyl-α-butyl-α-(3-trifluoromethylphenyl) |
| 149 | 1H-Imidazol -2-methanol, 1-benzyl-α-(4-chlorophenyl)-α-methyl |
| 150 | 1H-Imidazol -2-methanol-1-(2-cyanoethyl)-α-(4-chlorophenyl) |
| 151 | 1H-Imidazol -2-methanol-1-(3-aminopropyl)-α-(4-chlorophenyl) |
| 152 | 1H-Imidazol -2-methanol-1-(propan -3-carboxyl )-α-(3-chlorophenyl) |
| 153 | 1H-Imidazol -2-methanol-1-(3-hydroxypropyl)-α-(4-chlorophenyl) |
| 154 | (1H-Imidazol -2-methanol-1-(3-methylpropanoat )-α-(4-chlorophenyl) |
| 155 | 1H-Imidazol -2-methanol-1-(3-hydroxypropyl)-α-phenyl |

| | |
|---|---|
| 156 | 1H-Imidazol -2-methanol-1-(3-hydroxypropyl)-α-(4-methylphenyl) |
| 157 | 1H-Imidazol -2-methanol-1-(3-hydroxypropyl)-α-(4-methoxyphenyl) |
| 158 | 1H-Imidazol -2-methanol-1-(3-hydroxypropyl)-α-(3,4-dichlorophenyl) |
| 159 | 1H-Imidazol -2-methanol-1-(3-methylpropanoat )-α-phenyl |
| 160 | 1H-Imidazol -2-methanol-1-(4-hydroxybutyl)-α-(4-chlorophenyl) |
| 161 | 1H-Imidazol -2-methanol-1-(3-cyanopropyl)-α-(4-chlorophenyl) |
| 162 | 1H-Imidazol -2-methanol-1-(4-butyroyl )-α-(4-chlorophenyl) |
| 163 | 1H-Imidazol -2-methanol-1-(4-methylbutyrat )-α-(4-chlorophenyl) |
| 164 | 1H-Pyrazol -5-methanol, 1-butyl-α-phenyl |
| 165 | 1H-Pyrazol -5-methanol, α-(4-chlorophenyl)-1-methyl |
| 166 | 1H-Pyrazol -5-methanol, 1-methyl-α-(3,4,5-trimethoxyphenyl) |
| 167 | 1H-Pyrazol -5-methanol, 1-butyl-α-(3,4,5-trimethoxyphenyl) |
| 168 | 1H-Pyrazol -5-methanol, 4-bromo-1-methyl-α-phenyl |
| 169 | 1H-Pyrazol -5-methanol, α-(4-chlorophenyl)-1-methyl-α-phenyl |
| 170 | 1H-Pyrazol -5-methanol, 1-butyl-α-(4-chlorophenyl)-α-methyl |
| 171 | 1H-Pyrazol -5-methanol, 1-methyl-α-methyl-α-phenyl |
| 172 | 1H-Pyrazol -5-methanol, 1-methyl-α-methyl-α-(3,4,5-trimethoxyphenyl) |
| 173 | 1H-Pyrazol -5-methanol, 1,3-dimethyl-α-methyl-α-phenyl |

| | |
|---|---|
| 174 | 1H-Pyrazol -5-methanol, α-ethenyl-1-methyl-α-phenyl |
| 175 | 1H-Pyrazol -5-methanol, 1-butyl-α-(4-chlorophenyl)-α-ethenyl |
| 176 | 1H-Pyrazol -5-methanol, 4-chloro-1-methyl-α-phenyl |
| 177 | 1H-Pyrazol -5-methanol, 1-methyl-α-(2-methylphenyl) |
| 178 | 1H-Pyrazol -5-methanol-1-methyl-α-(3-chlorophenyl) |
| 180 | 1H-Pyrazol -5-methanol-1-methyl-α-(2-chlorophenyl) |
| 181 | 1H-Pyrazol -5-methanol-1-methyl-α-(4-methoxyphenyl) |
| 182 | 1H-Imidazol -2-methanol, α-(4-chlorophenyl)-α-methyl-1-methyl-O-(2-trimethylammonium)-ethyl-jodid |
| 183 | 1H-Imidazol -2-methanol, α-(4-chlorophenyl)-α-methyl-1-methyl-O-(3-trimethylammonium)-propyljodid |
| 184 | 1H-Imidazol -2-methanol, α-(4-methoxyphenyl)-α-methyl-1-methyl-O-(2-trimethylammonium)-ethyl-jodid |
| 185 | 1H-Imidazol -2-methanol, α-(4-methoxyphenyl)-α-methyl-1-methyl-O-(3-trimethylammonium)-propyl-jodid |
| 186 | 1,1-Dimethylimidazolium-2-methanol, α-(4-methoxyphenyl)-α-methyl-jodid |

3. Verfahren zur Herstellung der Verbindungen nach einem der Ansprüche 1 oder 2, dadurch gekennzeichnet, daß man mindestens eine der folgenden Operationen durchführt:

3.1. Umsetzung einer Verbindung der allgemeinen Formel (II)

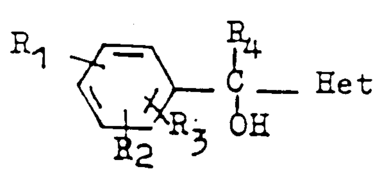

II

mit einer Verbindung der allgemeinen Formel (III)

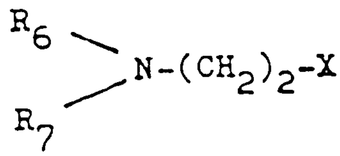

III

worin $R_1$ bis $R_4$, $R_6$ und $R_7$, X und Het die in Anspruch 1 angegebenen Bedeutungen haben;
3.2 Umsetzung einer Verbindung der allgemeinen Formel (II) mit einer Verbindung der allgemeinen Formel (IV)

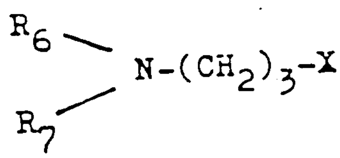

IV

worin $R_1$ bis $R_4$, $R_6$ und $R_7$, X und Het die in Anspruch 1 angegebenen Bedeutungen haben.

**4.** Verfahren zur Herstellung der Verbindungen der allgemeinen Formel (II), dadurch gekennzeichnet, daß man mindestens eine der folgenden Operationen durchführt:
4.1. Reduktion einer Verbindung der allgemeinen Formel (V)

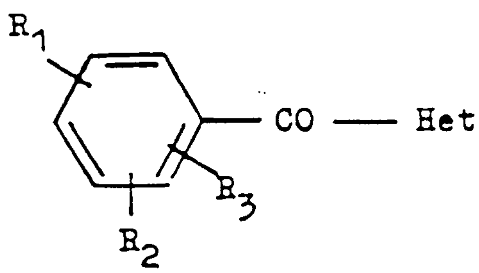

V

worin $R_1$, $R_2$, $R_3$ und Het die in Anspruch 1 angegebenen Bedeutungen haben;
4.2. Umsetzung einer Verbindung der allgemeinen Formel (V) mit Grignard-Reagentien vom Typ

$R_4$-Mg-X

worin $R_4$ die in Anspruch 1 angegebenen Bedeutungen hat.

**5.** Verfahren zur Herstellung von Verbindungen der allgemeinen Formel (II), dadurch gekennzeichnet, daß man die folgende Operation durchführt:
Umsetzung eines Aldehyds der allgemeinen Formel (VI)

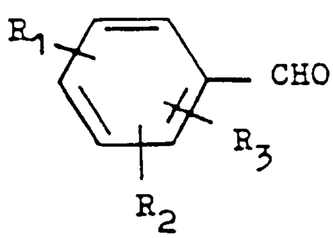

VI

mit Verbindungen vom Het-Li-Typ, worin $R_1$, $R_2$, $R_3$ die in Anspruch 1 angegebenen Bedeutungen haben, Het ein Pyrazol der in Anspruch 1 angegebenen allgemeinen Formel darstellt und Li für ein Lithiumatom steht.

**6.** Verfahren zur Herstellung von Verbindungen der allgemeinen Formel (VII)

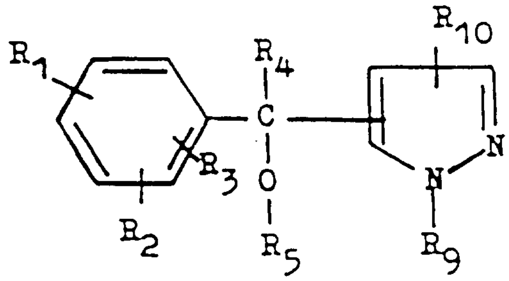

VII

worin $R_1$ bis $R_9$ die in Anspruch 1 angegebenen Bedeutungen haben und $R_{10}$ für Chlor oder Brom steht,
durch Halogenierung der Vorläuferverbindung der allgemeinen Formel (VII), worin $R_1$ bis $R_9$ die in den Ansprüchen 1 bis 10 angegebenen Bedeutungen haben und $R_{10}$ für ein Wasserstoffatom steht.

**7.** Verfahren zur Herstellung von Verbindungen der allgemeinen Formel (VIII)

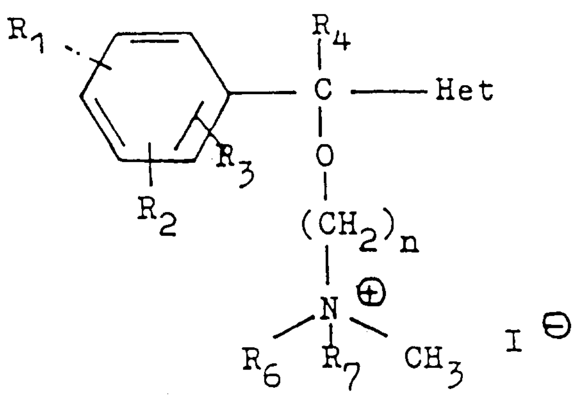

VIII

durch Umsetzung von Verbindungen der allgemeinen Formel (I)

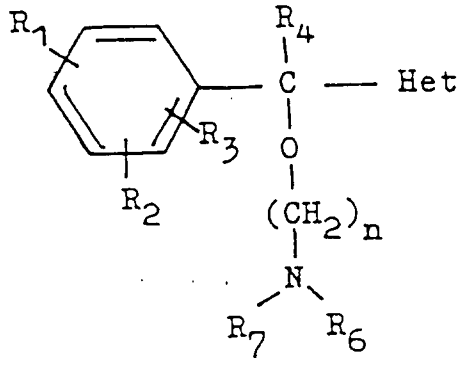

I

worin $R_1$ bis $R_4$, $R_6$, $R_7$ und Het die in Anspruch 1 angegebenen Bedeutungen haben,
mit Methyljodid.

8. Verfahren zur Herstellung von Verbindungen der allgemeinen Formel (IX)

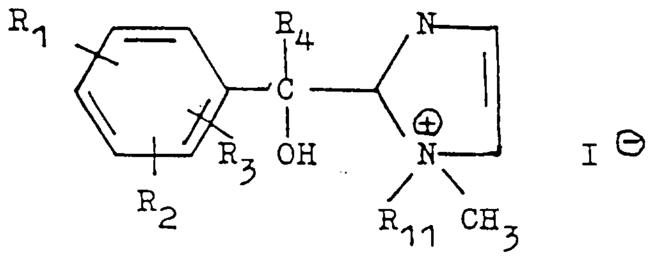

IX

durch Umsetzung von Verbindungen der allgemeinen Formel (X)

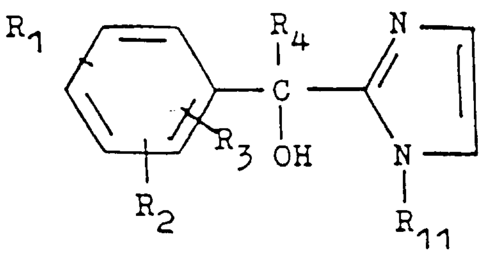

X

worin $R_1$ bis $R_4$ und $R_{11}$ die in Anspruch 1 angegebenen Bedeutungen haben,
mit Methyljodid.

9. Derivate der allgemeinen Formel (I) und ihre therapeutisch akzeptablen Salze nach einem der Ansprüche 1 und 2 als Arzneimittel, insbesondere als Arzneimittel für die Schmerzbehandlung.

10. Pharmazeutische Zusammensetzungen, dadurch gekennzeichnet, daß sie außer einem pharmazeutisch akzeptablen Träger mindestens ein Derivat der allgemeinen Formel (I) oder eines seiner physiologisch akzeptablen Salze nach einem der Ansprüche 1 und 2 enthalten.

11. Verwendung der Aryl-heteroaryl-carbinol-Derivate der allgemeinen Formel (I)

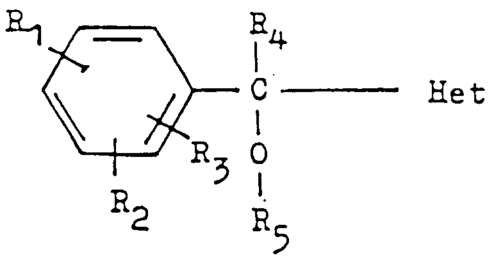

I

sowie ihrer therapeutisch akzeptablen Salze, worin bedeuten:

| | |
|---|---|
| $R_1$, $R_2$ und $R_3$, | die gleich oder verschieden sind, jeweils ein Wasserstoffatom, ein Halogenatom, einen niederen Alkylrest, einen niederen Alkoxyrest oder eine Trifluoromethylgruppe, |
| $R_4$ | ein Wasserstoffatom, einen $C_1$-$C_4$-Niedrigalkylrest, einen Cycloalkylrest, einen niederen Alkenylrest oder einen durch ein Stickstoffatom substituierten Cycloalkylaminorest, |
| $R_5$ | ein Wasserstoffatom oder einen Rest der Formel |

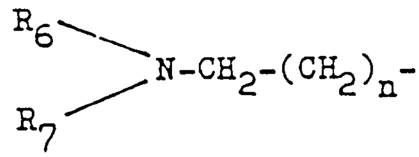

in der n die Werte 1 oder 2 haben kann und die Gruppierung

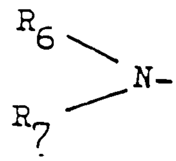

eine der folgenden Bedeutungen haben kann:
eine niedere Dialkylaminogruppe oder einen cyclischen Aminorest, ausgewählt unter den Piperidino-, Morpholino- oder Pyrrolidino-Gruppen,

$R_5$ außerdem auch eine Gruppe der allgemeinen Formel

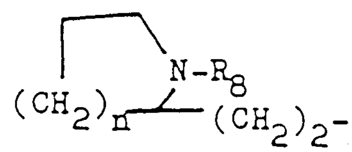

in der n den Wert 1 oder 2 haben kann und in der $R_8$ einen niederen Alkylrest darstellt,

Het ein Azol, ausgewählt unter den folgenden:

a) ein Pyrazol der allgemeinen Formel

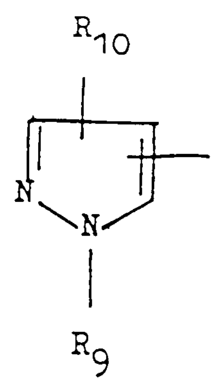

in der $R_9$ eine der folgenden Bedeutungen hat: ein Wasserstoffatom, einen $C_1$-$C_4$-Niedrigalkrest, den Dodecylrest, den Benzylrest oder einen Rest des Typs

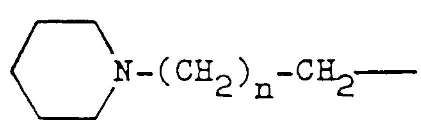

in der n = 1 oder 2, und

in der $R_{10}$ ein Wasserstoffatom, einen Methylrest oder ein Halogenatom darstellt,

b) ein Imidazol der allgemeinen Formel

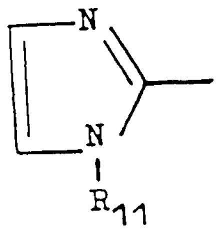

in der $R_{11}$ irgendeine der folgenden Gruppen darstellt: ein Wasserstoffatom, einen $C_1$-$C_4$-Niedrigalkylrest, einen Dodecylrest, einen Rest der Formel A-$(CH_2)_n$-, in der n = 2, 3 oder 4 und A eine beliebige der folgenden Gruppen darstellt: einen Piperidinrest, eine Cyanogruppe, einen Hydroxyrest, einen Carboxyrest, eine Aminogruppe oder eine Methylestergruppe,

c) ein Imidazol der Formel

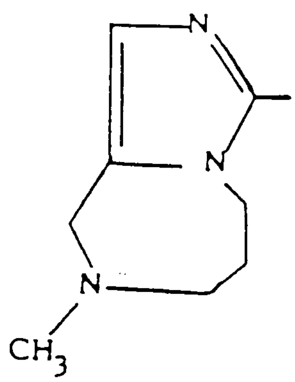

zur Herstellung von Arzneimitteln für die Schmerzbehandlung, insbesondere für die Herstellung von Analgetika.

**12.** 1H-Pyrazol-5-methanol-1-methyl-α-(4-methylphenyl) als Arzneimittel.